(19) Europäisches Patentamt
European Patent Office
Office européen des brevets

(11) EP 2 995 688 A1

(12) **EUROPEAN PATENT APPLICATION**

(43) Date of publication:
**16.03.2016 Bulletin 2016/11**

(51) Int Cl.:
***C12Q 1/68*** *(2006.01)*

(21) Application number: **14184663.4**

(22) Date of filing: **12.09.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Genclis**
**54500 Vandoeuvre-les-Nancy (FR)**

(72) Inventors:
- **Bihain, Bernard**
  **54000 Nancy (FR)**
- **Verdun, Stéphane**
  **54000 Nancy (FR)**
- **Thouvenot, Benoit**
  **54230 Chaligny (FR)**

(74) Representative: **Becker, Philippe et al**
**Cabinet Becker & Associés**
**25, rue Louis Le Grand**
**75002 Paris (FR)**

(54) **Methods of prognosing and treating triple negative breast cancer**

(57) The invention relates to the field of cancer prognosis, diagnosis and therapeutics. More particularly, the invention provides methods to predict the clinical outcome and severity of a breast cancer, based on the measure of the rate of divergences between RNA and DNA sequences in at least one target gene in a sample from a patient. The invention is particularly suited for diagnosing and predicting outcome in patients having triple negative breast cancer and for optimizing treatment strategies.

EP 2 995 688 A1

SEQUENCE LISTING

<110> Transmedi SA

<120> Methods of prognosing and treating triple negative breast cancer

<130> B1867EP00

<140> B1867
<141> 2014-09-12

<160> 8

<170> PatentIn version 3.5

<210> 1
<211> 25
<212> RNA
<213> Artificial sequence

<220>
<223> GDI2 small interfering RNA molecule

<400> 1
agaaccaagu caaucgaaag ucaga 25

<210> 2
<211> 25
<212> RNA
<213> Artificial Sequence

<220>
<223> GDI2 small interfering RNA molecule

<400> 2
agcgcaagaa gaaugacauc uaugg 25

<210> 3
<211> 25
<212> DNA/RNA
<213> Artificial sequence

<220>
<223> GDI2 small interfering RNA molecule

<400> 3
gagaggaagc gaacauaaag cgggt 25

<210> 4
<211> 2412
<212> DNA
<213> Artificial sequence

<220>
<223> GDI2 cDNA; NM_001494.3

<400> 4
agagtgccgg gagcgcctga ttggcggccc tcccggcgcc tgcaggcccc gccccggctg 60

cggaggctca gtcacagccc tcccctcctc gctccctccc ctcctctccc cgcccagttc 120

```
ttctcttccc gtctgaggtg gcggtcggtc tcgccttgtc gccagctcca ttttcctctc      180
tttctcttcc cctttccttc gcgcccaaga gcgcctccca gcctcgtagg gtggtcacgg      240
agcccctgcg ccttttcctt gctcgggtcc tgcgtccgcg cctgccccgc catgaatgag      300
gagtacgacg tgatcgtgct gggcaccggc ctgacggaat gtatcctgtc aggtataatg      360
tcagtgaatg gcaagaaagt tcttcatatg gatcgaaacc cttactacgg aggagagagt      420
gcatctataa caccattgga agatttatac aaaagattta aaataccagg atcaccaccc      480
gagtcaatgg ggagaggaag agactggaat gttgacttga ttcccaagtt ccttatggct      540
aatggtcagc tggttaagat gctgctttat acagaggtaa ctcgctatct ggattttaaa      600
gtgactgaag ggagctttgt ctataagggt ggaaaaatct acaaggttcc ttccactgaa      660
gcagaagccc tggcatctag cctaatggga ttgtttgaaa aacgtcgctt caggaaattc      720
ctagtgtatg ttgccaactt cgatgaaaaa gatccaagaa cttttgaagg cattgatcct      780
aagaagacca caatgcgaga tgtgtataag aaatttgatt tgggtcaaga cgttatagat      840
tttactggtc atgctcttgc actttacaga actgatgatt acttagatca accgtgttat      900
gaaaccatta atagaattaa actttacagt gaatctttgg caagatatgg caaaagccca      960
tacctttatc cactctatgg ccttggagaa ctgccccaag gatttgcaag gctaagtgct     1020
atttatggag gtacctatat gctgaataaa cccattgaag aaatcattgt acagaatgga     1080
aaagtaattg gtgtaaaatc tgaaggagaa attgctcgct gtaagcagct catctgtgac     1140
cccagctacg taaaagatcg ggtagaaaaa gtgggccagg tgatcagagt tatttgcatc     1200
ctcagccacc ccatcaagaa caccaatgat gccaactcct gccagatcat tattccacag     1260
aaccaagtca atcgaaagtc agatatctac gtctgcatga tctcctttgc gcacaatgta     1320
gcagcacaag ggaagtacat tgctatagtt agtacaactg tggaaaccaa ggagcctgag     1380
aaggaaatca gaccagcttt ggagctcttg gaaccaattg aacagaaatt tgttagcatc     1440
agtgacctcc tggtaccaaa agacttggga acagaaagcc agatctttat ttcccgcaca     1500
tatgatgcca ccactcattt tgagacaacg tgtgatgaca ttaaaaacat ctataagagg     1560
atgacaggat cagagtttga ctttgaggaa atgaagcgca agaagaatga catctatggg     1620
gaagactaac agcagtacat gttattatgt aattaggaca catttaaaat ttggcaaata     1680
atgcatataa tgaaatcaat attgtaaggc ctgcttttgt aatgaaaatg gagagaatga     1740
agagcgctgt gccagtaaat actccccttc acctttctaa ttattaactt gttttcatgg     1800
agtggctatt cagcattggc agttaccaca ttctgttcaa tttaaccaaa ctggcttttt     1860
tttttctag tgaagttaaa caaacattgg gataccgaca cagacaactt gagacagttt      1920
ttttaatctt ttaatcagtg tagctatgtg ctgctgctgc tcaagagctg gatccataca     1980
```

ggttgtgtgt catctgtcct cttgaggttc aggagattct aaattgaata ttccacggtt 2040

tgggacagca tccggaagtt ttccctatga ctttatattt tgtattatgt caaatgttat 2100

ggcagggccc aaatagcata gccacaagtt tggtttatgt gggcataaaa ttctaaccaa 2160

accccagaca tagggagtca tttggagaaa gcctgtatgt ggtgttttaa cctaataaag 2220

ttgatgagag agaaggggag aggaagcgaa cataaagcgg gtcaagtgta gtgcatcttt 2280

tgtatctcag gcgtggcttc ttcagtggac caagctgcaa tgcagtattg acttgacaga 2340

gcctctactt ctgtctcaaa atggctccaa atgatttctg tactgcaaaa taaagccaaa 2400

ttctggaaac ta 2412

<210> 5
<211> 5865
<212> DNA
<213> Artificial sequence

<220>
<223> POM121C cDNA; NM_001099415.2

<400> 5
ttcctgtgcc tcttcaggtc atcgcttgct ctcgttccca ggctttggct tctagtggac 60

gagaatcacc gagtctgcgg ggctggatgc tgaccgcccg gaccagcacc taggcgggcg 120

ggagctgtgc ggcccagggt tcgcgcgggc cgggtagagg ctcgagctgg gacccccgag 180

cgtgaacccc ggagccggcg gcgctggggc cagaggggcc gggagcccca gggaggcgga 240

tctgggcccc gagaaggaca cccgcctgga tttgccccgt aggcccggcc cgggcccctc 300

gggagcagaa cagccttggt gaggtggaca ggaggggacc tcgcgagcag acgcgcgcgc 360

cagcgacagc agcccgcccc ggcctctcgg gagccgtggg gcagaggctg cggagcccca 420

ggagggggcc agtgtcattc aaagatgtgg ctgtggattt cacccaggag gagtggcggc 480

aactggaccc tgatgagaag ataacatacg gggatgtgat gttggagaac tacagccatc 540

tagtttcctt ggcttatgag gtggcaacat cttgtacttc ggagattctg aagccgagca 600

acttgcccaa gtccttcttc ttttcccatt aacaagatat gatatcacca agccaaacgt 660

catcattaag ttggagcagg gagaggagct gtggataacg ggaggtgaat ttccatgtca 720

acatagtcct gggatcgtgg gactttacca gatcggtttg taataacacc tcgaagacgc 780

tatccgatcc atcagaccca gtattcctgt ccgggggtac ttcccacagt gtgctggaat 840

ggttatcaca agaaggctgt gctgtcccct cgcaactcca ggatggtgtg tagcccagtg 900

actgtgagga tcgcccctcc tgacagaaga ttttcacgtt ctgcgatacc agagcagata 960

atcagctcaa cactgtcgtc accatcaagt aatgccccag acccatgtgc aaaggagact 1020

gtactgagtg ccctcaaaga gaagaagaag aaaaggacag tggaggaaga agaccaaata 1080

ttccttgatg gccaggaaaa taaaagaagg cgccatgata gcagtggcag tggacattca 1140

```
gcatttgagc ccctggtggc cagtggagtc cccgcttctt ttgtgcctaa gcctgggtct      1200
ctgaagagag gcctcaattc tcagagctca gatgaccact tgaataagag atcccgaagc      1260
tcttccatga gctccttgac aggcgcttac acaagtggca tccctagctc cagccgcaat      1320
gccattacca gttcctacag ctccactcga ggcatctcac agctctggaa gagaaatggc      1380
cccagttcat caccttctc tagcccagcc tcatcccgct cccagacacc ggagaggcca      1440
gcaaagaaaa taagagaaga agagctgtgt catcattcca gttcttcaac tccattggca      1500
gcagacaagg agtcccaggg agaaaaggct gcagatacaa ccccaaggaa gaaacaaaac      1560
tcgaattctc agtctacacc tggcagctct gggcagcgta agcggaaagt tcagctgctg      1620
ccttctcggc gaggggaaca gctgaccttg cctccacctc cccagcttgg ctattcgatc      1680
actgccgagg acctagactt agagaagaag gcttcattac agtggttcaa ccaggccttg      1740
gaggacaaga gtgatgctgc ctcgaactct gtcactgaga ccccacctac cactcagcct      1800
tcatttacct ttaccctgcc tgctgctgca actgcctccc cacccacctc cctcctggcc      1860
ccaagcacca acccactgtt agagagcttg aagaagatgc agactccccc gagcctgcca      1920
ccctgcccag aatctgctgg agcagcaacc actgaggccc tctcacctcc aaagacaccc      1980
agcctcctac ccccgctggg tttatcacag tcagggccgc cagggctgct ccccagcccc      2040
tcctttgact ccaaaccccc gaccactttg ctggggctga tccctgctcc atccatggta      2100
ccagccactg acaccaaggc acctccaacc cttcaagcag agacggctac caaaccccaa      2160
gccacatctg ccccgtcccc cgcccccaag caaagcttcc tgtttggaac acagaacacc      2220
tcaccttcca gccctgccgc ccctgctgca tcttcagcat ctcccatgtt caagcccatt      2280
ttcacggctc cacccaagag tgagaaggaa ggccccacac cgcctggccc ttcagtcaca      2340
gccacagcgc cctccagctc ctccctcccc acgaccacca gcaccacagc cccgaccttc      2400
cagcctgtct ttagcagcat ggggccacct gcatctgtgc ccttgcctgc tcccttcttc      2460
aagcagacaa ctactcccgc cactgctccc accacaactg ccccgctctt cactggcctg      2520
gccagcgcca cctctgctgt ggctcccatc acctctgcca gtccatccac agactctgct      2580
tcgaagcctg cgtttggctt tggcataaac agtgtgagca gcagcagtgt gagtaccacg      2640
accagcaccg ccactgccgc ctcacagcct ttcctcttcg ggcgccccca ggcctctgct      2700
gccagcttca ccccggccat gggctccata ttccagtttg gcaaacctcc tgccttgccc      2760
acaaccacca cagtcaccac cttcagccag tccctgccca ctgccgtgcc aacggccacc      2820
agcagcagcg ctgccgactt tagtggtttt ggcagcaccc tcgccacctc cgccccggcc      2880
accagcagcc agcccactct gacgttcagt aacacgagca cccccacgtt caacattccc      2940
tttggctcaa gcgccaagtc cccgctccca tcatatccgg gagccaaccc ccagcccgca      3000
```

tttggggccg ctgaggggca gccaccgggg gccgccaagc cagcccttac ccccagcttt 3060
ggcagctctt tcacttttgg aaactctgca gccccggccc cggctactgc acccacacct 3120
gcacctgcgt ccacgatcaa gatcgtgcct gcgcacgtgc ctacgcccat ccagcctacc 3180
tttggcggtg ccacgcactc ggcgtttgga ttgaaagcca cggcttccgc cttcggcgct 3240
cccgccagct cacagcccgc ctttggcggc tccactgctg tcttctcctt cggtgcagcc 3300
accagctccg gctttggagc caccacccag accgccagca gcgggagcag cagctcggtg 3360
tttggcagca caacaccatc acccttcacg tttgggggtt cggcagcccc cgctggcagt 3420
gggagctttg ggatcaacgt ggccacccca ggctccagcg ccaccaccgg agctttcagc 3480
tttggagcag gacagagtgg gagcacagcc acctccaccc ccttcacagg gggcttaggt 3540
cagaacgccc tgggcaccac cggccagagc acaccgtttg ccttcaacgt gggcagcaca 3600
actgagagca aacctgtgtt tggaggcacc gccaccccca cctttggtca gaacacccct 3660
gcgcctggag tgggcacatc gggcagcagc ctctcctttg gggcatcttc agcacccgcc 3720
caaggctttg ttggtgttgg accgttcgga tcggcggccc cttcattttc cattggtgcg 3780
ggatccaaga ccccaggggc tcgacagcga ctgcaggccc gaaggcagca cacccgcaaa 3840
aagtagcctt tgtcccctgt ccctgttccc cccacccctt ccctaaatct ggaccttggc 3900
acgtgctaga aagagccttg gacccttcca gctgcgtaaa gcaaacctac cccggatctc 3960
tggcttcagc cgccaggggg cagtggcagc cctggggccc tttcccttct ggaggaagca 4020
caagcctcag ggaaggggaa gcaggatgcg gagggccaaa gcccgggacc tctacttgaa 4080
cagttccact ggggaggctg gagaactaag gacctgtaca tagtgtccgc tgccctgact 4140
cccgcttagc gcacccttag gcaggcgccc cttccacctt tccccgagag ccgtcgtcgc 4200
tggagggggc agggtccagc ccgcctggat cggtggtgtg cacctgatgg gatttgggaa 4260
atgggttatc cctaaagctt tatcttgctt ggcttagctg tgagaagtgg ttctcttcct 4320
ctggtccctt ctggggactc tgtttcccca tttcttgctg ctgtgtccct caccggttcc 4380
ttgcaggatt ccctcctttt taaatgccct tgaatctagc tttgccttgg agaccccagt 4440
gggtgctgct cctgccgttt tcttcctgcc aagcctgaat caatgtttca tctccaaccc 4500
tctgccagtt tggcccctca gagcttggtg gctcaagact gttagcctgg cagagccagg 4560
ggtgaaggga gaagctcttg gagcaggcag gatgcccacc gctgcttcag ctgcctcctc 4620
gcccagctac cctttggccc cattgggccc tcgtctgcct ctccaggatt gtatgtttca 4680
agccttgtcc tgtgttcctt tgtctgacgc tctgtgtatt gctctttgaa tcgagtttgg 4740
aggaagagtt gagttgtatg agtggcggca tgttggtagt gccggacttc ctgtttcaag 4800
ttttctgggg cctcgctaat tgaatgtgga aagtagcacc acttgacggc tacaagtgcc 4860
gactcctgaa ttttcccatg gtgttctgac ttcaagggct ggcagccagg gagaatgggc 4920

ccaggggaag caaagacctc ttccctctgc ggtttctgtc ccacttaact gacctcactg 4980

gaggctacgt cacccaaagt agatgttaga aaacctaaat taatgaacca tatttttaaa 5040

atcctatttt tcccaaacag ggccctctgc agcccatcct ttccttccgt ccttctgaaa 5100

ccacataccc caggcccaag cgccttgctg tcacgcccaa cctctttggg agaagtatga 5160

atgcgtgtgt ctaaattaaa agaaaaaaat atttaaacgt tttttaacaa aaatttattt 5220

ttgtatttaa gctaaattgc cttttaaatt ccttcaagct tggttcattg aggtggttaa 5280

gtataaatgc tattaactag gaattagctg tatagttaag ttatgcctgt gcaaagaaga 5340

ggctcaaatg ctgtccccgg cagctttcct gggggactag agctccttct ggccatgtta 5400

tatgaaatgt aattcttatt ttataaataa tgtgatgtag atgtaaccgg tgccccctcc 5460

ccgttgtgac tgagggcgag tgttatacct ggctgcgtgt gcagctgagg agggagtgaa 5520

cctcaagcct aaatacctgt taggattgga gggtctgggt gggcctgggc ctagcaatca 5580

atcaagcttc tacctgtacc ttatgtaagg tagaccctcc tagtgtcagt acctgagctt 5640

gtttacctca gttccgcagg caggacagcc ggtccgggaa ccctgagtga gagtgagtgt 5700

ggatgtgtac agtacacaca ctggacggca gcgggaggct gggactttcc attacaaata 5760

gagacttcat tcctgttgag tctagttgga atttttagta tgaatgtgag atttttctcc 5820

tgcttgtgac attaagaata aaaaactgtg atctatcgta gagta 5865

<210> 6
<211> 3123
<212> DNA
<213> Artificial sequence

<220>
<223> SORBS3 cDNA; NM_005775.4

<400> 6
ctccggccca gccccggccc gcagtccaga tccgagaccc aaactccgcc cgccccgccg 60

cgcgactctc acggtccggc ctccggcgcg cgccccttcg gcctccctct tcctgcgccg 120

gcgcccggcc cggcccggcc cgtcctgacc cggtcacgag ggccgcgacg gcccgcgctt 180

ctccttgccc ttcctcctcg agcgcccgcg ccaggcagca gccgggcagg gatgctcctg 240

cgctcccggg cggcctcggg cccagccacc tgctcgccgg ggaagaggac acgcagagga 300

gcagctggct tgcccggagt cctcccacct tgacccaagc atgcagggcc caccccgcag 360

cctccgcgct gggctcagcc tggacgactt catccctggc cacctccagt cccacatagg 420

gtcttcctcc cgggggacac gggtgcccgt gatccggaat ggtggctcca acacccttaa 480

tttccagttc cacgaccccg cgcccaggac tgtgtgcaat gggggctaca caccaagacg 540

agatgcttcc cagcacccgg accctgcgtg gtatcagacc tggccaggcc ctgggagcaa 600

gccctctgca agcacaaaga tccctgcctc ccagcacacc cagaactggt cagccacgtg 660
gaccaaggac agcaagcgtc gggacaagcg ctgggtcaag tacgagggaa tcgggcccgt 720
ggacgagagc ggcatgccca ttgcccccg atccagcgtt gacagaccca gagactggta 780
ccggagaatg ttccagcaga ttcaccggaa aatgccagac ttgcagctgg actggacctt 840
cgaggagcca cccagagacc ccaggcatct aggagcccag caaagacctg cccacaggcc 900
cggcccggca acatcttcca gtggaagaag ctgggaccac tctgaagagt tacctagaag 960
caccttcaac tacagacctg gagcattctc cactgtgctg cagccctcaa atcaggtgct 1020
cagacgccgg gaaaaagtag acaatgtctg gacggaagag tcctggaacc agtttctgca 1080
ggaactagag actgggcaga ggcccaagaa accgctggtg gacgaccctg gtgagaagcc 1140
ctcccagccc attgaggtgc tgctggagag agagctggcc gagctgagcg ccgagctgga 1200
caaggacctg cgggcaattg agacccgact gccgtccccc aagagctcgc cggcgccccg 1260
acgggccccg gagcagcggc ccccggccgg cccggcctca gcctggagct ccagctaccc 1320
acatgcacct tacctgggtt ccgcccggtc cctgagtccc cacaaaatgg ctgatggagg 1380
aagcccccttc ctaggtcgga gggactttgt ctacccttcc tcaacccgag accctagtgc 1440
ctctaacgga gggggcagcc cagccaggag ggaagagaag aagagaaagg ccgccaggct 1500
caagtttgac ttccaggcgc agtcccccaa ggagctgact ctgcagaagg gtgacattgt 1560
ctacatccac aaggaggtgg acaagaactg gctggaggga gagcaccacg gccgcctggg 1620
catcttccct gctaattatg tggaggtgct gcccgcagat gagatcccta agcccatcaa 1680
gcccccgacc taccaggtgc tggagtatgg agaggctgtg gcccagtaca ccttcaaggg 1740
ggacctggag gtggagctgt ccttccgcaa gggagagcac atctgcctga tccgcaaggt 1800
gaacgagaac tggtacgagg gacgcatcac gggcacgggg cgccaaggca tattccctgc 1860
cagctacgtg caggtgtctc gtgaaccccg gctccggctc tgtgacgacg gcccccagct 1920
ccccacgtct ccccgcctga ccgctgccgc ccgctcagcc cgtcacccca gctcccccctc 1980
agccctgcgc agcccagctg accccatcga cttgggggga cagacctccc cccgtcgcac 2040
tggcttctcc ttccccaccc aggagcctag accccagacc cagaatcttg gcaccccctgg 2100
tccagctctg tcccactctc gaggtcccag ccatcccctg gacctgggga cctcctctcc 2160
taacacctct cagatacact ggaccccgta ccgggcgatg taccagtaca ggccccagaa 2220
cgaagacgag ctggagctgc gcgaggggga cagggtggat gtcatgcagc agtgtgacga 2280
tggctggttt gtgggtgtct cccggaggac ccagaaattc ggaacgttcc ctggaaatta 2340
cgttgccccg gtgtgagtgg tctccatggc aacttggagc cagccaggat ggggtgggga 2400
gcggtggcac tcgtgggagg gagaggaccc ccgcccacat cctccttccc caggacctga 2460
gctcccagca tctgcagacg acccccgcag cctttccctc ggaccccccct cgaagccccc 2520

tggactgatt cccacccacg actcacaggc attcctccca cagccctttc atttcctccc 2580

caccccactc cccaaataca gaggtctgct ttgaagcgga gaccatttcc aggccttatt 2640

gagaccagac cccaagtccc ccaccccccat cctgctccag cgtttcctct aacagggacc 2700

agctctccgc tttgccccca cggggttcct ctaaccagaa ccagcttcct agcctcgtag 2760

agaccaaagg ccgccccccgc ctgctggggt tcctcccagc accccagctt gctggctgcc 2820

ctctttgcct tctggcctcc agctgggtgt gggggggcgg agcaaggcgg gggacagacg 2880

cagcaccttc ttagcgatct aggcctggca agagctctgg ccccaaggcc tcctcttccc 2940

aggggctgcc aagtcctggc cctggccctg gcatatcacc ccgcactgtg gggccaggca 3000

ccactagcct ggctcaaata ttccccaggg agactgctgt gtgctgcccg cctgcctgct 3060

ggctctcccc cagccccaca tcccctctgg aagagaatgt aaaataaacc tggacacaag 3120

gga 3123

<210> 7
<211> 538
<212> DNA
<213> Artificial sequence

<220>
<223> UQCRHL cDNA; NM_001089591.1

<400> 7
ttgaggtgcc gctgttgctg ctcgtgttaa atctagaacc gtagccagac atgggactgg 60

aggacgagca aaagatgctt accgaatccg gagatcctga ggaggaggaa gaggaagagg 120

aggaattagt ggatccccta acaacagtga gagagcaatg cgagcagttg gagaaatgtg 180

taaaggcccg ggagcggcta gagctctatg atgagcatgt atcctctcga tcacatacag 240

aagaggattg cacggaggag ctctttgact tcttgcatgc aaaggaccat tgcgtggccc 300

acaaactctt taacaacttg aaataaatgt gtggacttaa ttcaccccag ccttcatcat 360

ctgggcatca gaatatttcc ttatggtttc ggatgtacca tttgtttctt atttgtgtaa 420

ctgtaagttc acatgaacct cgtgggtttt ggcttaggct ggtagcttct atgtaattcg 480

cagtgattcc atctaaataa aagttctgtg atctgcaaaa aaaaaaaaaa attagccg 538

<210> 8
<211> 2834
<212> DNA
<213> Artificial sequence

<220>
<223> ADAM15 cDNA; NM_003815.4

<400> 8
ggggcctggt ggccgcgcgg cgctgctggg ttctccgagg cgacctggcc gccggccgct 60

```
cctccgcgcg ctgttccgca cttgctgccc tcgcccggcc cggagcgccg ctgccatgcg      120
gctggcgctg ctctgggccc tggggctcct gggcgcgggc agccctctgc cttcctggcc      180
gctcccaaat ataggtggca ctgaggagca gcaggcagag tcagagaagg ccccgaggga      240
gcccttggag ccccaggtcc ttcaggacga tctcccaatt agcctcaaaa aggtgcttca      300
gaccagtctg cctgagcccc tgaggatcaa gttggagctg gacggtgaca gtcatatcct      360
ggagctgcta cagaataggg agttggtccc aggccgccca accctggtgt ggtaccagcc      420
cgatggcact cgggtggtca gtgagggaca cactttggag aactgctgct accagggaag      480
agtgcgggga tatgcaggct cctgggtgtc catctgcacc tgctctgggc tcagaggctt      540
ggtggtcctg accccagaga gaagctatac cctggagcag gggcctgggg accttcaggg      600
tcctcccatt atttcgcgaa tccaagatct ccacctgcca ggccacacct gtgccctgag      660
ctggcgggaa tctgtacaca ctcagaagcc accagagcac cccctgggac agcgccacat      720
tcgccggagg cgggatgtgg taacagagac caagactgtg gagttggtga ttgtggctga      780
tcactcggag gcccagaaat accgggactt ccagcacctg ctaaaccgca cactggaagt      840
ggccctcttg ctggacacat tcttccggcc cctgaatgta cgagtggcac tagtgggcct      900
ggaggcctgg acccagcgtg acctggtgga gatcagccca aacccagctg tcaccctcga      960
aaacttcctc cactggcgca gggcacattt gctgcctcga ttgccccatg acagtgccca     1020
gctggtgact ggtacttcat tctctgggcc tacggtgggc atggccattc agaactccat     1080
ctgttctcct gacttctcag gaggtgtgaa catggaccac tccaccagca tcctgggagt     1140
cgcctcctcc atagcccatg agttgggcca cagcctgggc ctggaccatg atttgcctgg     1200
gaatagctgc ccctgtccag gtccagcccc agccaagacc tgcatcatgg aggcctccac     1260
agacttccta ccaggcctga acttcagcaa ctgcagccga cgggccctgg agaaagccct     1320
cctggatgga atgggcagct gcctcttcga acggctgcct agcctacccc ctatggctgc     1380
tttctgcgga aatatgtttg tggagccggg cgagcagtgt gactgtggct tcctggatga     1440
ctgcgtcgat ccctgctgtg attctttgac ctgccagctg aggccaggtg cacagtgtgc     1500
atctgacgga ccctgttgtc aaaattgcca gctgcgcccg tctggctggc agtgtcgtcc     1560
taccagaggg gattgtgact tgcctgaatt ctgcccagga gacagctccc agtgtccccc     1620
tgatgtcagc ctaggggatg gcgagccctg cgctggcggg caagctgtgt gcatgcacgg     1680
gcgttgtgcc tcctatgccc agcagtgcca gtcactttgg ggacctggag cccagcccgc     1740
tgcgccactt tgcctccaga cagctaatac tcggggaaat gcttttggga gctgtgggcg     1800
caaccccagt ggcagttatg tgtcctgcac ccctagagat gccatttgtg ggcagctcca     1860
gtgccagaca ggtaggaccc agcctctgct gggctccatc cgggatctac tctgggagac     1920
aatagatgtg aatgggactg agctgaactg cagctgggtg cacctggacc tgggcagtga     1980
```

```
tgtggcccag cccctcctga ctctgcctgg cacagcctgt ggccctggcc tggtgtgtat     2040
agaccatcga tgccagcgtg tggatctcct gggggcacag gaatgtcgaa gcaaatgcca     2100
tggacatggg gtctgtgaca gcaacaggca ctgctactgt gaggagggct gggcaccccc     2160
tgactgcacc actcagctca aagcaaccag ctccctgacc acagggctgc tcctcagcct     2220
cctggtctta ttggtcctgg tgatgcttgg tgccagctac tggtaccgtg cccgcctgca     2280
ccagcgactc tgccagctca agggacccac ctgccagtac agggcagccc aatctggtcc     2340
ctctgaacgg ccaggacctc cgcagagggc cctgctggca cgaggcacta agtctcaggg     2400
gccagccaag cccccacccc caaggaagcc actgcctgcc gacccccagg gccggtgccc     2460
atcgggtgac ctgcccggcc caggggctgg aatcccgccc ctagtggtac cctccagacc     2520
agcgccaccg cctccgacag tgtcctcgct ctacctctga cctctccgga ggttccgctg     2580
cctccaagcc ggacttaggg cttcaagagg cgggcgtgcc ctctggagtc ccctaccatg     2640
actgaaggcg ccagagactg gcggtgtctt aagactccgg gcaccgccac gcgctgtcaa     2700
gcaacactct gcggacctgc cggcgtagtt gcagcggggg cttggggagg ggctgggggt     2760
tggacgggat tgaggaaggt ccgcacagcc tgtctctgct cagttgcaat aaacgtgaca     2820
tcttgggagc gttc                                                       2834
```

## Description

The invention relates to the field of cancer prognosis, diagnosis and therapeutics. More particularly, the invention provides compositions and methods to predict the clinical outcome and severity of triple negative breast cancer as well as to treat said cancer. The invention is based on the discovery that the rate of divergences between RNA and DNA sequences is a clear and predictive indication of the outcome of such cancer and allows the design of improved treatment regimens.

### BACKGROUND OF THE INVENTION

Worldwide, breast cancer claims 520,000 lives annually (1). The prognosis in Western countries has improved significantly with a current survival rate of 80% (2). Expression profiling (EP) studies revealed that breast cancer is a combination of various diseases (3, 4). State-of-art EP genomic tests allow therapeutic strategies to optimize projected risk/benefit trade-off (5-7).

However, the most severe form of the disease, called triple-negative breast cancer (TNBC) for its properties of being immunohistologically negative for estrogen receptors (ER), progesterone receptors (PR), and Her2/Neu receptors, is not addressed by EP.

TNBC represents 15-20% of all breast cancers, and more than 25% of TNBC patients treated pre-metastasis die within 5 years (8). Most importantly, when patients with TNBC remain disease-free for 5 years, their risk of TNBC recurrence drops, but they remain at elevated risk for other forms of breast cancer (9).

There are several manifestations of genomic instability in cancer, including the extensively documented occurrence of somatic mutations (SMs) and changes in gene expression.

Comparisons of RNA-derived sequences led the inventors to the finding that RNAs from cancer cells are more heterogeneous than those from normal cells and comparisons of cancer versus normal tissues with highly stringent bioinformatics filters have confirmed such increased sequence heterogeneity in cancer (10). The inventors have also demonstrated that the transfer of DNA information into mRNA is dramatically reduced in cancer cells, and they discovered the mechanism of Transcription Infidelity (TI) explaining the observed cancer mRNA heterogeneity (European patent EP 2 046 986 B1).

However, questions have been raised regarding whether RNA vs DNA divergences (RDD) reflect biological effects or may be due to technical limitations (e.g., sequencing errors)(11-19). Currently, no functional role of RDD has been described.

### SUMMARY OF THE INVENTION

The invention stems from the unexpected finding by the inventors that the rate of RDD is strongly correlated to the outcome of triple negative breast cancer (TNBC). The invention also stems from the identification, by the inventors, of particular target genes in TNBC cells of patients that are subject to altered RDD rate and can serve as very predictive and specific marker of TNBC outcome. Altered RDD of such target genes represents a biological characteristic of cancer and reflects cancer severity. The inventors also propose that such genes represent potent target for therapeutic treatment of TNBC.

The invention therefore provides methods for analyzing, monitoring, prognosing and/or staging of triple negative breast cancer (TNBC) based on a measure of RDD or the rate of RDD in at least one target gene. The present invention also provides methods for aiding in the diagnosis, prognosis and/or classification of the stage (for example, non-metastatic stage) of TNBC in a subject having TNBC.

A particular object of the present invention relates to a method of prognosis of TNBC in a subject, comprising measuring the rate of RDD in at least one target gene in a sample from the subject.

Another particular object of this invention relates to a method for predicting the clinical outcome of TNBC in a subject having TNBC, comprising measuring the rate of RDD in at least one target gene in a sample from said subject, the rate of RDD being indicative of the clinical outcome of said cancer. Preferably, the sample is a TNBC biopsy, or any extract or derivative of such biopsy.

Another particular object of this invention relates to a method of prognosis of survival in a subject having TNBC, comprising (i) obtaining a sample from the subject, and (ii) determining the rate of divergences between RNA and DNA sequences (RDD) in at least one target gene in said sample.

A further object of the invention relates to a method for predicting the clinical outcome of TNBC in a subject having TNBC, comprising measuring the rate of RDD in at least one target gene in a sample from said subject, wherein the clinical outcome of TNBC is the likelihood of long-term survival of said patient without the recurrence of TNBC.

In a further embodiment of this invention, the clinical outcome is the capacity of triple negative breast cancer to metastasize.

Another object of this invention relates to a method of adapting the TNBC treatment in a subject having TNBC, based on the measure of RDD rate in at least one target gene in a sample from the subject.

Another object of this invention relates to a method of reducing side effects of adjuvant therapy for patients with good prognosis, based on the measure of RDD rate in at least one target gene in a sample from the subject.

Preferably, the sample is (an extract/derivative of) a TNBC biopsy.

In a preferred embodiment, the rate of divergences between RNA and DNA sequences (RDD) is determined by measuring the number of sequence variations between a target RNA and a reference DNA sequence, preferably relative to transcript length.

Another object of this invention relates to a method, wherein the rate of divergences between RNA and DNA sequences (RDD) is measured in one or more target gene(s), and wherein a combination of RDD rate for said one or more target gene(s) is an indication of the clinical outcome of TNBC in the subject.

In a particular embodiment, the method comprises the following steps:

a) providing a sample containing RNAs encoded by said at least one target gene from the subject;
b) extracting from said sample RNAs encoded by said at least one target gene;
c) synthesizing cDNAs from RNAs of step b);
d) sequencing of the cDNAs;
e) comparing the cDNA sequences with a reference DNA sequence;
f) determining the rate of divergences between RNA and DNA sequences (RDD) as the number of sequence variations between the cDNA and the reference DNA sequences, preferably relative to transcript length.

The target genes are preferably selected from GDI2, SORBS3, POM121C, ADAM15 and UQCRHL, or a combination thereof.

A further object of this invention relates to a method of predicting the clinical outcome of TNBC in a subject having TNBC, the method comprising measuring the rate of divergences between RNA and DNA sequences (RDD) in at least GDI2 gene in a sample from said subject, wherein an elevated RDD rate for said gene is an indication of good clinical outcome in the subject.

A further object of this invention relates to a method of predicting the clinical outcome of TNBC in a subject having TNBC, the method comprising measuring the rate of divergences between RNA and DNA sequences (RDD) in at least one target gene selected from GDI2, SORBS3, POM121C, ADAM15 and UQCRHL in a sample from said subject, or in at least two of said target genes (e.g., GDI2 and SORBS3, GDI2 and POM121C, GDI2 and ADAM15, GDI2 and UQCRHL, POM121C and SORBS3, POM121C and ADAM15, POM121C and UQCRHL, etc.), wherein an elevated RDD rate for each of said at least one target gene is an indication of good clinical outcome in the subject.

A further object of this invention relates to a method of predicting the clinical outcome of TNBC in a subject having TNBC, the method comprising measuring the rate of divergences between RNA and DNA sequences (RDD) in at least three target genes selected from GDI2, SORBS3 POM121C, ADAM15 and UQCRHL in a sample from said subject, wherein an elevated RDD rate for said target genes combination (e.g., GDI2, SORBS3 and POM121C; GDI2, SORBS3 and ADAM15; GDI2, SORBS3 and UQCRHL; GDI2, POM121C and ADAM15; GDI2, POM121C and UQCRHL, etc.) is an indication of good clinical outcome in the subject.

A further object of this invention relates to a method of predicting the clinical outcome of TNBC in a subject having TNBC, the method comprising measuring the rate of divergences between RNA and DNA sequences (RDD) in at least four target genes selected from GDI2, SORBS3 POM121C, ADAM15 and UQCRHL in a sample from said subject, wherein an elevated RDD rate for said for said target genes combination (e.g., GDI2, SORBS3, POM121C and ADAM15; GDI2, SORBS3, POM121C and UQCRHL, etc.) is an indication of good clinical outcome in the subject.

A further object of this invention relates to a method of predicting the clinical outcome of TNBC in a subject having TNBC, the method comprising measuring the rate of divergences between RNA and DNA sequences (RDD) in the following five target genes: GDI2, SORBS3 POM121C, ADAM15 and UQCRHL in a sample from said subject, wherein an elevated RDD rate for said target gene combination is an indication of good clinical outcome in the subject.

The invention further relates to methods and products (such as probes, primers, antibodies or derivatives thereof), for detecting the divergences between RNA and DNA sequences in at least one target gene in a sample from the subject.

The invention further relates to methods and products for measuring the rate of divergences between RNA and DNA sequences in at least one target gene in a sample from the subject, as well as to corresponding kits.

A further object of the invention relates to a method for treating TNBC in a subject having TNBC, the method comprising (i) determining the RDD rate for at least one target gene in a sample from the subject and (ii) treating the subject according to its RDD rate.

Another object of the invention relates to a method for treating TNBC in a subject having TNBC, the method comprising administering to the subject a compound that modulates the amount or activity of a protein selected from GDI2, SORBS3, POM121C, ADAM15 and UQCRHL, or a combination thereof. Preferably, a compound that modulates the amount or activity of GDI2 is a siRNA molecule comprising a sequence of SEQ ID NO: 1, 2 or 3.

The invention may be used in any mammalian subject, particularly any human subject, to detect the predisposition to, diagnose, prognose and/or predict the severity or the outcome of triple negative breast cancer in a patient. The invention may also be used to monitor and/or treat TNBC and/or to optimize the treatment of a patient having TNBC.

## BRIEF DESCRIPTION OF THE DRAWINGS

**Figure 1:** First two components of a partial least squares (PLS) discriminant analysis of selected variables for **(A)** RDDs, **(B)** SMs, and **(C)** EP. Performance predicting good **(D)** and poor **(E)** outcomes from 30 repetitions of 10-fold cross-validation of each model.

**Figure 2:** Kaplan-Meier curves for groups defined by the output of the models for the validation set. The models were based on **(A)** RDD, **(B)** SM, (C) EP, and **(D)** tumor size (T). P values shown were obtained from log-rank tests. Non-significant KM curve for node involvement is not shown ($p = 0.89$).

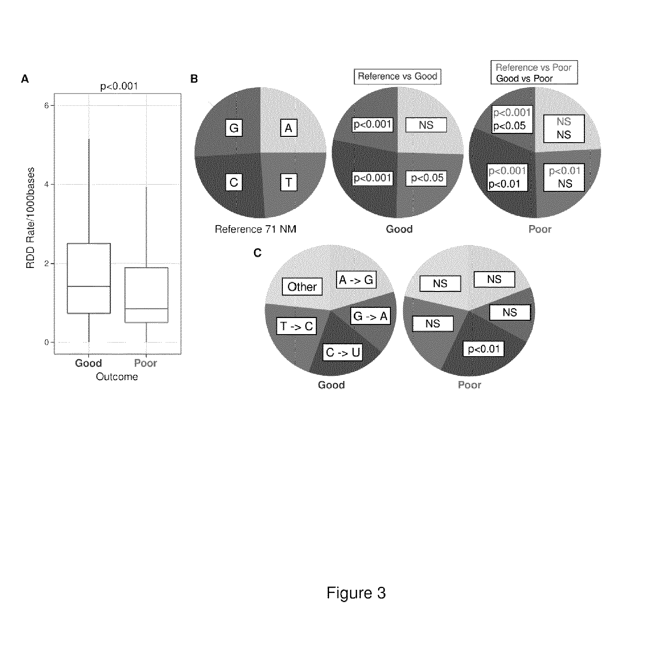

**Figure 3:** Analysis of RDDs for a panel of 71 transcripts in 52 patients separated into outcome groups according to the test results. **(A)** Boxplots of RDD rates. **(B)** Proportions of affected bases by groups. Base composition differed between the groups and versus reference set (Chi-square $p < 0.0001$). Proportions were compared with t-tests; p values for good, poor, or both outcomes are shown in blue, red, and black, respectively. (C) The proportions of specific events (A to G, G to A, C to U and T to C) were compared between the groups (Chi-square $p < 0.0001$). The other eight events constitute the "other" category. The proportions of each event type were compared with t-tests.

**Figure 4:** Application of RDD to cell-lines. **(A)** Projection of RDD rates on PLS discriminant analysis components (black line separates outcome groups). **(B)** Proliferation over 144 h did not differ between cell lines. Demonstration of cell-line differences in migration transwell (C) and invasion Matrigel-coated transwell **(D)** assays (15 count trials, 5 fields counted at 20× magnification per trial) revealed cell line differences. ***$p < 0.001$, t-test; NS, t-test not significant. Means from triplicate experiments are reported in all cases.

**Figure 5:** Effect of GDI2 expression silencing using siRNA (i.e., GDI2 siRNA-B) in migration transwell **(A)** and invasion Matrigel-coated transwell **(B)** assays (15 count trials, 5 fields counted at 20× magnification per trial). ***$p < 0.001$, t-test; *$p < 0.05$, t-test; NS, t-test not significant. Means from triplicate experiments are reported in all cases. The control siRNA was used as reference (100%) for each cell-line.

**Figure 6:** Projection of RDD rates on PLS discriminant analysis components for the 32 patients of the replication set (black line separates outcome groups).

**Figure 7** (Supplementary Table 1): Clinical data for the 52 TNBC patients and 12 non TNBC patients.

**Figure 8** (Supplementary Table 2): PLS Components of the RDD, SM and EP models.

**Figure 9** (Supplementary Table 3): 71 representative transcripts selected on the basis of their RDD rate and on their presence in model construction.

**Figure 10** (Supplementary Table 4A): Transcripts statistically significantly more expressed in the Good outcome group.

**Figure 11** (Supplementary Table 4B): Transcripts statistically significantly more expressed in the Poor outcome group.

**Figure 12:** RDD rate calculation of RDD rates for 52 TNBC patients. **(A)** Method of RDD rate calculation. **(B)** RDD rates and model prediction for 52 TNBC patients. The five first columns are RDD rates for GDI2 (NM_001494.3), UQCRHL (NM_001089591.1), ADAM15 (NM_003815.4), POM121C (NM_001099415.2) and SORBS3 (NM_005775.4). The PLS score that is calculated with RDD rates is used to predict clinical outcome (good or poor) for the 52 patients. Vital status and survival are also indicated.

DETAILED DESCRIPTION OF THE INVENTION

The present invention provides novel diagnostic, prognostic and therapeutic methods and compositions for triple negative breast cancer (TNBC).

As shown in the experimental part, the inventors have surprisingly demonstrated that RDD rate in transcripts encoded by target genes from TNBC cells represents a clear and predictable indicator of the clinical outcome of such cancers. Establishing the clinical significance of this finding is critical since it allows a better staging of cancer with respect to its severity and allows the prediction of a clinical outcome related to TNBC patients and the prognosis of disease-free survival of patients without the recurrence of TNBC.

In the present application, the inventors have shown that RDD rates are predictive of TNBC clinical outcome and may be used to design optimized treatment strategies. As illustrated in the examples, the method is more reliable than methods based on SM (somatic mutations) data or EP (expression profiling) data.

RDD

Within the context of the present invention, the term "RDD" designates RNA-DNA sequence divergence(s) or difference(s). RDD includes all possible differences between a DNA sequence and the encoded RNA sequence. RDD may include transcription errors, errors resulting from editing, transcriptional and/or post-transcriptional processing of RNA, etc. RDD also include RNA-DNA divergences resulting from yet unknown molecular mechanisms. RDD may encompass one or more differences in one or more transcripts. Such differences may be e.g., substitutions, insertions and/or deletions. Preferred RDD according to the invention are substitutions such as family base substitutions, e.g., C to U, G to A, A to G or T to C substitutions. RDD may be related to gains or losses of function of target gene(s), or a combination of both.

Within the context of the present invention, the term "RDD rate" reflects the level of RDD in a cell for at least one target gene. The RDD rate designates, for instance, the ratio between the number of RNA-DNA differences and transcript(s) length for one target gene. RDD rate according to the invention may be measured using molecular techniques known *per se* in the art such as sequencing, mass spectrometry, nucleic acid hybridization, cDNA synthesis, RT-PCR, PCR (e.g., real-time PCR), nucleic acid or protein microarrays, genomic microarrays, comparative genomic hybridization (CGH), or combination(s) thereof.

In a preferred embodiment, RDD rate is measured by high-throughput sequencing of the cDNAs synthetized from the extracted patient's RNAs. A most preferred high-throughput sequencing technique used according to the invention is next-gen sequencing technique.

RDD in TNBC

The invention is based on a measure of RDD rate in transcripts encoded by target genes in TNBC subjects, as an indication of disease outcome. In a particular embodiment, RDD rate measured in a sample from a subject having TNBC may be compared to RDD rate from another sample taken from the same subject at the same or at a different stage of the disease, or to RDD from a sample from another subject having TNBC. In another particular embodiment, RDD measured in a sample from a subject having TNBC is compared to RDD measured in one or more TNBC cell lines. TNBC cell lines used as standards may be cell lines with different *in vitro* migration and/or invasion properties. Typically, such TNBC cell lines that can be used as internal standards are selected from BT-20 (ATCC HTB-19), HCC1143 (ATCC CRL-2321), HCC1395 (ATCC CRL-2324), HCC1806 (ATCC CRL-2335), HCC1937 (ATCC CRL-2336), HCC38 (ATCC CRL-2314), Hs 578T (ATCC HTB-126), MDA-MB-231 (ATCC HTB-26), MDA-MB-436 (ATCC HTB-130), MDA-MB-453 (ATCC HTB-131) and MDA-MB-468 (ATCC HTB-132) cell lines. Preferred TNBC cell lines are BT20, MDA-MB-231 and/or MDA-MB-468. The control cell line is preferably MCF 10A (ATCC CRL-103117). In another particular embodiment, the RDD rate of a given gene is compared to a reference or mean value, wherein a rate superior to said value indicates a high or elevated RDD rate for said gene in said subject, and a rate lower than said value, indicates a low or reduced RDD rate for said gene in said subject.

Determining RDD or the rate of RDD typically comprises determining, for a given target gene, the number of sequence variations between the mRNA (or a corresponding cDNA) and a reference DNA sequence. This may be obtained by:

a) providing a sample containing RNAs encoded by said at least one target gene from the subject;
b) extracting from said sample RNAs encoded by said at least one target gene;
c) synthesizing cDNAs from RNAs of step b);
d) sequencing of the cDNAs;
e) comparing the cDNA sequences with a reference DNA sequence of said at least one target gene; and

f) determining the rate of divergences between RNA and DNA sequences (RDD), preferably by determining the number of sequence variations between the cDNA and the reference DNA sequence, preferably relative to transcript length.

The method typically comprises an initial step of providing or obtaining a sample from a subject. Typically, the sample is any biological sample of the subject to be tested, in particular any sample comprising cells from the TNBC or extracts of such cells. Examples of such samples include any TNBC biopsy sample, as well as any extract or derivative of such biopsy (e.g., a fraction thereof comprising nucleic acids, a hydrolyzate thereof, a pretreated sample, a labelled sample, etc.). Preferred samples are obtained prior to therapeutic intervention.

The sample can be obtained by any technique known per se in the art, for example, by collection using e.g., non-invasive techniques, or from collections or banks of samples, etc. The sample can in addition be pretreated to facilitate the accessibility of the target molecules, for example, by lysis (mechanical, chemical, enzymatic, etc.), purification, centrifugation, separation, etc.

In order to evaluate RDD, the samples are typically treated to extract RNAs, DNAs and/or proteins. Extraction of RNAs, DNAs and/or proteins from biological samples may be carried out by techniques known per se in the art such as, without limitation, by genetic means (e.g., nucleic acid hybridization, RT-PCR, cDNA synthesis, etc.), enzymatic techniques, chemical methods, or combinations thereof.

In a preferred embodiment, RNA is extracted from a subject's sample and then cDNA is synthesized from the extracted RNA. cDNA synthesis may be performed by any technique known per se in the art such as e.g., by RT-PCR. cDNA sequencing may then be performed by any technique known per se in the art. In a particular embodiment, the cDNA is sequenced using next-gen sequencing technique.

Comparing the cDNA sequences with a reference DNA sequence of said at least one target gene can be carried out by conventional techniques. Comparison may be performed over the entire length of the sequences, or over selected domains thereof.

In a particular embodiment the reads identified following sequencing are trimmed and aligned against the reference sequence for the target gene.

The reference sequence is, preferably, the last update of the reference genome sequence from NCBI database. The GRCh37 genome version is used as the most preferred DNA reference sequence. Examples of preferred reference sequence for the 5 preferred target genes are provided as SEQ ID NOs: 4-8, respectively.

RDD target genes in TNBC patients

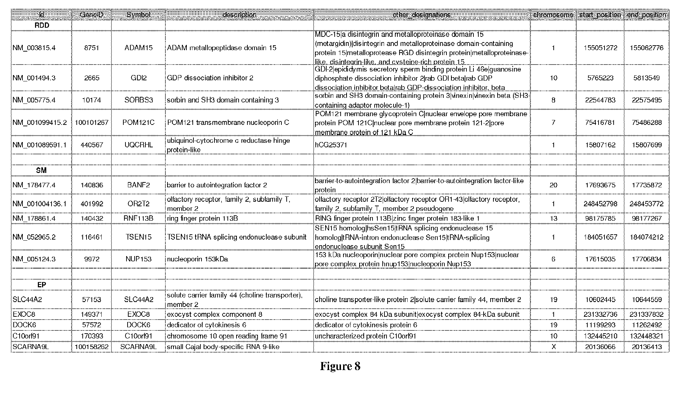

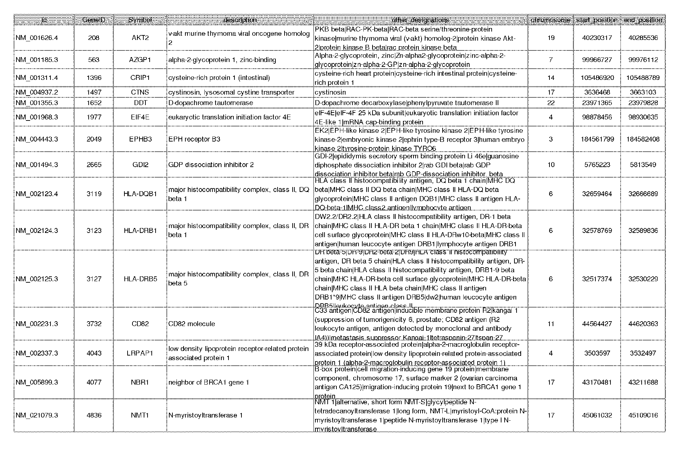

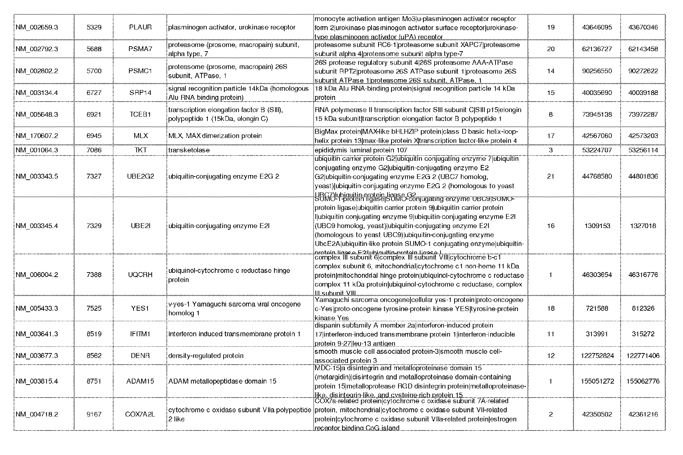

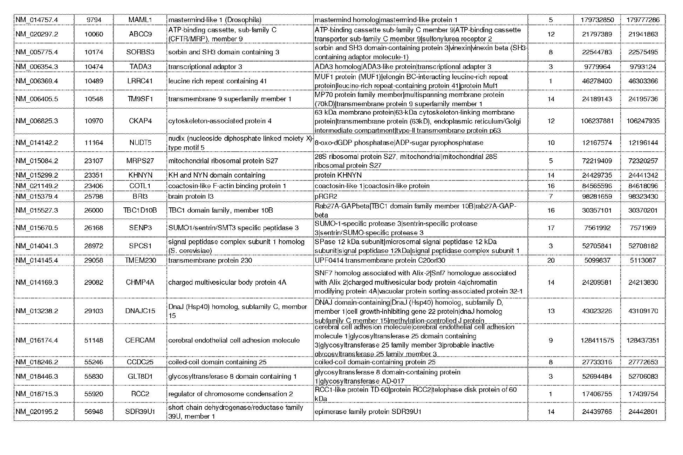

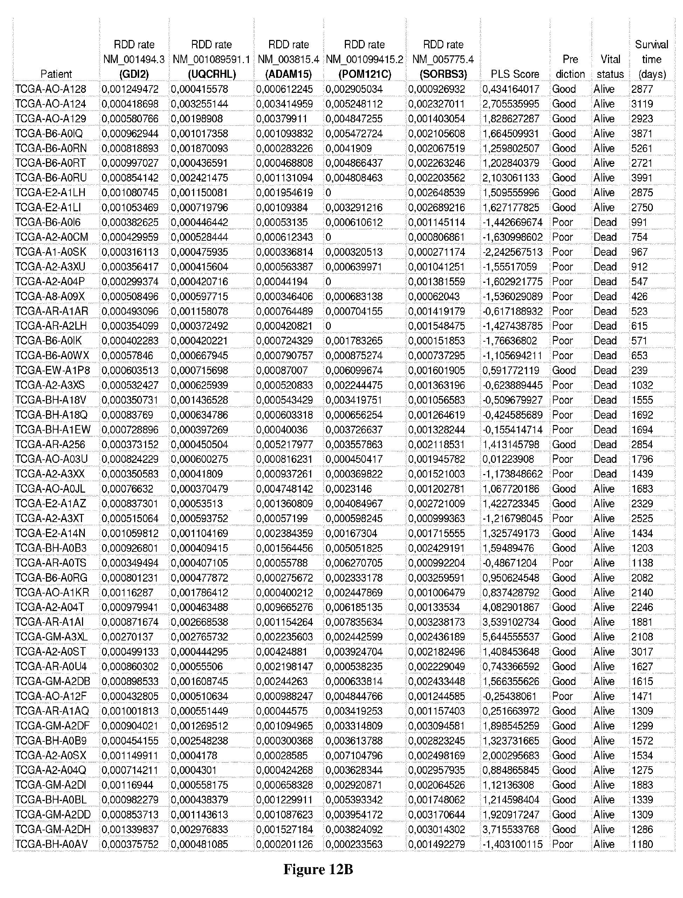

By analyzing multiple samples from TNBC, the inventors were able to identify five target genes/transcripts, GDI2, SORBS3, POM121C, ADAM15 and UQCRHL which are subject to RDD. More particularly, measuring the rate of RDD in anyone of said target genes in a TNBC sample from a human patient allows effective and reliable determination of the clinical outcome of the subject. The five transcripts that contributed to the RDD model (Fig. 8) encode the following proteins: GDI2 (rab GDP dissociation inhibitor beta), vinexin (encoded by SORBS3), ADAM15 (disintegrin and metalloproteinase domain-containing protein 15), UQCRHL (ubiquinol-cytochrome c reductase hinge protein-like), and POM121c (121-kDa pore membrane protein C). GDI2 regulates GDP-GTP exchange reactions of rab proteins involved in vesicular trafficking of molecules between cellular organelles. Vinexin is an SH3 domain-containing adaptor protein. It implicated in cytoskeletal organization, cell adhesion and migration, signaling, and gene expression. ADAM15 is an enzyme with gelatinolytic and collagenolytic activity that plays a role in wound healing. ADAM family members are type I transmembrane glycoproteins involved in cell adhesion, cellular signaling and the proteolytic processing of cytokines and adhesion molecules. The UQCRHL protein resembles its namesake UQCRH, a component of the ubiquinolcytochrome c reductase complex in the mitochondrial respiratory chain. POM121c is a component of the nuclear pore complex, which enables the transport of molecules across the nuclear envelope. These five transcripts (i.e., GDI2, UQCRHL, ADAM15, POM121C and SORBS3) of final RDD model had surprisingly a lower RDD rate in the poor outcome group. Thus, in a preferred embodiment, a decreased RDD rate is observed for 1, 2, 3, 4 or 5 of the above transcripts in patients with poor clinical outcomes, and an increased RDD rate is observed for 1, 2, 3, 4 or 5 of the above transcripts in patients with good clinical outcomes (see Figure 12B).

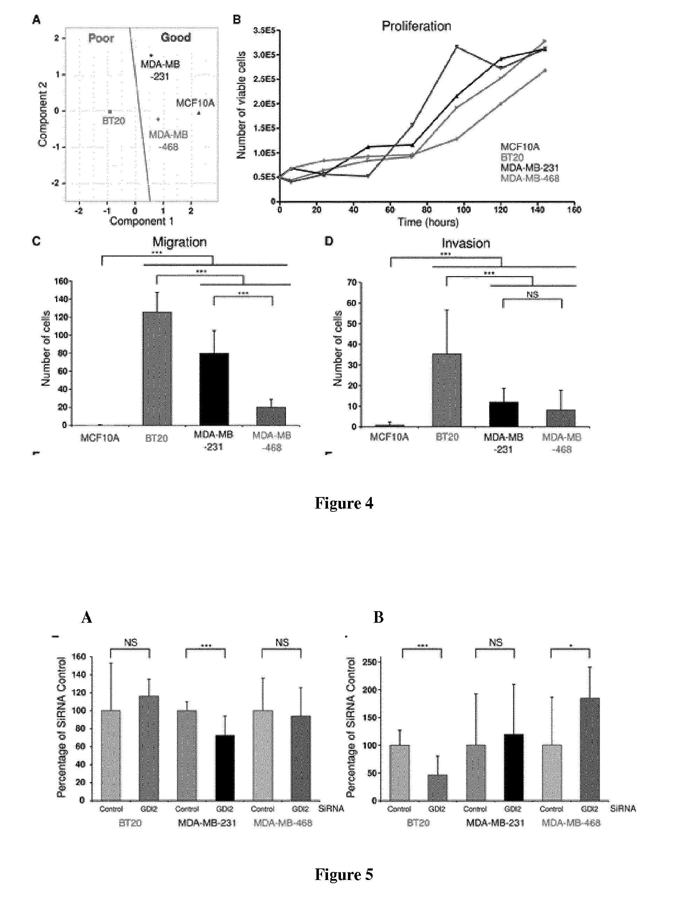

The most preferred transcript that contributes to RDD model is GDI2 transcript. Data provided by the inventors clearly demonstrate that selective silencing of GDI2 significantly modifies *in vitro* migration and invasion in a cell line specific manner (see Fig. 4). In particular embodiments, RDDs may correspond to a gain or loss of function of one or more target gene(s), or of one or more genes which are modulated (i.e., down-regulated or up-regulated) by target gene(s), or a combination of both.

RDD model predictivity of TNBC outcome

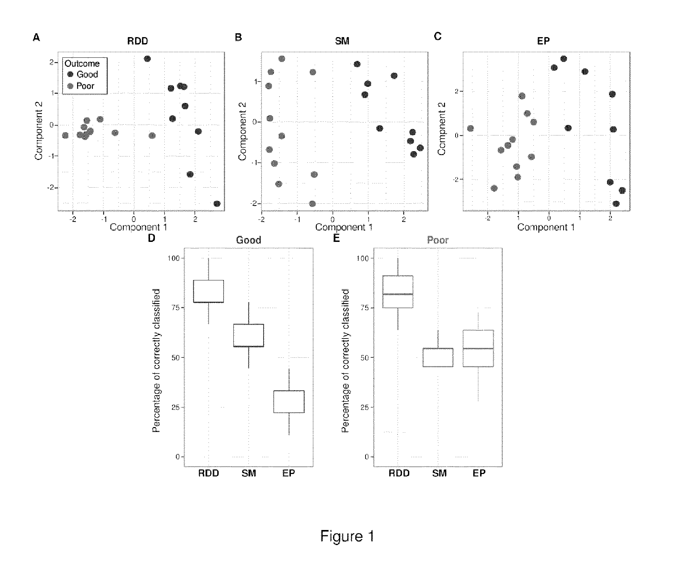

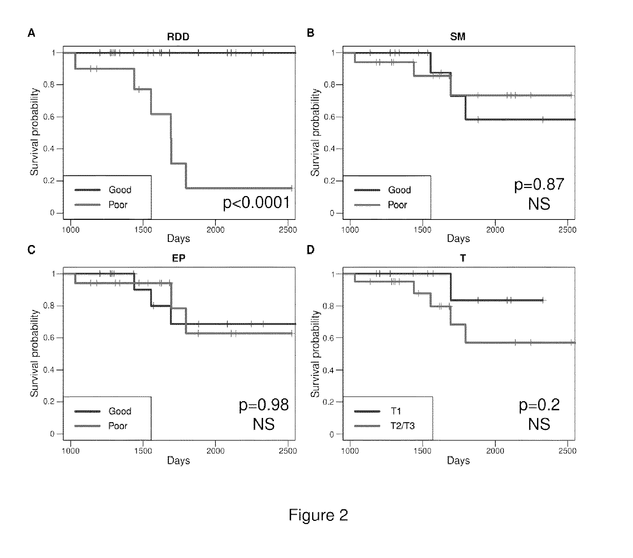

The inventors have clearly demonstrated that RDD model is highly predictive of TNBC clinical outcome while other known models using genomic information such as EP (Expression Profiling) and SM (somatic mutation) are not at all predictive of TNBC outcome. As shown in the experimental part, both the SM and EP models failed to predict patient outcome (Figures 1 and 2). The present invention shows that quantitation of divergences between TNBC patients' RNA and DNA RefSeq sequences can provide insights into individuals' TNBC-associated risks. Although African Americans are affected by TNBC at a higher rate than other populations, the inventors have verified and confirmed that the RDD model output was not related to ethnicity. The model's performance did not require knowledge of menopausal status or treatment. The model's results were validated with independent samples and not affected by recruitment center bias.

Furthermore, the method and target genes described are specific for TNBC and not affected by distinct pathologies.

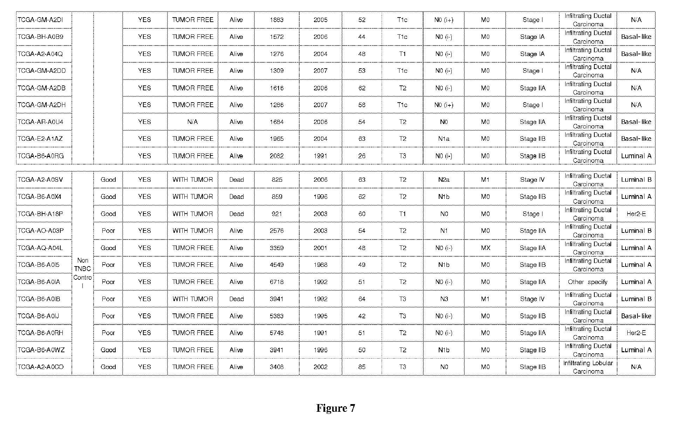

The method of the invention yielded accurate outcome classification of TNBCs of both basal-like and non-basal-like origin (Fig. 7).

The characterization of DNA changes on an individual-patient basis are investigated for medical personalization (20). However, SM data are insufficient to predict TNBC outcome. The inventors have obtained robust outcome prediction with the RDD, but not the SM, model, indicating that SMs do not provide a comprehensive recapitulation of disease complexity, perhaps due to their heterogeneity within tumors (21). Currently, EP studies are the cornerstone of tumor severity prediction. These tests were initially proposed for all breast cancers, but EP is now restricted to ER+ breast cancer where consensus on clinical utility exists (5). None of the transcripts involved in the definition of the MammaPrint and Oncotype DX signatures (22, 23) were expressed differentially in relation to outcome, nor did they contribute to the RDD rate model.

Another EP-based molecular signature involving the XBP1 gene was proposed to apply specifically to TNBC (24). The inventors have examined the expression of XBP1 and did not find a statistically significant difference between the good and poor outcome groups. KM analysis indicated that differential expression of XBP1 did not have significant prognostic value ($p = 0.07$). Finally, Ki67, associated previously with breast cancer cell proliferation (25), was not differentially expressed between the outcome groups in the present study.

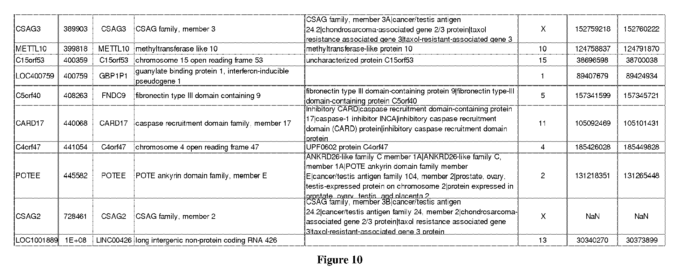

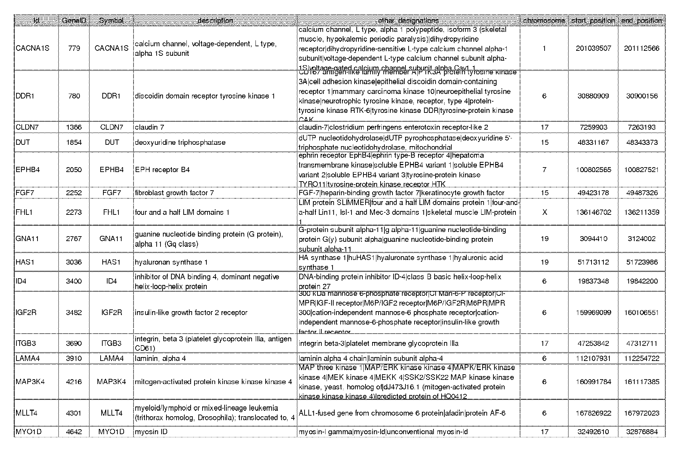

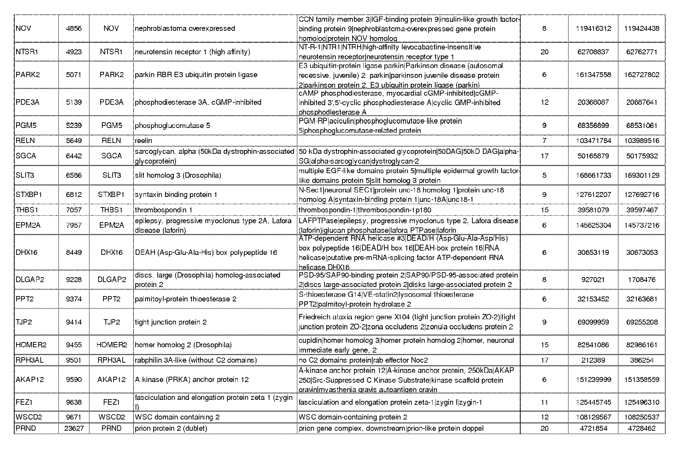

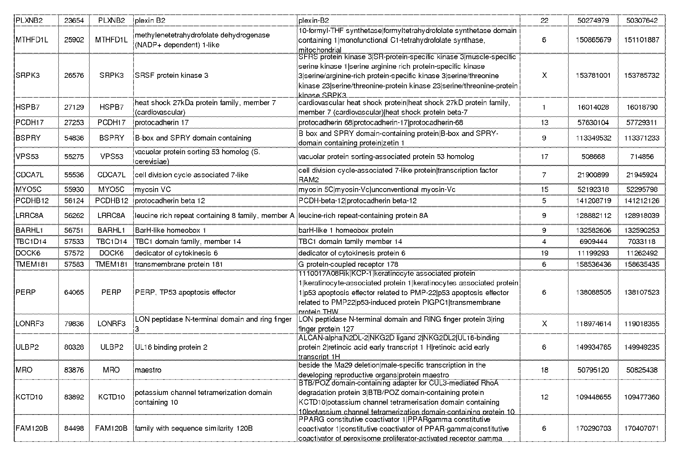

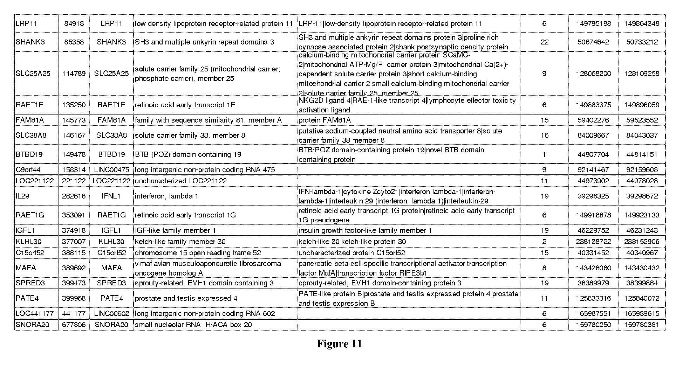

In the search for genes that were differentially expressed in relation to outcome, the inventors have found that 200 transcripts exhibited differential expression at a 1% significance threshold level (Fig. 10 and 11). This number is within the expected number of false positives, given that 20,532 transcripts were tested. Hence, combining expression data information did not result in any replicable predictive value.

The experimental results provided by the inventors clearly suggest that RDD occurrence in TNBC is associated with disease severity. Cancer severity reflects a balance between tumor cells' ability to proliferate, invade and metastasize and the immune system's ability to keep the cancer in check (26).

RDD in TNBC cell-lines

The inventors have also demonstrated that RDD model is associated with such an ability of TNBC cells to migrate, invade and metastasize. In this regard, RNAs of one control and three TNBC cell lines were sequenced and the RDD model was used to measure the RDD rate and to classify the cell lines. As shown, for example, in Fig. 4A, the control cell line, MCF10A, was classified by the RDD model as being associated with a good outcome, as were two of the TNBC lines, MDA-MB-231 and MDA-MB-468 (Fig. 4A).

The proliferation rates of these four cell lines did not differ (Fig. 4B), whereas all three TNBC cell lines had higher migration rates than the control line.

The BT20 cell line had a significantly greater migration rate than the MDA-MB-231 cell line, which itself had a greater migration rate than the MDA-MB-468 cell line (Fig. 4C). The invasion capacities of the three TNBC cell lines were greater than that of the control line (Fig. 4D) and the invasion capacity of the BT20 cell line was greater than the rates of the MDA-MB-231 and -468 cell lines, which did not differ from each other (Fig. 4D).

Hence, RDDs were associated not only with clinical outcome, but also with the capacity of TNBC cells to migrate and invade.

Therapy of TNBC

Early prognosis can spare patients with good prognoses adjuvant therapies with severe adverse effects. Furthermore, better isolation of low-survival TNBCs should accelerate clinical evaluation of novel therapeutic strategies. This is the first report to describe the use of RDD to predict the severity of a specific disease (TNBC) at the level of individual patients.

The present invention also proposes a novel therapy of TNBC based on the RDD rate. In this regard, in a particular embodiment, the invention relates to a method for treating TNBC in a subject having TNBC, the method comprising (i) determining the RDD rate for at least one target gene in a sample from the subject, as described in the present application, and (ii) treating the subject according to its RDD rate.

In another particular embodiment, the invention relates to a method for treating TNBC in a subject having TNBC, the method comprising administering to the subject a compound that modulates the amount or activity of a protein selected from GDI2, SORBS3, POM121C, ADAM 15 and UQCRHL. Such modulating compounds may up-regulate, down-regulate the amount or activity of the above proteins. The modulating agents may also completely inhibit the activity of one or more proteins encoded by target genes. The modulating compounds/agents may be selected from proteins, peptides, antibodies, inhibitory nucleic acids (e.g., antisense small interfering RNAs (siRNAs), ribozymes, etc.), synthetic molecules, etc.

In a specific embodiment, a modulating compound according to the invention is a small interfering RNA molecule that modulates the activity of GDI2, SORBS3, POM121C, ADAM15 or UQCRHL protein. Preferably, a modulating compound according to the invention is a small interfering RNA (siRNA) molecule that blocks GDI2 mRNA and inhibits the activity of GDI2 protein. For example, such an siRNA molecule of the invention may comprise one of the following sequences:

- AGAACCAAGUCAAUCGAAAGUCAGA (siRNA-B; SEQ ID NO: 1);
- AGCGCAAGAAGAAUGACAUCUAUGG (siRNA-A; SEQ ID NO: 2); or
- GAGAGGAAGCGAACAUAAAGCGGGT (siRNA-C; SEQ ID NO: 3).

In another particular embodiment, a modulating siRNA compound of the invention consists of the sequence of SEQ ID NO: 1, 2 or 3.

The present invention also relates to a composition comprising a compound that modulates the amount or activity of a protein selected from GDI2, SORBS3, POM121C, ADAM15 and UQCRHL. The compositions of the invention typically comprise one or several pharmaceutically acceptable carriers or excipients. Also, for use in the present invention, the compounds are usually mixed with pharmaceutically acceptable excipients or carriers. In this regard, a further object of this invention is a method of preparing a pharmaceutical composition, the method comprising mixing the above compounds in an appropriate excipient or carrier.

The invention may also be used to monitor and/or treat TNBC and/or to optimize the treatment of a patient having TNBC (including the treatment by chemotherapy, radiotherapy, adjuvant therapy or a combination thereof).

Further aspects and advantages of the invention will be disclosed in the following experimental section, which illustrates the invention and does not limit the scope of the present application.

## EXAMPLES

## MATERIELS AND METHODS

### A. Patient characteristics

Access to The Cancer Genome Atlas (TCGA) controlled-access data was approved by the TCGA data access committee. All patient specimens were obtained with consent from the relevant institutional review board. Next-generation RNA and DNA sequencing data available through the TCGA portal were downloaded from CGHub in BAM format, and clinical information was used to select non-metastatic triple-negative breast cancer (TNBC) patients. Only patients who were ≤65 years old when their cancer was diagnosed were included. The training set consisted of patients with extremely divergent survival data including 11 patients with a poor clinical outcome (death within 1000 days of diagnosis) and 9 patients with a good clinical outcome (survival for more than 2500 days after diagnosis). All other TNBC patients whose negative Her2/Neu status was validated based on ERBB2 expression, available clinical data (duplication status and/or immunochemistry receptor status), or annotations from published scientific literature (3), were included in the validation set (N = 32). Data from patients with non-TNBC disease were also downloaded (N = 12). Samples were received from 10 different centers, 5 of which contributed to both the training and validation sets. RNA and DNA were extracted from primary solid tumors obtained prior to therapeutic interventions. All of the sequencing was performed using the same Illumina platform (HiSeq 2000, Illumina, San Diego, CA).

B. Sequencing

The rate of RDD was measured by sequencing the cDNAs synthetized from the extracted patient's RNAs using next-gen sequencing technique also called "next-gen sequencing" or "NGS", which is a novel high-throughput sequencing technique based on the following concept: the bases of a small fragment of DNA are sequentially identified from signals emitted as each fragment is re-synthesized from a DNA template strand, and NGS extends this process across millions of reactions in a massively parallel way, thus allowing rapid sequencing of huge quantities of DNA base pairs. When NGS technique is used, a single DNA is first fragmented into a library of small fragments that can be uniformly and accurately sequenced in millions of parallel reactions. The newly identified strings of bases are called "reads".

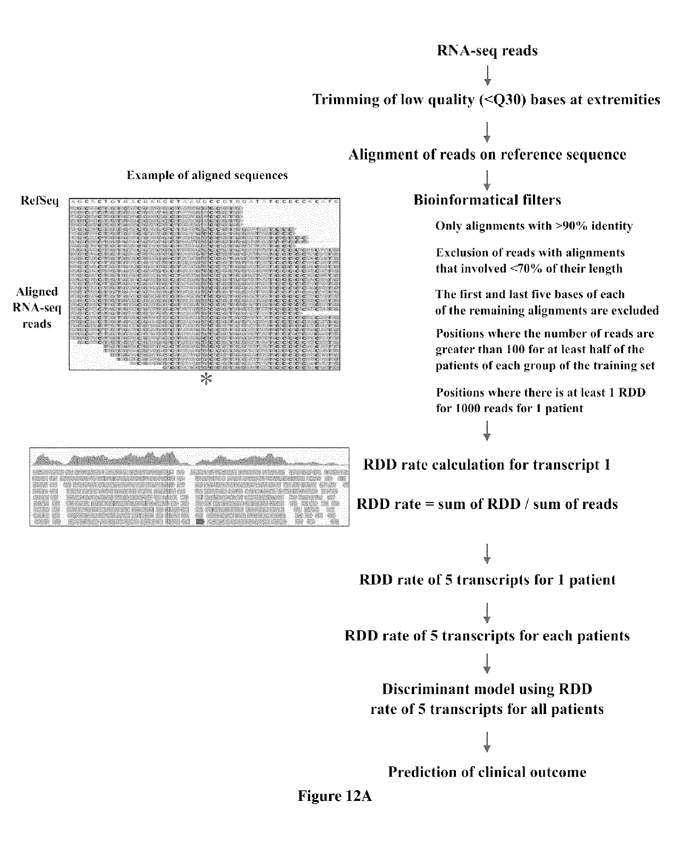

The downloaded BAM sequence files were converted into FASTQ files using SAMtools to obtain the RNA-seq reads (Figure 12A). The reads were trimmed and low-quality extremities were eliminated according to a quality threshold (Q = 30). The trimmed reads were aligned against reference sequences using the Burrows-Wheeler Alignment tool (BWA 0.5.8c) (27). The optimal alignment parameters were determined by benchmarking the performance of the tool (using a procedure with 1000 iterations) to identify differences between the sequences (l = 50, n = 10,000) and the reference. Perfectly matched sequences were modified using a random number (≤5) of all three types of mismatch events (substitution, deletion, and insertion) and were aligned relative to the reference. Different parameter values were tested and global performances were calculated, counting the number of mapped sequences and the portion of differences that were properly identified. The parameter values that yielded the best performances were conserved. RNA reference sequences were created from exon sequences in the reference genome (NCBI GRCh37). The exons were joined to create transcript reference sequences for each isoform of all known and predicted genes. The GRCh37 genome version was used as a DNA reference sequence.

*Data filtering:*

As shown in Figure 12A, only alignments with >90% identity, and, if multiple alignment on the reference exist, only best scores were assigned. Subsequently, reads with alignments that involved <70% of their length were excluded. The first and last five bases of each of the remaining alignments were excluded.

C. Gene expression analysis

The RNA-Seq gene expression level-3 data in the TCGA contains RNA-Seq data calculated with the Expectation-Maximization (RSEM) algorithm, which is based on a generative probabilistic model of maximum expectation (28). The available gene expression data for the 52 patients (analyzed with RSEM) were downloaded from the TCGA portal. Normalized gene expression data were used.

D. RDD (RNA-DNA difference) rate calculation

The example of RDD rate calculation is illustrated, for instance, in Figure 12A. The filtered alignments were parsed to quantify the number of reads that were identical to the reference sequence. The number of reads that deviated from the reference sequence at any position was also identified. Different filters were applied to improve the quality of the alignments (figure 12A) : a) only positions with number of reads greater than 100 for at least half of the patients of each group of the training set were considered (considered a necessary quality standard for a position); b) Only positions at which at least one patient has an error rate higher than the machine error (1 error for 1000 reads) are retained. The RDD rate was calculated as the sum of RDD at all the selected positions divided by the number of reads at all the selected positions of the transcript as shown below:

$$\mathrm{RDDrate}\,(\mathrm{transcript}_i) = \frac{\Sigma\, \mathrm{NbDivergence}_{\mathrm{position}}}{\Sigma\, \mathrm{NbReads}_{\mathrm{position}}}$$

The RDD rate can be computed according to event type (divergence = substitution, insertion, or deletion). The SM rate was calculated with the same method applied to DNA data.

E. Variable selection and model construction

Variables of interest were selected based on a sparse PLS discriminant analysis (sPLS-DA) procedure (29). Two strategies were used: (1) sPLS-DA directly on the training set; and (2) bootstraping with selection of a "bootstrap sample" obtained by random selection of a new set of patients in the training set with replacements and selection of variables by applying sPLS-DA to the bootstrap sample. This selection procedure was repeated several times, and the variables that appeared most frequently were retained. Three variables were selected from each strategy (with some redundancy in the selected variables in the two strategies), limiting the variable set to a maximum of six. A PLS-DA model was then constructed using the selected set of variables. Subsequent assignment of each new patient to a group was based entirely on the values of the selected variables. RDD rate computation, variable selection, and model construction were performed with R version 3.0.2 (30) and the mixOmics package (31).

F. Cell lines and culture

MCF10A, BT20, MDA-MB-231, and MDA-MB-468 cell lines were obtained from ATCC (LGC, Molsheim, France). MCF10A cells were grown in MEGM medium (LONZA, Basel, Swiss) containing 5% bovine pituitary extract, 10 μg/ml insulin, 0.5 μg/ml hydrocortisone, 50 μg/ml gentamicin/amphotericin B, 20 ng/ml epidermal growth factor, and 100 ng/ml cholera toxin (Servibio, Courtaboeuf, France). BT20 cells were maintained in DMEM medium (Dutscher, Brumath, France) supplemented with 10% fetal bovine serum (Dutscher) and 10,000 U of penicillin/streptomycin (Dutscher). The MDA-MB-231 and MDA-MB-468 cells were grown in Leibovitz medium (Dutscher) supplemented with 10% fetal bovine serum and 10,000 U of penicillin/streptomycin.

G. Proliferation assay

A total of 50,000 cells were seeded into a 24-well plate. Cell viability was determined every 24 h using a Trypan blue (Sigma-Aldrich, St. Quentin Fallavier, France) exclusion assay with a Thoma cell-counting chamber. These experiments were performed in triplicate, and the results were averaged.

H. Migration and invasion assays

*In vitro* migration and invasion assays were performed as described by Ma et al. (32) with 24-well modified Boyden chamber transwells with a PET (polyethylene terephthalate) membrane containing 8 μm pores (Falcon by Dutscher). Uncoated membranes were used for the migration assay, and Matrigel-coated membranes were used for the invasion assay (1/4 Matrigel and 3/4 serum-free medium, BD Bioscience, Le Pont De Claix, France). The upper chamber was seeded with 50,000 cells in 100 μl of serum-free medium, and 750 μl of supplemented medium specific for each cell line (see cell lines and culture section above) was added to the lower chamber. Cells were incubated at 37 °C for 24 h, rinsed with phosphate buffered saline, fixed in 4% paraformaldehyde for 2 min, rinsed with phosphate buffered saline, and permeabilized in methanol for 20 min, and stained with DAPI. The cells that remained in the upper chamber were removed mechanically with a cotton swab. The cells that passed through the membrane were counted (5 fields per transwell at 20× magnification). The transwell assays were performed in triplicate, and the results were averaged.

I. GDI2 RNA Silencing

GDI2-targeted siRNA was purchased at Origene (GDI2 Trilencer-27 Human siRNA, Clinisciences, Nanterre, France). The siRNA molecules of SEQ ID NO: 1, 2 and 3 were transfected into BT20, MDA-MB-231 and MDA-MB-468 cell lines with Polyplus Transfection Interferin transfecting agent (Ozyme, Saint Quentin En Yvelines, France). As recommended by the manufacturer, 20 μl of interferin was combined with the siRNA in serum free medium (final concentration 10 nM), vortexed for 10 s, and incubated for 10 min at RT before being applied to cells seeded in a large flask (25cm$^2$ base area). After 72h, the treated cells were trypsynated, harvested and counted after application of a trypan blue exclusion assay with a Thoma cell-counting chamber. The cells were then seeded to perform migration and invasion assays as described above.

J. GDI2 mRNA expression analysis by qRT-PCR

Real-time PCR was performed to confirm silencing of GDI2 expression. Total RNA samples were extracted from BT20, MDA-MB-231 and MDA-MB-468 cell lines (100,000 cells) that had been transfected with siRNA with the RNeasy Plus Mini kit (Qiagen, Courtaboeuf, France). Total RNA (400 ng) were reverse transcribed using the superscript II enzyme (Fisher Scientific, Illkirch, France) and random hexamers. qPCR was carried out by the 7500 PCR system (Applied biosystems, Courtaboeuf, France) in a 96-well plate format with SYBR Green JumpStart Taq Ready Mix (Sigma-Aldrich, Saint Quentin Falavier, France). The $2^{-\Delta\Delta Ct}$ method was used for quantitative analysis of RNA expression. HPRT, B2M and POLR2A RNA expression were used as references standards.

K. Statistical analysis of GDI2 mRNA expression

Transcription data were analyzed with R software and the SlqPCR package (33). As the first step, the inventors verified that the HPRT, B2M and POLR2A RNAs were suitable reference standards based on their stability measure M (selectHKgenes function). The inventors normalized the data by geometric averaging of the three reference RNAs using the normPCR function. The percentage of silencing was calculated by determining the mean RNA expression obtained for each cell line without siRNA and for each cell line transfected with control siRNA. The extent of siRNA(SEQ ID NO: 1)-induced silencing of GDI2 expression was 94% for BT20 cells, 84% for MDA-MB-231 cells, and 90% for MDA-MB-468 cells.

**RESULTS**

Expression analysis of good versus poor outcome groups

The training outcome-polarized set consisted of 20 patients, ≤65 years old, diagnosed with TNBC of basal-like origin or non-basal like origin; all were free of metastasis. The cohort was divided into two outcome groups. The good outcome group included 9 patients who were alive after 2500 days (6.8 y) of follow-up, and the poor outcome group included 11 patients who died from TNBC within 1000 days (2.7 y) of diagnosis. Table 1 below summarizes the clinical characteristics of the patients in the training set (for individual clinical data, see Fig. 7). The inventors did not consider treatment or menopausal status in the patient selection process.

**Table 1:** Clinical and pathological characteristics of the cohort of 52 patients

| | **Training set (N = 20)** | | **Replication set (N = 32)** | |
|---|---|---|---|---|
| **Outcome** | **Good** | **Poor** | **Predicted Good** | **Predicted Poor** |
| **Age at initial pathologic diagnosis** | | | | |
| Mean | 48 | 49 | 50 | 46 |
| Median | 49 | 50 | 52 | 47 |
| Min | 29 | 35 | 26 | 31 |
| Max | 61 | 63 | 63 | 62 |
| **Vital status** | | | | |
| Alive | 9 | 0 | 21 | 5 |
| Dead | 0 | 11 | 1 | 5 |
| **Days to last followup or days to death** | | | | |
| Mean | 3377 | 655 | 1635 | 1496 |
| Median | 2923 | 616 | 1594 | 1514 |
| Min | 2721 | 239 | 1203 | 1032 |
| Max | 5261 | 991 | 2854 | 2238 |
| **Pathologic T** | | | | |
| T1 | 3 | 2 | 9 | 3 |
| T2 | 4 | 6 | 12 | 7 |
| T3 | 2 | 2 | 1 | 0 |
| T4 | 0 | 1 | 0 | 0 |
| **Pathologic stage** | | | | |
| Stage I | 3 | 0 | 7 | 2 |
| Stage II | 4 | 4 | 14 | 7 |
| Stage III | 2 | 7 | 1 | 1 |
| **Pathologic N** | | | | |
| N0 | 5 | 2 | 16 | 4 |
| N1 | 4 | 4 | 5 | 5 |
| N2 | 0 | 1 | 1 | 1 |
| N3 | 0 | 4 | 0 | 0 |

(continued)

| | | | | |
|---|---|---|---|---|
| **Pathologic M** | | | | |
| M0 | 9 | 11 | 22 | 10 |

The inventors compared how well three sources of genomic information, namely RDD rate, SM rate, and EP, differentiated between patients in relation to disease outcome. RDD rates were calculated by identifying variations in RNA sequences based on a comparison to reference sequences (i.e., RefSeq sequences), and expressed as the number of substitutions per unit sequencing reads. SMs were identified by comparing the patients' cancer cells DNA sequences to RefSeq sequences. Therefore, alterations confirmed as SMs but mistakenly defined as RDDs were also included in the SM analysis. The abundance of RNA transcripts was estimated with the RSEM algorithm. As shown in Fig. 1, good outcome cases could be separated from poor outcome cases by RDD- (A), SM- (B), or EP-(C) based genomic analysis. The same supervised statistical variable selection algorithm was used for all 3 sources of genomic data. Cross-validation led to a reduction in the statistical performance of the SM and EP models, but not the RDD model, which predicted outcome for >80% of patients correctly after cross-validation (Fig. 1D and E).

With a very large genomic dataset, two groups of clinically distinct patients were separated with good reliability, albeit with the risks of data over-fitting and model instability. These risks cannot be attributed to too many variables being included given that each model was limited to five transcripts. There was no overlap between the transcripts selected for the 3 different models (Fig. 8). Therefore, the lack of robustness of the SM and EP models resulted from the selection of parameters that were relevant to the individuals in the training set, but did not reflect general and transposable features of the disease. The robustness of the RDD model following cross-validation suggests that the RDD approach should be applicable to other patients.

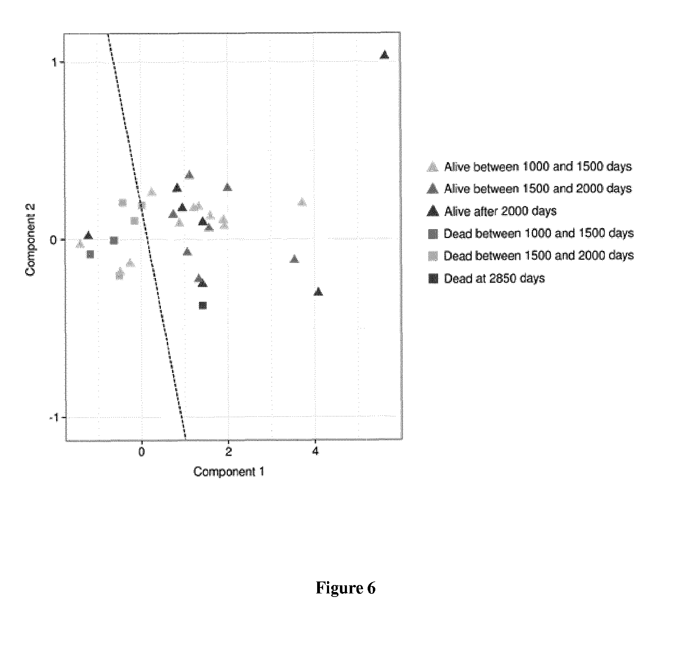

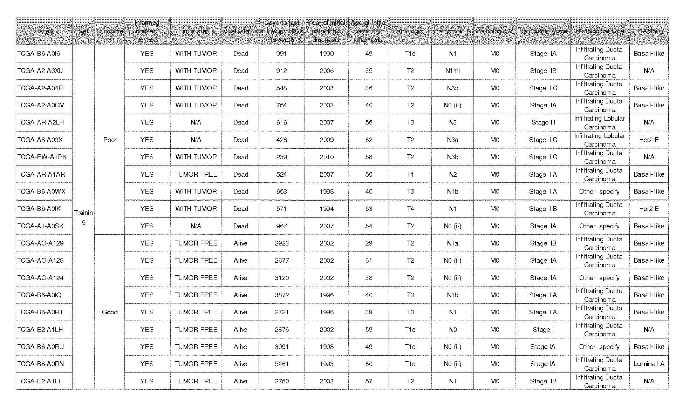

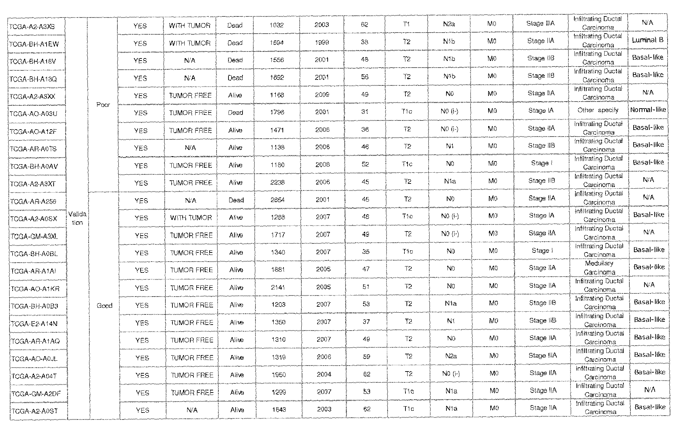

Data from a second group of TNBC patients who met the follow-up criteria (specified herein by the inventors) were retrieved. The clinical characteristics of the 32 patients included in this replication set are summarized in Table 1 (for individual clinical data, see Fig. 7). The three models that discriminated between the first set of patients (Fig. 1) were applied to data from the second group without changing any parameters. A Kaplan-Meier (KM) analysis showed that the RDD model was highly predictive of clinical outcome ($p < 0.0001$) (Fig. 2A). Another representation of the 32 patients of the replication set classified with the RDD model as shown in Fig. 1, is shown in Fig. 6. In agreement with the results of the first statistical analysis (Fig. 1D), both the SM and EP models failed to predict patient outcome (Fig. 2B and C). The predictive value of tumor size and positive lymph nodes were also assessed by the inventors. Larger tumors tended to be associated with a poorer outcome (Fig. 2D), although this finding did not reach statistical significance ($p = 0.20$). Lymph node positivity or negativity was not informative ($p = 0.80$, KM analysis not shown).

Differences between recruitment centers should not be a concern in the present study. The samples originated from 10 centers, which contributed 5 samples each on average (range, 1-10). Most importantly, the replication set included samples from five centers that were not sources for the training set.

We further investigated the predictive value of the TNBC RDD model for ER+ breast cancers (see Fig. 7 for clinical characteristics).

With the same survival criteria for outcome as defined above (defining a good outcome as survival after 2500 days of follow-up and a poor outcome as death within 1000 days after diagnosis), the TNBC RDD model failed when applied to ER+ breast cancer cases: 3 out of 4 poor outcome patients and 5 out of 8 good outcome patients with ER+ breast cancer were classified erroneously. Therefore, this RDD model is specific for TNBC cancers.

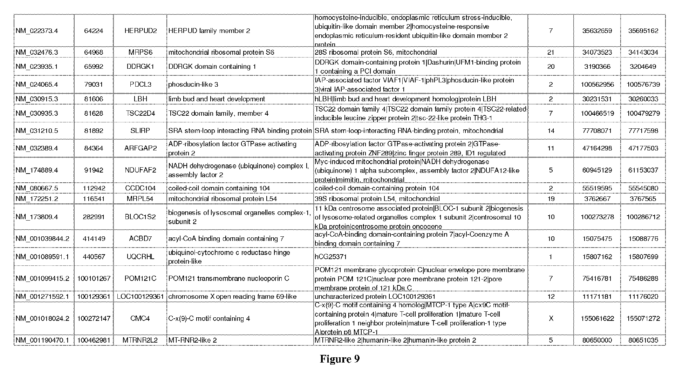

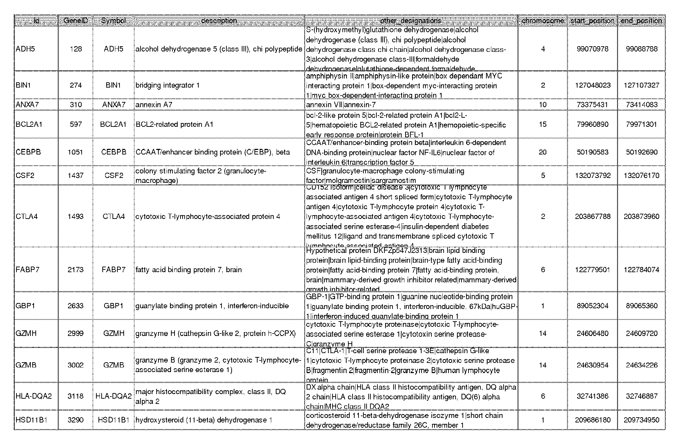

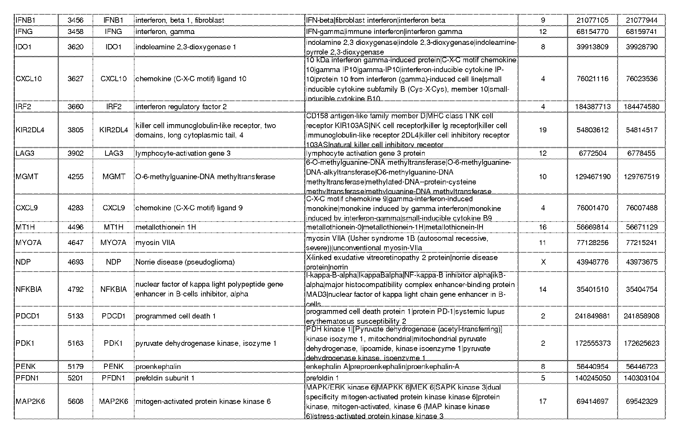

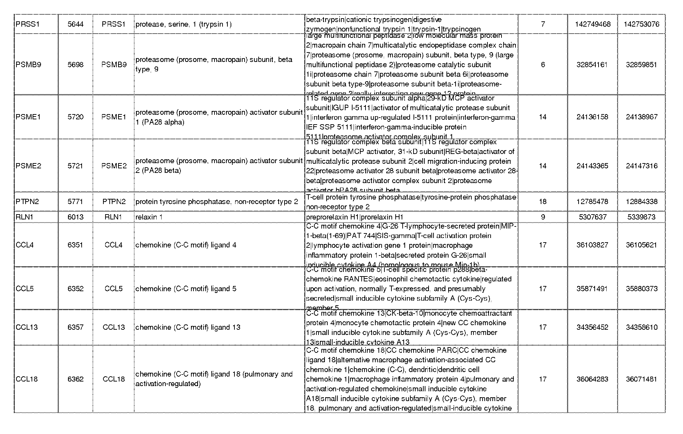

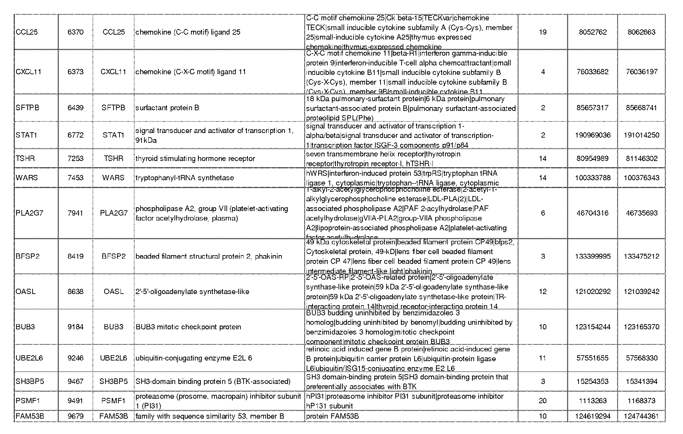

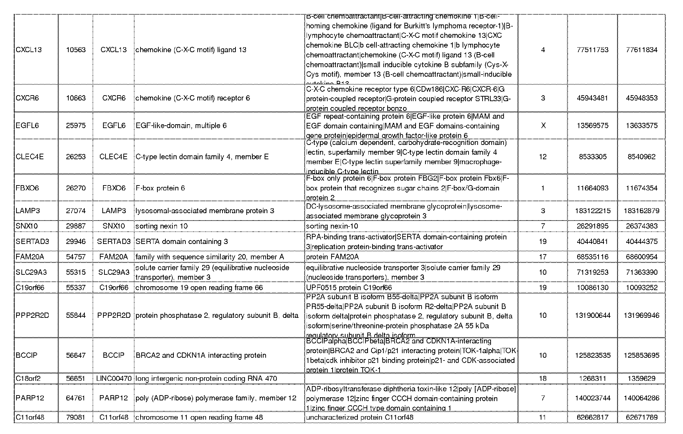

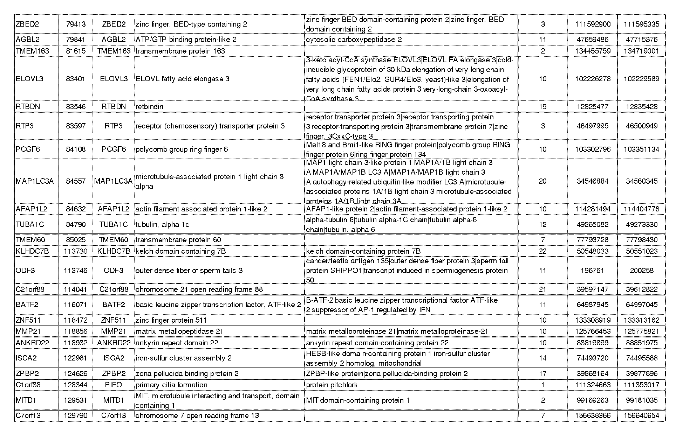

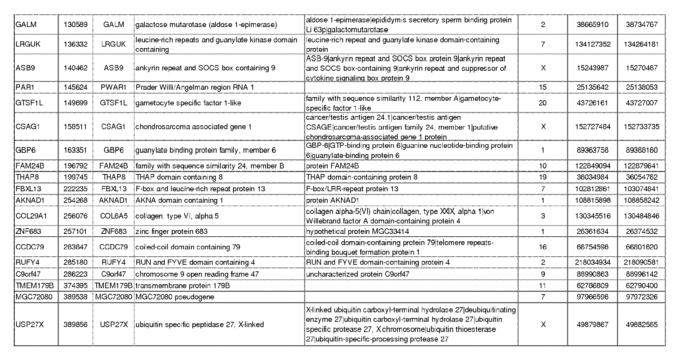

To characterize the RDD substitutions associated with TNBC severity, the inventors have combined patients from both the training and replication sets and focused on a panel of 71 representative transcripts (Fig. 9). These transcripts were selected on the basis of two criteria: 1) their RDD rates differed between the outcome groups in the training set, and 2) they were identified at least once by the iterative variable selection procedure that led to the five transcripts constituting the final model.

The overall RDD rate for the panel was significantly higher for patients with good outcomes than for those with poor outcomes (Fig. 3A and Fig. 12B). For example, Figure 12B lists RDD rates measured by the inventors for the main five transcripts (i.e., GDI2, UQCRHL, ADAM15, POM121C and SORBS3) in 52 TNBC patients. The measured RDD rates for GDI2 (NM_001494.3), UQCRHL (NM_001089591.1), ADAM15 (NM_003815.4), POM121C (NM_001099415.2) and SORBS3 (NM_005775.4) were used to calculate the PLS score which was then used to predict clinical outcome (good or poor) for the patients.

In this particular example of Fig. 12B, PLS scores based on RDD rates were calculated using the following formula having a different factor for each target gene RDD rate:

$$\text{Score} = 0.533563 * (\text{RDD_NM_001494.3} - 0.000637)/0.000298 + 0.39234 * (\text{RDD_NM_001089591.1} - 0.000975)/0.000801 + 0.3412 * (\text{RDD_NM_003815.4} - 0.001013)/0.000969 + 0.445596 * (\text{RDD_NM_001099415.2} - 0.002367)/0.002242 + 0.496394 * (\text{RDD_NM_005775.4} - 0.001468)/0.000762.$$

The PLSDA model using RDD rate of five transcripts (Fig. 12A) has allowed the inventors to conclude that RDD rate is clearly lower in the poor outcome group (shown as negative RDD Score values in Fig. 12B) while RDD score is higher in the good outcome group (shown as positive RDD score values of Figure 12B).

According to these results, RDD occurrence in TNBC is negatively associated with disease severity. For the five transcripts used in the final model, the RDD rate was 2 errors/1000 read bases for the good outcome group and 0.9 errors/1000 read bases for the poor outcome group ($p < 0.001$). Furthermore, not all bases were equally susceptible to RDDs (Fig. 3B). For the reference set, the pie-chart represents the proportion of bases in the 71 transcript sequences. In the poor and good outcome groups, RDDs affected C in excess, whereas G and T were less affected (Fig. 3B). The most striking group difference was a significant excess of C bases being affected in the poor outcome group. The G base was also less affected in the poor outcome group. Four base substitution event types (G to A, A to G, C to U, and T to C) were prominent (Fig. 3C). Replacement of C with U occurred with greater frequency in the poor outcome group than in the good outcome group. This asymmetry indicates that the RDD events observed were not random. They affected specific bases, which were preferentially replaced by bases from the same family. This information was generated retrospectively and did not contribute to the construction of the RDD model. These findings are nevertheless indicative of underlying mechanisms and their consideration may contribute to further increase in the robustness of RDD-based tests.

In the present application, the inventors have surprisingly found that the most severe forms of TNBC are associated with a lower RDD rate and a distortion in the proportions of affected bases. The inventors have demonstrated that the replacement bases found to affect transcripts with differential RDD rates between the outcome groups were preferably within the same base family.

Notably, C-to-U transitions were ~2-fold more common in patients with a poor outcome, suggesting that mechanisms that recognize and substitute specific bases are involved in RDD.

In light of the strong association demonstrated by the inventors between RDD rate and TNBC severity, the molecular mechanisms through which RDDs contribute directly to or associate indirectly with TNBC severity are presently studied by the inventors.

<u>RDD model applied to TNBC cell lines</u>

To evaluate the RDD model on a cellular level, RNAs of one control and three TNBC cell lines were sequenced. The RDD model was used to measure the RDD rate and to classify the cell lines. As shown in Fig. 4A, the control cell line, MCF10A, was classified by the RDD model as being associated with a good outcome, as were two of the TNBC lines, MDA-MB-231 and MDA-MB-468 (Fig. 4A). Only the BT20 TNBC cell line was classified as being associated with a poor outcome.

The proliferation rates measured for these four cell lines did not differ (Fig. 4B) whereas all three TNBC cell lines had higher migration rates than the control line. The BT20 cell line had a significantly greater migration rate than the MDA-MB-231 cell line, which itself had a greater migration rate than the MDA-MB-468 cell line (Fig. 4C). The invasion capacities of the three TNBC cell lines were greater than that of the control line and the invasion capacity of the BT20 cell line was greater than the rates of the MDA-MB-231 and -468 cell lines, which did not differ from each other (Fig. 4D). Hence, RDDs were associated not only with clinical outcome, but also with the capacity of TNBC cells to migrate and invade.

We next questioned whether the observed relationship between TNBC cell capacity to invade and migrate and the RDD model classification reflected only an association.

Expression of GDI2 mRNA (the most important and central transcript in the RDD model) was selectively silenced by >80% using siRNA. As shown in Fig. 5A and B, suppression of GDI2 expression modified the behavior of cell lines classified as being associated with poor versus good outcome differentially in a statistically significant manner. MDA-MB-231 had a reduced migration capacity, whereas MDA-MB-468 had an elevated invasion capacity. In contrast, BT20 show reduced invasion capacity. Therefore, a direct relationship exists between GDI2 expression and TNBC cell capacity to invade or migrate.

TNBC treatment using GDI2 siRNA

To evaluate the possibility of treating TNBC with GDI2 modulating agents, the activity of the following GDI2-siRNA molecules was tested on the activity of GDI2 protein: siRNA-B of SEQ ID NO: 1 siRNA-A of SEQ ID NO: 2 and siRNA-C of SEQ ID NO: 3.

As described above, the siRNA molecules of SEQ ID NO: 1, 2 and 3 were transfected into several TNBC cell lines (i.e., BT20, MDA-MB-231 and MDA-MB-468). After the treatment with GDI2-siRNA, total RNA samples were extracted from BT20, MDA-MB-231 and MDA-MB-468 cell lines, and GDI2 expression was analyzed by RT-PCR to confirm silencing of GDI2 expression. The percentage of GDI2 silencing was calculated by determining the mean RNA expression obtained for each cell line without siRNA and for each cell line transfected with control siRNA. The inventors have found that all the tested siRNAs allow to significantly inhibit the expression of GDI2 protein. For example, as shown in Figure 5, the percentage of GDI2 silencing induced by siRNA of SEQ ID NO: 1, was respectively 94% for BT20 cells, 84% for MDA-MB-231 cells, and 90% for MDA-MB-468 cells.

It is therefore possible to treat TNBC in a subject having TNBC with a method comprising administering to the subject a GDI2 siRNA molecule blocking the activity of a GDI2 protein.

Conclusion

The invention clearly shows that RDD occurrence in TNBC is associated with the clinical outcome of the disease as well as with the capacity of TNBC cells to migrate and invade. The invention also identifies particular target genes in TNBC that can be used as predictive and specific markers of TNBC outcome, i.e., GDI2, SORBS3, POM121C, ADAM15, UQCRHL, or a combination thereof. In particular, the inventors have discovered that RDD rate is higher for target genes in the good clinical outcome group as compared to the poor clinical outcome group. The inventors have also proposed that such genes represent potent targets for therapeutic treatment of TNBC. In particular, a crucial role of GDI2 in the RDD model has been demonstrated, and treating TNBC with GDI2 modulating agents (such as siRNAs) has been proposed. The inventors have provided useful internal standards by identifying TNBC cell lines with different *in vitro* migration and invasion properties and differentially classified by the RDD model. The inventors have also shown that RDD model is repeatable when such stringent sequencing technology controls were applied.

BIBLIOGRAPHY

1. Cancer Research UK (2014) Worldwide cancer mortality statistics. Available at: http://www.cancerresearchuk.org/cancer-info/cancerstats/world/mortality/ [Accessed June 11, 2014].

2. Coleman MP et al. (2008) Cancer survival in five continents: a worldwide population-based study (CONCORD). Lancet Oncol 9:730-756.

3. The Cancer Genome Atlas Network (2012) Comprehensive molecular portraits of human breast tumors. Nature 490:61-70.

4. Perou CM et al. (2000) Molecular portraits of human breast tumours. Nature 406:747-752.

5. Azim HA Jr et al. (2013) Utility of prognostic genomic tests in breast cancer practice: The IMPAKT 2012 Working Group Consensus Statement. Ann Oncol Off J Eur Soc Med Oncol ESMO 24:647-654.

6. Symmans WF (2012) A perspective on genomic tests for breast cancer: the need for progress. Oncol Williston Park N 26:364-365, 369.

7. Hornberger J et al. (2012) Clinical validity/utility, change in practice patterns, and economic implications of risk stratifiers to predict outcomes for early-stage breast cancer: a systematic review. J Natl Cancer Inst 104:1068-1079.

8. De Ruijter TC, Veeck J, de Hoon JPJ, van Engeland M, Tjan-Heijnen VC (2011) Characteristics of triple-negative breast cancer. J Cancer Res Clin Oncol 137:183-192.

9. Dent R et al. (2007) Triple-negative breast cancer: clinical features and patterns of recurrence. Clin Cancer Res Off JAm Assoc Cancer Res 13:4429-4434.

10. Brulliard M et al. (2007) Nonrandom variations in human cancer ESTs indicate that mRNA heterogeneity increases during carcinogenesis. Proc Natl Acad Sci U S A 104:7522-7527.

11. Wang IX et al. (2014) RNA-DNA Differences Are Generated in Human Cells within Seconds after RNA Exits Polymerase II. Cell Rep 6:906-915.

12. Li M, Wang IX, Cheung VG (2012) Response to Comments on "Widespread RNA and DNA Sequence Differences in the Human Transcriptome." Science 335.

13. Li M et al. (2011) Widespread RNA and DNA sequence differences in the human transcriptome. Science 333:53-58.

14. Lin W, Piskol R, Tan MH, Li JB (2012) Comment on "Widespread RNA and DNA sequence differences in the human transcriptome." Science 335:1302; author reply 1302.

15. Kleinman CL, Majewski J (2012) Comment on "Widespread RNA and DNA sequence differences in the human transcriptome." Science 335:1302; author reply 1302.

16. Pickrell JK, Gilad Y, Pritchard JK (2012) Comment on "Widespread RNA and DNA sequence differences in the human transcriptome." Science 335:1302; author reply 1302.

17. Bahn JH et al. (2012) Accurate identification of A-to-I RNA editing in human by transcriptome sequencing. Genome Res 22:142-50.

18. Bianchetti L, Kieffer D, Féderkeil R, Poch O (2012) Increased frequency of single base substitutions in a population of transcripts expressed in cancer cells. BMC Cancer 12:509.

19. Peng Z et al. (2012) Comprehensive analysis of RNA-Seq data reveals extensive RNA editing in a human transcriptome. Nat Biotechnol 30:253-60.

20. André F et al. (2014) Comparative genomic hybridisation array and DNA sequencing to direct treatment of metastatic breast cancer: a multicentre, prospective trial (SAFIR01/UNICANCER). Lancet Oncol 15:267-274.

21. Swanton C (2012) Intratumor heterogeneity: evolution through space and time. Cancer Res 72:4875-4882.

22. Paik S et al. (2004) A multigene assay to predict recurrence of tamoxifen-treated, node-negative breast cancer. N Engl J Med 351:2817-2826.

23. Van 't Veer LJ et al. (2002) Gene expression profiling predicts clinical outcome of breast cancer. Nature 415:530-536.

24. Chen X et al. (2014) XBP1 promotes triple-negative breast cancer by controlling the HIF1$\alpha$ pathway. Nature 508:103-107.

25. Pathmanathan N, Balleine RL (2013) Ki67 and proliferation in breast cancer. J Clin Pathol 66:512-516.

26. Schreiber RD, Old LJ, Smyth MJ (2011) Cancer immunoediting: integrating immunity's roles in cancer suppression and promotion. Science 331:1565-1570.

27. Li H, Durbin R (2009) Fast and accurate short read alignment with Burrows-Wheeler transform. Bioinforma Oxf Engl 25:1754-1760.

28. Li B, Dewey CN (2011) RSEM: accurate transcript quantification from RNA-Seq data with or without a reference genome. BMC Bioinformatics 12:323.

29. Lê Cao K-A, Boitard S, Besse P (2011) Sparse PLS discriminant analysis: biologically relevant feature selection and graphical displays for multiclass problems. BMC Bioinformatics 12:253.

30. R Development Core Team R: A language and environment for statistical computing. R Foundation for Statistical Computing, Vienna, Austria. ISBN 3-900051-07-0, URL http://www.R-project.org.

31. S. Dejean, I. Gonzalez, K.-A. Lê Cao (2013) mixOmics: Omics Data Integration Project. R Package Version 50-1 HttpCRANR-Proj.

32. Ma X-J et al. (2004) A two-gene expression ratio predicts clinical outcome in breast cancer patients treated with tamoxifen. Cancer Cell 5:607-616.

33. Kohl M (2007) SLqPCR: Functions for analysis of real-time quantitative PCR data at SIRS-Lab GmbH. R Package SIRS-Lab GmbH Jena.

## Claims

1. A method for predicting the clinical outcome of triple negative breast cancer (TNBC) in a subject having TNBC, the method comprising measuring the rate of divergences between RNA and DNA sequences (RDD) in at least one target gene in a sample from said subject, wherein the rate of divergences is indicative of the clinical outcome of said cancer.

2. The method of claim 1, wherein predicting the clinical outcome comprises predicting the capacity of said cancer to metastasize.

3. The method of claim 1, wherein the clinical outcome of TNBC is the likelihood of long-term survival of said patient without the recurrence of TNBC.

4. The method of any one of the preceding claims, wherein measuring the rate of divergences between RNA and DNA sequences (RDD) in at least one target gene comprises determining the ratio between the number of RNA sequence variations and transcript length for said at least one target gene.

5. The method of anyone of the preceding claims, wherein the rate of divergences between RNA and DNA sequences (RDD) is measured in one or more target gene(s), and wherein a combination of RDD rate for said one or more target gene(s) is an indication of the clinical outcome in the subject.

6. The method of any one of the preceding claims, wherein RDD rate is higher for target genes in a group of subjects with good clinical outcome than RDD rate in a group of subjects with poor clinical outcome.

7. The method of any one of the preceding claims, wherein the at least one target gene is selected from GDI2, SORBS3, POM121C, ADAM15 and UQCRHL, or a combination thereof.

8. The method of claim 7, wherein the at least one target gene is GDI2 and wherein an elevated RDD rate for said gene is an indication of good clinical outcome in the subject.

**9.** The method of claim 7, wherein the target genes are GDI2, SORBS3, POM121C, ADAM15 and UQCRHL, and wherein an elevated RDD rate for said target genes combination is an indication of good clinical outcome in the subject.

**10.** The method of any one of claims 4 to 9, wherein the sequence divergences are substitutions.

**11.** The method of claim 10, wherein the substitutions are family base substitutions, preferably selected from C to U, G to A, A to G and T to C substitutions.

**12.** The method of any one of the preceding claims, wherein the triple negative breast cancer is not metastatic.

**13.** The method of any one of the preceding claims, comprising a further step of adapting the TNBC treatment in said subject based on RDD rate.

**14.** A method of claim 1, comprising:

a) providing a sample containing RNAs encoded by said at least one target gene from the subject;
b) extracting from said sample RNAs encoded by said at least one target gene;
c) synthesizing cDNAs from RNAs of step b);
d) sequencing of the cDNAs;
e) comparing the cDNA sequences with a reference DNA sequence of said at least one target gene; and
f) determining the rate of divergences between RNA and DNA sequences (RDD) as the number of sequence variations between the cDNA and the reference DNA sequences, preferably relative to transcript length.

**15.** A compound that modulates the amount or activity of a protein selected from GDI2, SORBS3, POM121C, ADAM15, UQCRHL, or a combination thereof; for use in a method for treating TNBC in a subject having TNBC.

**16.** The compound of claim 15, wherein the compound modulating GDI2 activity is a small interfering RNA (siRNA) molecule comprising a sequence of SEQ ID NO: 1, 2 or 3.

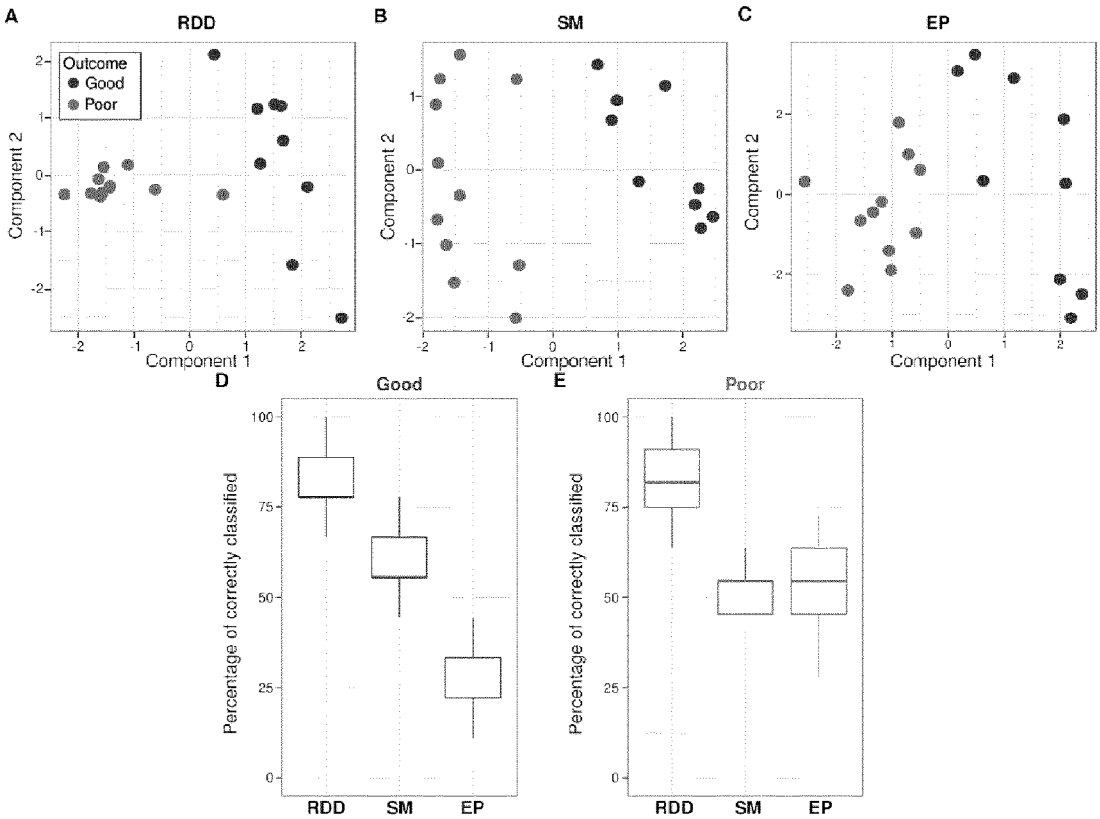
A
RDD
Outcome
Good
Poor
Component 2
Component 1
B
SM
Component 2
Component 1
C
EP
Compnent 2
Component 1
D
Good
Percentage of correctly classified
RDD
SM
EP
E
Poor
Percentage of correctly classified
RDD
SM
EP

Figure 1

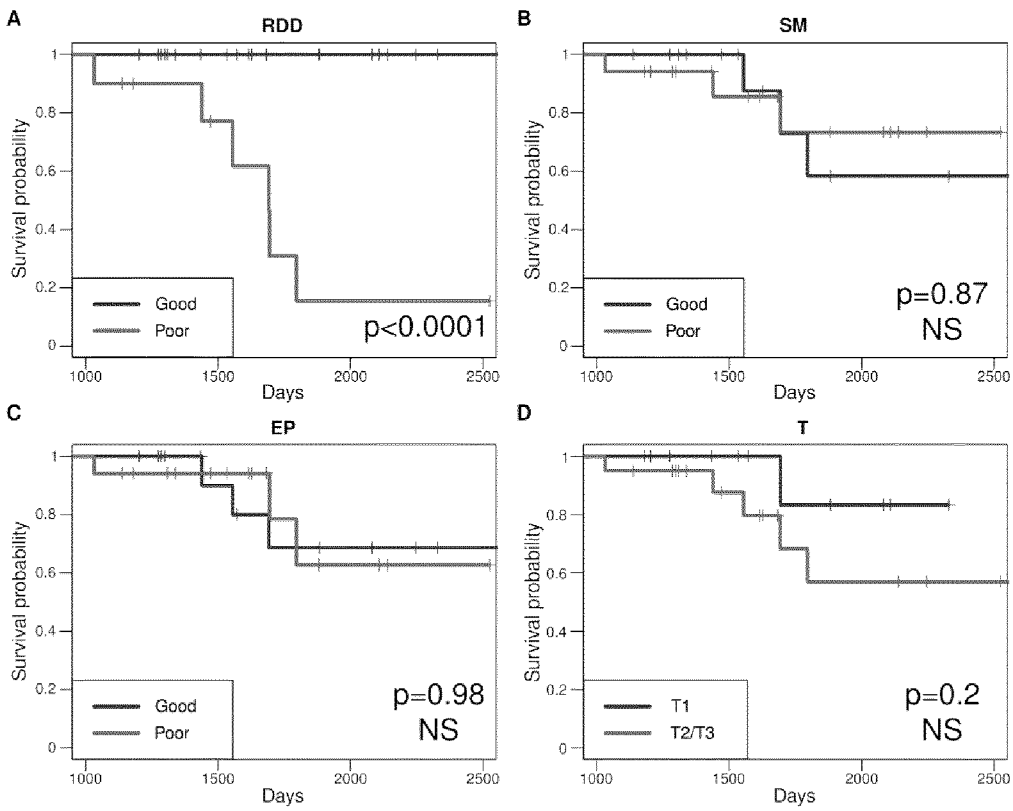
A
RDD
Survival probability
Good
Poor
p<0.0001
Days
B
SM
p=0.87
NS
C
EP
p=0.98
NS
D
T
T1
T2/T3
p=0.2
NS

Figure 2

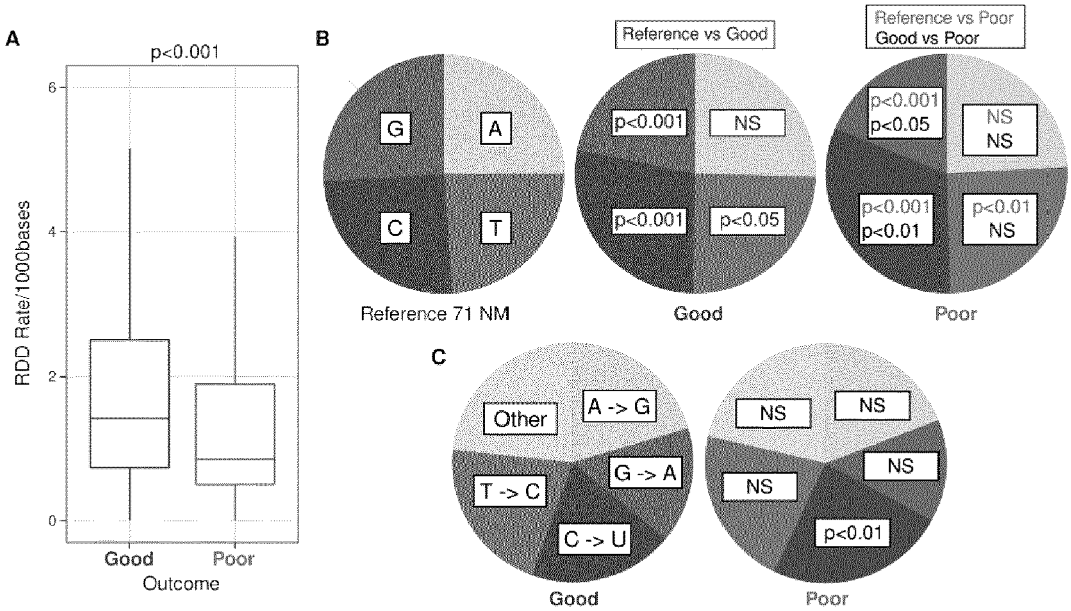
A
p<0.001
RDD Rate/1000bases
6
4
2
0
Good
Poor
Outcome
B
Reference vs Good
Reference vs Poor
Good vs Poor
G
A
C
T
Reference 71 NM
p<0.001
NS
p<0.001
p<0.05
Good
p<0.001
p<0.05
NS
NS
p<0.001
p<0.01
p<0.01
NS
Poor
C
Other
A -> G
G -> A
T -> C
C -> U
Good
NS
NS
NS
NS
p<0.01
Poor

Figure 3

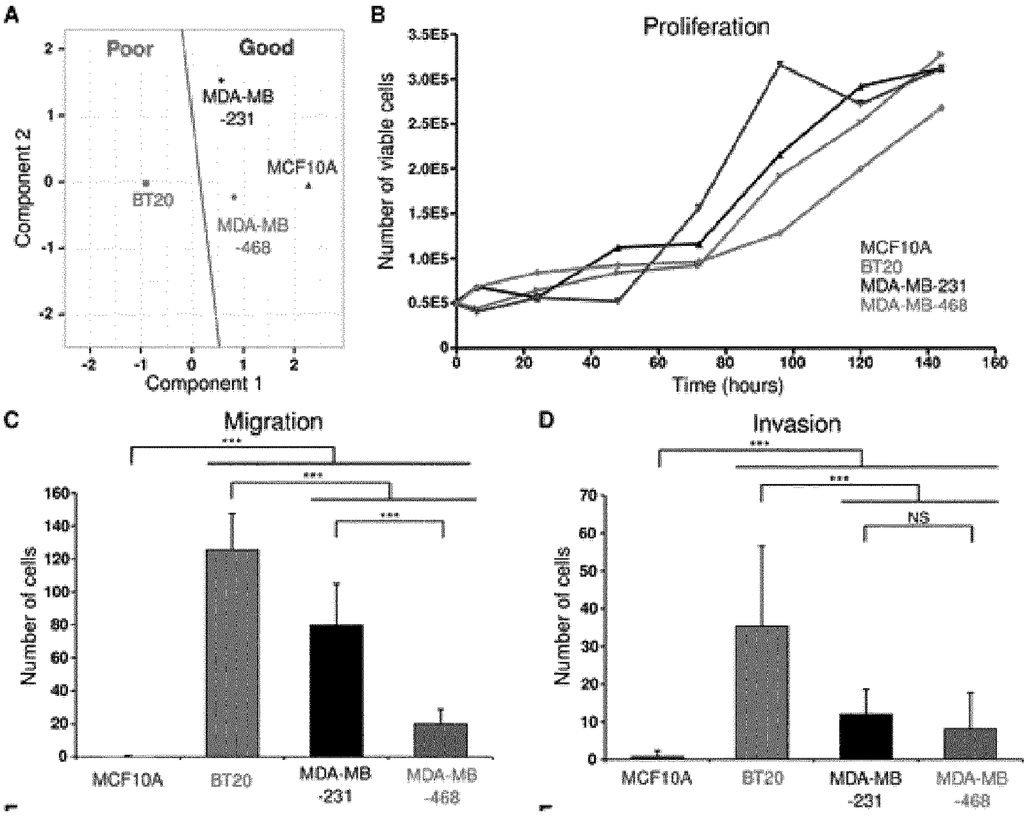


Figure 4

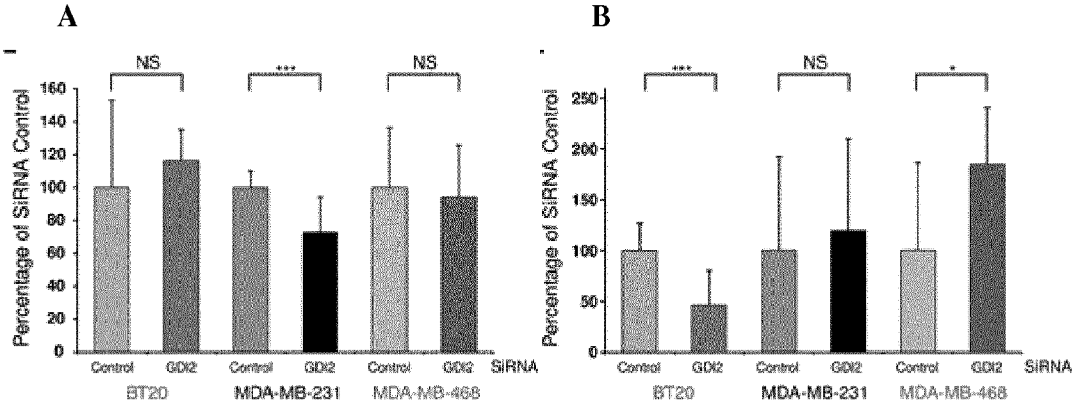


Figure 5

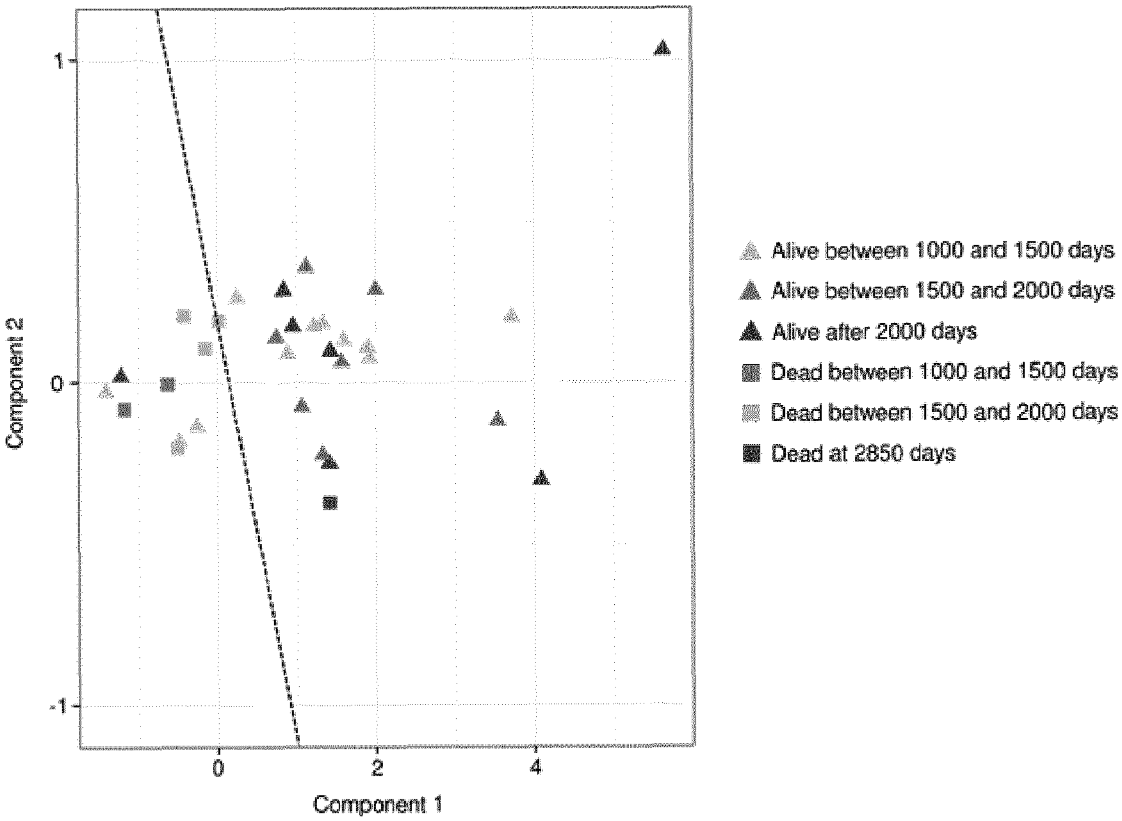
1
0
-1
Component 2
0
2
4
Component 1
Alive between 1000 and 1500 days
Alive between 1500 and 2000 days
Alive after 2000 days
Dead between 1000 and 1500 days
Dead between 1500 and 2000 days
Dead at 2850 days

Figure 6

| Patient | Set | Outcome | Informed consent verified | Tumor status | Vital status | Days to last followup / days to death | Year of initial pathologic diagnosis | Age at initial pathologic diagnosis | Pathologic T | Pathologic N | Pathologic M | Pathologic stage | Histological type | PAM50 |
|---|---|---|---|---|---|---|---|---|---|---|---|---|---|---|
| TCGA-B6-A0I6 | | | YES | WITH TUMOR | Dead | 991 | 1990 | 49 | T1c | N1 | M0 | Stage IIA | Infiltrating Ductal Carcinoma | Basal-like |
| TCGA-A2-A3XU | | | YES | WITH TUMOR | Dead | 912 | 2006 | 35 | T2 | N1mi | M0 | Stage IIB | Infiltrating Ductal Carcinoma | N/A |
| TCGA-A2-A04P | | | YES | WITH TUMOR | Dead | 548 | 2003 | 36 | T2 | N3c | M0 | Stage IIIC | Infiltrating Ductal Carcinoma | Basal-like |
| TCGA-A2-A0CM | | | YES | WITH TUMOR | Dead | 754 | 2003 | 40 | T2 | N0 (i-) | M0 | Stage IIA | Infiltrating Ductal Carcinoma | Basal-like |
| TCGA-AR-A2LH | | | YES | N/A | Dead | 616 | 2007 | 55 | T3 | N3 | M0 | Stage III | Infiltrating Lobular Carcinoma | N/A |
| TCGA-A8-A09X | | Poor | YES | N/A | Dead | 426 | 2009 | 62 | T2 | N3a | M0 | Stage IIIC | Infiltrating Lobular Carcinoma | Her2-E |
| TCGA-EW-A1P8 | | | YES | WITH TUMOR | Dead | 239 | 2010 | 58 | T2 | N3b | M0 | Stage IIIC | Infiltrating Ductal Carcinoma | N/A |
| TCGA-AR-A1AR | | | YES | TUMOR FREE | Dead | 524 | 2007 | 50 | T1 | N2 | M0 | Stage IIIA | Infiltrating Ductal Carcinoma | Basal-like |
| TCGA-B6-A0WX | | | YES | WITH TUMOR | Dead | 653 | 1998 | 40 | T3 | N1b | M0 | Stage IIIA | Other specify | Basal-like |
| TCGA-B6-A0IK | Training | | YES | WITH TUMOR | Dead | 571 | 1994 | 63 | T4 | N1 | M0 | Stage IIIB | Infiltrating Ductal Carcinoma | Her2-E |
| TCGA-A1-A0SK | | | YES | N/A | Dead | 967 | 2007 | 54 | T2 | N0 (i-) | M0 | Stage IIA | Other specify | Basal-like |
| TCGA-AO-A129 | | | YES | TUMOR FREE | Alive | 2923 | 2002 | 29 | T2 | N1a | M0 | Stage IIB | Infiltrating Ductal Carcinoma | Basal-like |
| TCGA-AO-A128 | | | YES | TUMOR FREE | Alive | 2877 | 2002 | 61 | T2 | N0 (i-) | M0 | Stage IIA | Infiltrating Ductal Carcinoma | Basal-like |
| TCGA-AO-A124 | | | YES | TUMOR FREE | Alive | 3120 | 2002 | 38 | T2 | N0 (i-) | M0 | Stage IIA | Other specify | Basal-like |
| TCGA-B6-A0IQ | | | YES | TUMOR FREE | Alive | 3872 | 1996 | 40 | T3 | N1b | M0 | Stage IIIA | Infiltrating Ductal Carcinoma | Basal-like |
| TCGA-B6-A0RT | | Good | YES | TUMOR FREE | Alive | 2721 | 1996 | 39 | T3 | N1 | M0 | Stage IIIA | Infiltrating Ductal Carcinoma | Basal-like |
| TCGA-E2-A1LH | | | YES | TUMOR FREE | Alive | 2876 | 2002 | 59 | T1c | N0 | M0 | Stage I | Infiltrating Ductal Carcinoma | N/A |
| TCGA-B6-A0RU | | | YES | TUMOR FREE | Alive | 3991 | 1998 | 49 | T1c | N0 (i-) | M0 | Stage IA | Other specify | Basal-like |
| TCGA-B6-A0RN | | | YES | TUMOR FREE | Alive | 5261 | 1993 | 60 | T1c | N0 (i-) | M0 | Stage IA | Infiltrating Ductal Carcinoma | Luminal A |
| TCGA-E2-A1LI | | | YES | TUMOR FREE | Alive | 2750 | 2003 | 57 | T2 | N1 | M0 | Stage IIB | Infiltrating Ductal Carcinoma | N/A |

| | | | | | | | | | | | | | | |
|---|---|---|---|---|---|---|---|---|---|---|---|---|---|---|
| TCGA-A2-A3XS | Validation | Poor | YES | WITH TUMOR | Dead | 1032 | 2003 | 62 | T1 | N2a | M0 | Stage IIIA | Infiltrating Ductal Carcinoma | N/A |
| TCGA-BH-A1EW | | | YES | WITH TUMOR | Dead | 1694 | 1999 | 38 | T2 | N1b | M0 | Stage IIA | Infiltrating Ductal Carcinoma | Luminal B |
| TCGA-BH-A18V | | | YES | N/A | Dead | 1556 | 2001 | 48 | T2 | N1b | M0 | Stage IIB | Infiltrating Ductal Carcinoma | Basal-like |
| TCGA-BH-A18Q | | | YES | N/A | Dead | 1692 | 2001 | 56 | T2 | N1b | M0 | Stage IIB | Infiltrating Ductal Carcinoma | Basal-like |
| TCGA-A2-A3XX | | | YES | TUMOR FREE | Alive | 1168 | 2009 | 49 | T2 | N0 | M0 | Stage IIA | Infiltrating Ductal Carcinoma | N/A |
| TCGA-AO-A03U | | | YES | TUMOR FREE | Dead | 1796 | 2001 | 31 | T1c | N0 (i-) | M0 | Stage IA | Other specify | Normal-like |
| TCGA-AO-A12F | | | YES | TUMOR FREE | Alive | 1471 | 2006 | 36 | T2 | N0 (i-) | M0 | Stage IIA | Infiltrating Ductal Carcinoma | Basal-like |
| TCGA-AR-A0TS | | | YES | N/A | Alive | 1138 | 2006 | 46 | T2 | N1 | M0 | Stage IIB | Infiltrating Ductal Carcinoma | Basal-like |
| TCGA-BH-A0AV | | | YES | TUMOR FREE | Alive | 1180 | 2008 | 52 | T1c | N0 | M0 | Stage I | Infiltrating Ductal Carcinoma | Basal-like |
| TCGA-A2-A3XT | | | YES | TUMOR FREE | Alive | 2238 | 2006 | 45 | T2 | N1a | M0 | Stage IIB | Infiltrating Ductal Carcinoma | N/A |
| TCGA-AR-A256 | | Good | YES | N/A | Dead | 2854 | 2001 | 45 | T2 | N0 | M0 | Stage IIA | Infiltrating Ductal Carcinoma | N/A |
| TCGA-A2-A0SX | | | YES | WITH TUMOR | Alive | 1288 | 2007 | 48 | T1c | N0 (i-) | M0 | Stage IA | Infiltrating Ductal Carcinoma | Basal-like |
| TCGA-GM-A3XL | | | YES | TUMOR FREE | Alive | 1717 | 2007 | 49 | T2 | N0 (i-) | M0 | Stage IIA | Infiltrating Ductal Carcinoma | N/A |
| TCGA-BH-A0BL | | | YES | TUMOR FREE | Alive | 1340 | 2007 | 35 | T1c | N0 | M0 | Stage I | Infiltrating Ductal Carcinoma | Basal-like |
| TCGA-AR-A1AI | | | YES | TUMOR FREE | Alive | 1881 | 2005 | 47 | T2 | N0 | M0 | Stage IIA | Medullary Carcinoma | Basal-like |
| TCGA-AO-A1KR | | | YES | TUMOR FREE | Alive | 2141 | 2005 | 51 | T2 | N0 | M0 | Stage IIA | Infiltrating Ductal Carcinoma | N/A |
| TCGA-BH-A0B3 | | | YES | TUMOR FREE | Alive | 1203 | 2007 | 53 | T2 | N1a | M0 | Stage IIB | Infiltrating Ductal Carcinoma | Basal-like |
| TCGA-E2-A14N | | | YES | TUMOR FREE | Alive | 1350 | 2007 | 37 | T2 | N1 | M0 | Stage IIB | Infiltrating Ductal Carcinoma | Basal-like |
| TCGA-AR-A1AQ | | | YES | TUMOR FREE | Alive | 1310 | 2007 | 49 | T2 | N0 | M0 | Stage IIA | Infiltrating Ductal Carcinoma | Basal-like |
| TCGA-AO-A0JL | | | YES | TUMOR FREE | Alive | 1319 | 2006 | 59 | T2 | N2a | M0 | Stage IIIA | Infiltrating Ductal Carcinoma | Basal-like |
| TCGA-A2-A04T | | | YES | TUMOR FREE | Alive | 1950 | 2004 | 62 | T2 | N0 (i-) | M0 | Stage IIA | Infiltrating Ductal Carcinoma | Basal-like |
| TCGA-GM-A2DF | | | YES | TUMOR FREE | Alive | 1299 | 2007 | 53 | T1c | N1a | M0 | Stage IIA | Infiltrating Ductal Carcinoma | N/A |
| TCGA-A2-A0ST | | | YES | N/A | Alive | 1643 | 2003 | 62 | T1c | N1a | M0 | Stage IIA | Infiltrating Ductal Carcinoma | Basal-like |

| | | | | | | | | | | | | | | |
|---|---|---|---|---|---|---|---|---|---|---|---|---|---|---|
| TCGA-GM-A2DI | | | YES | TUMOR FREE | Alive | 1883 | 2005 | 52 | T1c | N0 (i+) | M0 | Stage I | Infiltrating Ductal Carcinoma | N/A |
| TCGA-BH-A0B9 | | | YES | TUMOR FREE | Alive | 1572 | 2006 | 44 | T1c | N0 (i-) | M0 | Stage IA | Infiltrating Ductal Carcinoma | Basal-like |
| TCGA-A2-A04Q | | | YES | TUMOR FREE | Alive | 1276 | 2004 | 48 | T1 | N0 (i-) | M0 | Stage IA | Infiltrating Ductal Carcinoma | Basal-like |
| TCGA-GM-A2DD | | | YES | TUMOR FREE | Alive | 1309 | 2007 | 53 | T1c | N0 (i-) | M0 | Stage I | Infiltrating Ductal Carcinoma | N/A |
| TCGA-GM-A2DB | | | YES | TUMOR FREE | Alive | 1616 | 2006 | 62 | T2 | N0 (i-) | M0 | Stage IIA | Infiltrating Ductal Carcinoma | N/A |
| TCGA-GM-A2DH | | | YES | TUMOR FREE | Alive | 1286 | 2007 | 58 | T1c | N0 (i+) | M0 | Stage I | Infiltrating Ductal Carcinoma | N/A |
| TCGA-AR-A0U4 | | | YES | N/A | Alive | 1684 | 2006 | 54 | T2 | N0 | M0 | Stage IIA | Infiltrating Ductal Carcinoma | Basal-like |
| TCGA-E2-A1AZ | | | YES | TUMOR FREE | Alive | 1965 | 2004 | 63 | T2 | N1a | M0 | Stage IIB | Infiltrating Ductal Carcinoma | Basal-like |
| TCGA-B6-A0RG | | | YES | TUMOR FREE | Alive | 2082 | 1991 | 26 | T3 | N0 (i-) | M0 | Stage IIB | Infiltrating Ductal Carcinoma | Luminal A |

| | | | | | | | | | | | | | | |
|---|---|---|---|---|---|---|---|---|---|---|---|---|---|---|
| TCGA-A2-A0SV | | Good | YES | WITH TUMOR | Dead | 825 | 2006 | 63 | T2 | N2a | M1 | Stage IV | Infiltrating Ductal Carcinoma | Luminal B |
| TCGA-B6-A0X4 | | Good | YES | WITH TUMOR | Dead | 859 | 1996 | 62 | T2 | N1b | M0 | Stage IIB | Infiltrating Ductal Carcinoma | Luminal A |
| TCGA-BH-A18P | | Good | YES | WITH TUMOR | Dead | 921 | 2003 | 60 | T1 | N0 | M0 | Stage I | Infiltrating Ductal Carcinoma | Her2-E |
| TCGA-AO-A03P | | Poor | YES | WITH TUMOR | Alive | 2576 | 2003 | 54 | T2 | N1 | M0 | Stage IIA | Infiltrating Ductal Carcinoma | Luminal B |
| TCGA-AQ-A04L | | Good | YES | TUMOR FREE | Alive | 3359 | 2001 | 48 | T2 | N0 (i-) | MX | Stage IIA | Infiltrating Ductal Carcinoma | Luminal A |
| TCGA-B6-A0I5 | Non TNBC Control | Poor | YES | TUMOR FREE | Alive | 4549 | 1988 | 49 | T2 | N1b | M0 | Stage IIB | Infiltrating Ductal Carcinoma | Luminal A |
| TCGA-B6-A0IA | | Poor | YES | TUMOR FREE | Alive | 6718 | 1992 | 51 | T2 | N0 (i-) | M0 | Stage IIA | Other specify | Luminal A |
| TCGA-B6-A0IB | | Poor | YES | WITH TUMOR | Dead | 3941 | 1992 | 64 | T3 | N3 | M1 | Stage IV | Infiltrating Ductal Carcinoma | Luminal B |
| TCGA-B6-A0IJ | | Poor | YES | TUMOR FREE | Alive | 5383 | 1995 | 42 | T3 | N0 (i-) | M0 | Stage IIB | Infiltrating Ductal Carcinoma | Basal-like |
| TCGA-B6-A0RH | | Poor | YES | TUMOR FREE | Alive | 5748 | 1991 | 51 | T2 | N0 (i-) | M0 | Stage IIA | Infiltrating Ductal Carcinoma | Her2-E |
| TCGA-B6-A0WZ | | Good | YES | TUMOR FREE | Alive | 3941 | 1996 | 50 | T2 | N1b | M0 | Stage IIB | Infiltrating Ductal Carcinoma | Luminal A |
| TCGA-A2-A0CO | | Good | YES | TUMOR FREE | Alive | 3408 | 2002 | 85 | T3 | N0 | M0 | Stage IIB | Infiltrating Lobular Carcinoma | N/A |

Figure 7

<table>
<tr><th>id</th><th>GeneID</th><th>Symbol</th><th>description</th><th>other_designations</th><th>chromosome</th><th>start_position</th><th>end_position</th></tr>
<tr><td>RDD</td><td></td><td></td><td></td><td></td><td></td><td></td><td></td></tr>
<tr><td>NM_003815.4</td><td>8751</td><td>ADAM15</td><td>ADAM metallopeptidase domain 15</td><td>MDC-15|a disintegrin and metalloproteinase domain 15 (metargidin)|disintegrin and metalloproteinase domain-containing protein 15|metalloprotease RGD disintegrin protein|metalloproteinase-like, disintegrin-like, and cysteine-rich protein 15</td><td>1</td><td>155051272</td><td>155062776</td></tr>
<tr><td>NM_001494.3</td><td>2665</td><td>GDI2</td><td>GDP dissociation inhibitor 2</td><td>GDI-2|epididymis secretory sperm binding protein Li 46e|guanosine diphosphate dissociation inhibitor 2|rab GDI beta|rab GDP dissociation inhibitor beta|rab GDP-dissociation inhibitor, beta</td><td>10</td><td>5765223</td><td>5813549</td></tr>
<tr><td>NM_005775.4</td><td>10174</td><td>SORBS3</td><td>sorbin and SH3 domain containing 3</td><td>sorbin and SH3 domain-containing protein 3|vinexin|vinexin beta (SH3-containing adaptor molecule-1)</td><td>8</td><td>22544783</td><td>22575495</td></tr>
<tr><td>NM_001099415.2</td><td>100101267</td><td>POM121C</td><td>POM121 transmembrane nucleoporin C</td><td>POM121 membrane glycoprotein C|nuclear envelope pore membrane protein POM 121C|nuclear pore membrane protein 121-2|pore membrane protein of 121 kDa C</td><td>7</td><td>75416781</td><td>75486288</td></tr>
<tr><td>NM_001089591.1</td><td>440567</td><td>UQCRHL</td><td>ubiquinol-cytochrome c reductase hinge protein-like</td><td>hCG25371</td><td>1</td><td>15807162</td><td>15807699</td></tr>
<tr><td></td><td></td><td></td><td></td><td></td><td></td><td></td><td></td></tr>
<tr><td>SM</td><td></td><td></td><td></td><td></td><td></td><td></td><td></td></tr>
<tr><td>NM_178477.4</td><td>140836</td><td>BANF2</td><td>barrier to autointegration factor 2</td><td>barrier-to-autointegration factor 2|barrier-to-autointegration factor-like protein</td><td>20</td><td>17693675</td><td>17735872</td></tr>
<tr><td>NM_001004136.1</td><td>401992</td><td>OR2T2</td><td>olfactory receptor, family 2, subfamily T, member 2</td><td>olfactory receptor 2T2|olfactory receptor OR1-43|olfactory receptor, family 2, subfamily T, member 2 pseudogene</td><td>1</td><td>248452798</td><td>248453772</td></tr>
<tr><td>NM_178861.4</td><td>140432</td><td>RNF113B</td><td>ring finger protein 113B</td><td>RING finger protein 113B|zinc finger protein 183-like 1</td><td>13</td><td>98175785</td><td>98177267</td></tr>
<tr><td>NM_052965.2</td><td>116461</td><td>TSEN15</td><td>TSEN15 tRNA splicing endonuclease subunit</td><td>SEN15 homolog|hsSen15|tRNA splicing endonuclease 15 homolog|tRNA-intron endonuclease Sen15|tRNA-splicing endonuclease subunit Sen15</td><td>1</td><td>184051657</td><td>184074212</td></tr>
<tr><td>NM_005124.3</td><td>9972</td><td>NUP153</td><td>nucleoporin 153kDa</td><td>153 kDa nucleoporin|nuclear pore complex protein Nup153|nuclear pore complex protein hnup153|nucleoporin Nup153</td><td>6</td><td>17615035</td><td>17706834</td></tr>
<tr><td></td><td></td><td></td><td></td><td></td><td></td><td></td><td></td></tr>
<tr><td>EP</td><td></td><td></td><td></td><td></td><td></td><td></td><td></td></tr>
<tr><td>SLC44A2</td><td>57153</td><td>SLC44A2</td><td>solute carrier family 44 (choline transporter), member 2</td><td>choline transporter-like protein 2|solute carrier family 44, member 2</td><td>19</td><td>10602445</td><td>10644559</td></tr>
<tr><td>EXOC8</td><td>149371</td><td>EXOC8</td><td>exocyst complex component 8</td><td>exocyst complex 84 kDa subunit|exocyst complex 84-kDa subunit</td><td>1</td><td>231332736</td><td>231337832</td></tr>
<tr><td>DOCK6</td><td>57572</td><td>DOCK6</td><td>dedicator of cytokinesis 6</td><td>dedicator of cytokinesis protein 6</td><td>19</td><td>11199293</td><td>11262492</td></tr>
<tr><td>C10orf91</td><td>170393</td><td>C10orf91</td><td>chromosome 10 open reading frame 91</td><td>uncharacterized protein C10orf91</td><td>10</td><td>132445210</td><td>132448321</td></tr>
<tr><td>SCARNA9L</td><td>100158262</td><td>SCARNA9L</td><td>small Cajal body-specific RNA 9-like</td><td></td><td>X</td><td>20136066</td><td>20136413</td></tr>
</table>

**Figure 8**

<table>
<tr><th>id</th><th>GeneID</th><th>Symbol</th><th>description</th><th>other_designations</th><th>chromosome</th><th>start_position</th><th>end_position</th></tr>
<tr><td>NM_001626.4</td><td>208</td><td>AKT2</td><td>v-akt murine thymoma viral oncogene homolog 2</td><td>PKB beta|RAC-PK-beta|RAC-beta serine/threonine-protein kinase|murine thymoma viral (v-akt) homolog-2|protein kinase Akt-2|protein kinase B beta|rac protein kinase beta</td><td>19</td><td>40230317</td><td>40285536</td></tr>
<tr><td>NM_001185.3</td><td>563</td><td>AZGP1</td><td>alpha-2-glycoprotein 1, zinc-binding</td><td>Alpha-2-glycoprotein, zinc|Zn-alpha2-glycoprotein|zinc-alpha-2-glycoprotein|zn-alpha-2-GP|zn-alpha-2-glycoprotein</td><td>7</td><td>99966727</td><td>99976112</td></tr>
<tr><td>NM_001311.4</td><td>1396</td><td>CRIP1</td><td>cysteine-rich protein 1 (intestinal)</td><td>cysteine-rich heart protein|cysteine-rich intestinal protein|cysteine-rich protein 1</td><td>14</td><td>105486920</td><td>105488789</td></tr>
<tr><td>NM_004937.2</td><td>1497</td><td>CTNS</td><td>cystinosin, lysosomal cystine transporter</td><td>cystinosin</td><td>17</td><td>3636468</td><td>3663103</td></tr>
<tr><td>NM_001355.3</td><td>1652</td><td>DDT</td><td>D-dopachrome tautomerase</td><td>D-dopachrome decarboxylase|phenylpyruvate tautomerase II</td><td>22</td><td>23971365</td><td>23979828</td></tr>
<tr><td>NM_001968.3</td><td>1977</td><td>EIF4E</td><td>eukaryotic translation initiation factor 4E</td><td>eIF-4E|eIF-4F 25 kDa subunit|eukaryotic translation initiation factor 4E-like 1|mRNA cap-binding protein</td><td>4</td><td>98878456</td><td>98930635</td></tr>
<tr><td>NM_004443.3</td><td>2049</td><td>EPHB3</td><td>EPH receptor B3</td><td>EK2|EPH-like kinase 2|EPH-like tyrosine kinase 2|EPH-like tyrosine kinase-2|embryonic kinase 2|ephrin type-B receptor 3|human embryo kinase 2|tyrosine-protein kinase TYRO6</td><td>3</td><td>184561799</td><td>184582408</td></tr>
<tr><td>NM_001494.3</td><td>2665</td><td>GDI2</td><td>GDP dissociation inhibitor 2</td><td>GDI-2|epididymis secretory sperm binding protein Li 46e|guanosine diphosphate dissociation inhibitor 2|rab GDI beta|rab GDP dissociation inhibitor beta|rab GDP-dissociation inhibitor, beta</td><td>10</td><td>5765223</td><td>5813549</td></tr>
<tr><td>NM_002123.4</td><td>3119</td><td>HLA-DQB1</td><td>major histocompatibility complex, class II, DQ beta 1</td><td>HLA class II histocompatibility antigen, DQ beta 1 chain|MHC DQ beta|MHC class II DQ beta chain|MHC class II HLA-DQ beta glycoprotein|MHC class II antigen DQB1|MHC class II antigen HLA-DQ-beta-1|MHC class2 antigen|lymphocyte antigen</td><td>6</td><td>32659464</td><td>32666689</td></tr>
<tr><td>NM_002124.3</td><td>3123</td><td>HLA-DRB1</td><td>major histocompatibility complex, class II, DR beta 1</td><td>DW2.2/DR2.2|HLA class II histocompatibility antigen, DR-1 beta chain|MHC class II HLA-DR beta 1 chain|MHC class II HLA-DR-beta cell surface glycoprotein|MHC class II HLA-DRw10-beta|MHC class II antigen|human leucocyte antigen DRB1|lymphocyte antigen DRB1</td><td>6</td><td>32578769</td><td>32589836</td></tr>
<tr><td>NM_002125.3</td><td>3127</td><td>HLA-DRB5</td><td>major histocompatibility complex, class II, DR beta 5</td><td>DR beta-5|DR-9|DR2-beta-2|DR9|HLA class II histocompatibility antigen, DR beta 5 chain|HLA class II histocompatibility antigen, DR-5 beta chain|HLA class II histocompatibility antigen, DRB1-9 beta chain|MHC HLA-DR-beta cell surface glycoprotein|MHC HLA-DR-beta chain|MHC class II HLA beta chain|MHC class II antigen DRB1*9|MHC class II antigen DRB5|dw2|human leucocyte antigen DRB5|leukocyte antigen class II</td><td>6</td><td>32517374</td><td>32530229</td></tr>
<tr><td>NM_002231.3</td><td>3732</td><td>CD82</td><td>CD82 molecule</td><td>C33 antigen|CD82 antigen|inducible membrane protein R2|kangai 1 (suppression of tumorigenicity 6, prostate; CD82 antigen (R2 leukocyte antigen, antigen detected by monoclonal and antibody IA4))|metastasis suppressor Kangai-1|tetraspanin-27|tspan-27</td><td>11</td><td>44564427</td><td>44620363</td></tr>
<tr><td>NM_002337.3</td><td>4043</td><td>LRPAP1</td><td>low density lipoprotein receptor-related protein associated protein 1</td><td>39 kDa receptor-associated protein|alpha-2-macroglobulin receptor-associated protein|low density lipoprotein-related protein-associated protein 1 (alpha-2-macroglobulin receptor-associated protein 1)</td><td>4</td><td>3503597</td><td>3532497</td></tr>
<tr><td>NM_005899.3</td><td>4077</td><td>NBR1</td><td>neighbor of BRCA1 gene 1</td><td>B-box protein|cell migration-inducing gene 19 protein|membrane component, chromosome 17, surface marker 2 (ovarian carcinoma antigen CA125)|migration-inducing protein 19|next to BRCA1 gene 1 protein</td><td>17</td><td>43170481</td><td>43211688</td></tr>
<tr><td>NM_021079.3</td><td>4836</td><td>NMT1</td><td>N-myristoyltransferase 1</td><td>NMT 1|alternative, short form NMT-S|glycylpeptide N-tetradecanoyltransferase 1|long form, NMT-L|myristoyl-CoA:protein N-myristoyltransferase 1|peptide N-myristoyltransferase 1|type I N-myristoyltransferase</td><td>17</td><td>45061032</td><td>45109016</td></tr>
</table>

<table>
<tr><td>NM_002659.3</td><td>5329</td><td>PLAUR</td><td>plasminogen activator, urokinase receptor</td><td>monocyte activation antigen Mo3|u-plasminogen activator receptor form 2|urokinase plasminogen activator surface receptor|urokinase-type plasminogen activator (uPA) receptor</td><td>19</td><td>43646095</td><td>43670346</td></tr>
<tr><td>NM_002792.3</td><td>5688</td><td>PSMA7</td><td>proteasome (prosome, macropain) subunit, alpha type, 7</td><td>proteasome subunit RC6-1|proteasome subunit XAPC7|proteasome subunit alpha 4|proteasome subunit alpha type-7</td><td>20</td><td>62136727</td><td>62143458</td></tr>
<tr><td>NM_002802.2</td><td>5700</td><td>PSMC1</td><td>proteasome (prosome, macropain) 26S subunit, ATPase, 1</td><td>26S protease regulatory subunit 4|26S proteasome AAA-ATPase subunit RPT2|proteasome 26S ATPase subunit 1|proteasome 26S subunit ATPase 1|proteasome 26S subunit, ATPase, 1</td><td>14</td><td>90256550</td><td>90272622</td></tr>
<tr><td>NM_003134.4</td><td>6727</td><td>SRP14</td><td>signal recognition particle 14kDa (homologous Alu RNA binding protein)</td><td>18 kDa Alu RNA-binding protein|signal recognition particle 14 kDa protein</td><td>15</td><td>40035690</td><td>40039188</td></tr>
<tr><td>NM_005648.3</td><td>6921</td><td>TCEB1</td><td>transcription elongation factor B (SIII), polypeptide 1 (15kDa, elongin C)</td><td>RNA polymerase II transcription factor SIII subunit C|SIII p15|elongin 15 kDa subunit|transcription elongation factor B polypeptide 1</td><td>8</td><td>73945138</td><td>73972287</td></tr>
<tr><td>NM_170607.2</td><td>6945</td><td>MLX</td><td>MLX, MAX dimerization protein</td><td>BigMax protein|MAX-like bHLHZIP protein|class D basic helix-loop-helix protein 13|max-like protein X|transcription factor-like protein 4</td><td>17</td><td>42567060</td><td>42573203</td></tr>
<tr><td>NM_001064.3</td><td>7086</td><td>TKT</td><td>transketolase</td><td>epididymis luminal protein 107</td><td>3</td><td>53224707</td><td>53256114</td></tr>
<tr><td>NM_003343.5</td><td>7327</td><td>UBE2G2</td><td>ubiquitin-conjugating enzyme E2G 2</td><td>ubiquitin carrier protein G2|ubiquitin conjugating enzyme 7|ubiquitin conjugating enzyme G2|ubiquitin-conjugating enzyme E2 G2|ubiquitin-conjugating enzyme E2G 2 (UBC7 homolog, yeast)|ubiquitin-conjugating enzyme E2G 2 (homologous to yeast UBC7)|ubiquitin-protein ligase G2</td><td>21</td><td>44768580</td><td>44801836</td></tr>
<tr><td>NM_003345.4</td><td>7329</td><td>UBE2I</td><td>ubiquitin-conjugating enzyme E2I</td><td>SUMO-1-protein ligase|SUMO-conjugating enzyme UBC9|SUMO-protein ligase|ubiquitin carrier protein 9|ubiquitin carrier protein I|ubiquitin conjugating enzyme 9|ubiquitin-conjugating enzyme E2I (UBC9 homolog, yeast)|ubiquitin-conjugating enzyme E2I (homologous to yeast UBC9)|ubiquitin-conjugating enzyme UbcE2A|ubiquitin-like protein SUMO-1 conjugating enzyme|ubiquitin-protein ligase E2|ubiquitin-protein ligase I</td><td>16</td><td>1309153</td><td>1327018</td></tr>
<tr><td>NM_006004.2</td><td>7388</td><td>UQCRH</td><td>ubiquinol-cytochrome c reductase hinge protein</td><td>complex III subunit 6|complex III subunit VIII|cytochrome b-c1 complex subunit 6, mitochondrial|cytochrome c1 non-heme 11 kDa protein|mitochondrial hinge protein|ubiquinol-cytochrome c reductase complex 11 kDa protein|ubiquinol-cytochrome c reductase, complex III subunit VIII</td><td>1</td><td>46303654</td><td>46316776</td></tr>
<tr><td>NM_005433.3</td><td>7525</td><td>YES1</td><td>v-yes-1 Yamaguchi sarcoma viral oncogene homolog 1</td><td>Yamaguchi sarcoma oncogene|cellular yes-1 protein|proto-oncogene c-Yes|proto-oncogene tyrosine-protein kinase YES|tyrosine-protein kinase Yes</td><td>18</td><td>721588</td><td>812326</td></tr>
<tr><td>NM_003641.3</td><td>8519</td><td>IFITM1</td><td>interferon induced transmembrane protein 1</td><td>dispanin subfamily A member 2a|interferon-induced protein 17|interferon-induced transmembrane protein 1|interferon-inducible protein 9-27|leu-13 antigen</td><td>11</td><td>313991</td><td>315272</td></tr>
<tr><td>NM_003677.3</td><td>8562</td><td>DENR</td><td>density-regulated protein</td><td>smooth muscle cell associated protein-3|smooth muscle cell-associated protein 3</td><td>12</td><td>122752824</td><td>122771406</td></tr>
<tr><td>NM_003815.4</td><td>8751</td><td>ADAM15</td><td>ADAM metallopeptidase domain 15</td><td>MDC-15|a disintegrin and metalloproteinase domain 15 (metargidin)|disintegrin and metalloproteinase domain-containing protein 15|metalloprotease RGD disintegrin protein|metalloproteinase-like, disintegrin-like, and cysteine-rich protein 15</td><td>1</td><td>155051272</td><td>155062776</td></tr>
<tr><td>NM_004718.2</td><td>9167</td><td>COX7A2L</td><td>cytochrome c oxidase subunit VIIa polypeptide 2 like</td><td>COX7a-related protein|cytochrome c oxidase subunit 7A-related protein, mitochondrial|cytochrome c oxidase subunit VII-related protein|cytochrome c oxidase subunit VIIa-related protein|estrogen receptor binding CpG island</td><td>2</td><td>42350502</td><td>42361216</td></tr>
</table>

<table>
<tr><td>NM_014757.4</td><td>9794</td><td>MAML1</td><td>mastermind-like 1 (Drosophila)</td><td>mastermind homolog|mastermind-like protein 1</td><td>5</td><td>179732850</td><td>179777286</td></tr>
<tr><td>NM_020297.2</td><td>10060</td><td>ABCC9</td><td>ATP-binding cassette, sub-family C (CFTR/MRP), member 9</td><td>ATP-binding cassette sub-family C member 9|ATP-binding cassette transporter sub-family C member 9|sulfonylurea receptor 2</td><td>12</td><td>21797389</td><td>21941863</td></tr>
<tr><td>NM_005775.4</td><td>10174</td><td>SORBS3</td><td>sorbin and SH3 domain containing 3</td><td>sorbin and SH3 domain-containing protein 3|vinexin|vinexin beta (SH3-containing adaptor molecule-1)</td><td>8</td><td>22544783</td><td>22575495</td></tr>
<tr><td>NM_006354.3</td><td>10474</td><td>TADA3</td><td>transcriptional adaptor 3</td><td>ADA3 homolog|ADA3-like protein|transcriptional adapter 3</td><td>3</td><td>9779964</td><td>9793124</td></tr>
<tr><td>NM_006369.4</td><td>10489</td><td>LRRC41</td><td>leucine rich repeat containing 41</td><td>MUF1 protein (MUF1)|elongin BC-interacting leucine-rich repeat protein|leucine-rich repeat-containing protein 41|protein Muf1</td><td>1</td><td>46278400</td><td>46303366</td></tr>
<tr><td>NM_006405.5</td><td>10548</td><td>TM9SF1</td><td>transmembrane 9 superfamily member 1</td><td>MP70 protein family member|multispanning membrane protein (70kD)|transmembrane protein 9 superfamily member 1</td><td>14</td><td>24189143</td><td>24195736</td></tr>
<tr><td>NM_006825.3</td><td>10970</td><td>CKAP4</td><td>cytoskeleton-associated protein 4</td><td>63 kDa membrane protein|63-kDa cytoskeleton-linking membrane protein|transmembrane protein (63kD), endoplasmic reticulum/Golgi intermediate compartment|type-II transmembrane protein p63</td><td>12</td><td>106237881</td><td>106247935</td></tr>
<tr><td>NM_014142.2</td><td>11164</td><td>NUDT5</td><td>nudix (nucleoside diphosphate linked moiety X)-type motif 5</td><td>8-oxo-dGDP phosphatase|ADP-sugar pyrophosphatase</td><td>10</td><td>12167574</td><td>12196144</td></tr>
<tr><td>NM_015084.2</td><td>23107</td><td>MRPS27</td><td>mitochondrial ribosomal protein S27</td><td>28S ribosomal protein S27, mitochondrial|mitochondrial 28S ribosomal protein S27</td><td>5</td><td>72219409</td><td>72320257</td></tr>
<tr><td>NM_015299.2</td><td>23351</td><td>KHNYN</td><td>KH and NYN domain containing</td><td>protein KHNYN</td><td>14</td><td>24429735</td><td>24441342</td></tr>
<tr><td>NM_021149.2</td><td>23406</td><td>COTL1</td><td>coactosin-like F-actin binding protein 1</td><td>coactosin-like 1|coactosin-like protein</td><td>16</td><td>84565596</td><td>84618096</td></tr>
<tr><td>NM_015379.4</td><td>25798</td><td>BRI3</td><td>brain protein I3</td><td>pRGR2</td><td>7</td><td>98281659</td><td>98323430</td></tr>
<tr><td>NM_015527.3</td><td>26000</td><td>TBC1D10B</td><td>TBC1 domain family, member 10B</td><td>Rab27A-GAPbeta|TBC1 domain family member 10B|rab27A-GAP-beta</td><td>16</td><td>30357101</td><td>30370201</td></tr>
<tr><td>NM_015670.5</td><td>26168</td><td>SENP3</td><td>SUMO1/sentrin/SMT3 specific peptidase 3</td><td>SUMO-1-specific protease 3|sentrin-specific protease 3|sentrin/SUMO-specific protease 3</td><td>17</td><td>7561992</td><td>7571969</td></tr>
<tr><td>NM_014041.3</td><td>28972</td><td>SPCS1</td><td>signal peptidase complex subunit 1 homolog (S. cerevisiae)</td><td>SPase 12 kDa subunit|microsomal signal peptidase 12 kDa subunit|signal peptidase 12kDa|signal peptidase complex subunit 1</td><td>3</td><td>52705841</td><td>52708182</td></tr>
<tr><td>NM_014145.4</td><td>29058</td><td>TMEM230</td><td>transmembrane protein 230</td><td>UPF0414 transmembrane protein C20orf30</td><td>20</td><td>5099837</td><td>5113087</td></tr>
<tr><td>NM_014169.3</td><td>29082</td><td>CHMP4A</td><td>charged multivesicular body protein 4A</td><td>SNF7 homolog associated with Alix-2|Snf7 homologue associated with Alix 2|charged multivesicular body protein 4a|chromatin modifying protein 4A|vacuolar protein sorting-associated protein 32-1</td><td>14</td><td>24209581</td><td>24213830</td></tr>
<tr><td>NM_013238.2</td><td>29103</td><td>DNAJC15</td><td>DnaJ (Hsp40) homolog, subfamily C, member 15</td><td>DNAJ domain-containing|DnaJ (Hsp40) homolog, subfamily D, member 1|cell growth-inhibiting gene 22 protein|dnaJ homolog subfamily C member 15|methylation-controlled J protein</td><td>13</td><td>43023226</td><td>43109170</td></tr>
<tr><td>NM_016174.4</td><td>51148</td><td>CERCAM</td><td>cerebral endothelial cell adhesion molecule</td><td>cerebral cell adhesion molecule|cerebral endothelial cell adhesion molecule 1|glycosyltransferase 25 domain containing 3|glycosyltransferase 25 family member 3|probable inactive glycosyltransferase 25 family member 3</td><td>9</td><td>128411575</td><td>128437351</td></tr>
<tr><td>NM_018246.2</td><td>55246</td><td>CCDC25</td><td>coiled-coil domain containing 25</td><td>coiled-coil domain-containing protein 25</td><td>8</td><td>27733316</td><td>27772653</td></tr>
<tr><td>NM_018446.3</td><td>55830</td><td>GLT8D1</td><td>glycosyltransferase 8 domain containing 1</td><td>glycosyltransferase 8 domain-containing protein 1|glycosyltransferase AD-017</td><td>3</td><td>52694484</td><td>52706083</td></tr>
<tr><td>NM_018715.3</td><td>55920</td><td>RCC2</td><td>regulator of chromosome condensation 2</td><td>RCC1-like protein TD-60|protein RCC2|telophase disk protein of 60 kDa</td><td>1</td><td>17406755</td><td>17439754</td></tr>
<tr><td>NM_020195.2</td><td>56948</td><td>SDR39U1</td><td>short chain dehydrogenase/reductase family 39U, member 1</td><td>epimerase family protein SDR39U1</td><td>14</td><td>24439766</td><td>24442801</td></tr>
</table>

<table>
<tr><td>NM_022373.4</td><td>64224</td><td>HERPUD2</td><td>HERPUD family member 2</td><td>homocysteine-inducible, endoplasmic reticulum stress-inducible, ubiquitin-like domain member 2|homocysteine-responsive endoplasmic reticulum-resident ubiquitin-like domain member 2 protein</td><td>7</td><td>35632659</td><td>35695162</td></tr>
<tr><td>NM_032476.3</td><td>64968</td><td>MRPS6</td><td>mitochondrial ribosomal protein S6</td><td>28S ribosomal protein S6, mitochondrial</td><td>21</td><td>34073523</td><td>34143034</td></tr>
<tr><td>NM_023935.1</td><td>65992</td><td>DDRGK1</td><td>DDRGK domain containing 1</td><td>DDRGK domain-containing protein 1|Dashurin|UFM1-binding protein 1 containing a PCI domain</td><td>20</td><td>3190366</td><td>3204649</td></tr>
<tr><td>NM_024065.4</td><td>79031</td><td>PDCL3</td><td>phosducin-like 3</td><td>IAP-associated factor VIAF1|VIAF-1|phPL3|phosducin-like protein 3|viral IAP-associated factor 1</td><td>2</td><td>100562956</td><td>100576739</td></tr>
<tr><td>NM_030915.3</td><td>81606</td><td>LBH</td><td>limb bud and heart development</td><td>hLBH|limb bud and heart development homolog|protein LBH</td><td>2</td><td>30231531</td><td>30260033</td></tr>
<tr><td>NM_030935.3</td><td>81628</td><td>TSC22D4</td><td>TSC22 domain family, member 4</td><td>TSC22 domain family 4|TSC22 domain family protein 4|TSC22-related-inducible leucine zipper protein 2|tsc-22-like protein THG-1</td><td>7</td><td>100466519</td><td>100479279</td></tr>
<tr><td>NM_031210.5</td><td>81892</td><td>SLIRP</td><td>SRA stem-loop interacting RNA binding protein</td><td>SRA stem-loop-interacting RNA-binding protein, mitochondrial</td><td>14</td><td>77708071</td><td>77717598</td></tr>
<tr><td>NM_032389.4</td><td>84364</td><td>ARFGAP2</td><td>ADP-ribosylation factor GTPase activating protein 2</td><td>ADP-ribosylation factor GTPase-activating protein 2|GTPase-activating protein ZNF289|zinc finger protein 289, ID1 regulated</td><td>11</td><td>47164298</td><td>47177503</td></tr>
<tr><td>NM_174889.4</td><td>91942</td><td>NDUFAF2</td><td>NADH dehydrogenase (ubiquinone) complex I, assembly factor 2</td><td>Myc-induced mitochondrial protein|NADH dehydrogenase (ubiquinone) 1 alpha subcomplex, assembly factor 2|NDUFA12-like protein|mimitin, mitochondrial</td><td>5</td><td>60945129</td><td>61153037</td></tr>
<tr><td>NM_080667.5</td><td>112942</td><td>CCDC104</td><td>coiled-coil domain containing 104</td><td>coiled-coil domain-containing protein 104</td><td>2</td><td>55519595</td><td>55545080</td></tr>
<tr><td>NM_172251.2</td><td>116541</td><td>MRPL54</td><td>mitochondrial ribosomal protein L54</td><td>39S ribosomal protein L54, mitochondrial</td><td>19</td><td>3762667</td><td>3767565</td></tr>
<tr><td>NM_173809.4</td><td>282991</td><td>BLOC1S2</td><td>biogenesis of lysosomal organelles complex-1, subunit 2</td><td>11 kDa centrosome associated protein|BLOC-1 subunit 2|biogenesis of lysosome-related organelles complex 1 subunit 2|centrosomal 10 kDa protein|centrosome protein oncogene</td><td>10</td><td>100273278</td><td>100286712</td></tr>
<tr><td>NM_001039844.2</td><td>414149</td><td>ACBD7</td><td>acyl-CoA binding domain containing 7</td><td>acyl-CoA-binding domain-containing protein 7|acyl-Coenzyme A binding domain containing 7</td><td>10</td><td>15075475</td><td>15088776</td></tr>
<tr><td>NM_001089591.1</td><td>440567</td><td>UQCRHL</td><td>ubiquinol-cytochrome c reductase hinge protein-like</td><td>hCG25371</td><td>1</td><td>15807162</td><td>15807699</td></tr>
<tr><td>NM_001099415.2</td><td>100101267</td><td>POM121C</td><td>POM121 transmembrane nucleoporin C</td><td>POM121 membrane glycoprotein C|nuclear envelope pore membrane protein POM 121C|nuclear pore membrane protein 121-2|pore membrane protein of 121 kDa C</td><td>7</td><td>75416781</td><td>75486288</td></tr>
<tr><td>NM_001271592.1</td><td>100129361</td><td>LOC100129361</td><td>chromosome X open reading frame 69-like</td><td>uncharacterized protein LOC100129361</td><td>12</td><td>11171181</td><td>11176020</td></tr>
<tr><td>NM_001018024.2</td><td>100272147</td><td>CMC4</td><td>C-x(9)-C motif containing 4</td><td>C-x(9)-C motif containing 4 homolog|MTCP-1 type A|cx9C motif-containing protein 4|mature T-cell proliferation 1|mature T-cell proliferation 1 neighbor protein|mature T-cell proliferation-1 type A|protein p8 MTCP-1</td><td>X</td><td>155061622</td><td>155071272</td></tr>
<tr><td>NM_001190470.1</td><td>100462981</td><td>MTRNR2L2</td><td>MT-RNR2-like 2</td><td>MTRNR2-like 2|humanin-like 2|humanin-like protein 2</td><td>5</td><td>80650000</td><td>80651035</td></tr>
</table>

Figure 9

<table>
<tr><th>Id</th><th>GeneID</th><th>Symbol</th><th>description</th><th>other_designations</th><th>chromosome</th><th>start_position</th><th>end_position</th></tr>
<tr><td>ADH5</td><td>128</td><td>ADH5</td><td>alcohol dehydrogenase 5 (class III), chi polypeptide</td><td>S-(hydroxymethyl)glutathione dehydrogenase|alcohol dehydrogenase (class III), chi polypeptide|alcohol dehydrogenase class chi chain|alcohol dehydrogenase class-3|alcohol dehydrogenase class-III|formaldehyde dehydrogenase|glutathione-dependent formaldehyde</td><td>4</td><td>99070978</td><td>99088788</td></tr>
<tr><td>BIN1</td><td>274</td><td>BIN1</td><td>bridging integrator 1</td><td>amphiphysin II|amphiphysin-like protein|box dependant MYC interacting protein 1|box-dependent myc-interacting protein 1|myc box-dependent-interacting protein 1</td><td>2</td><td>127048023</td><td>127107327</td></tr>
<tr><td>ANXA7</td><td>310</td><td>ANXA7</td><td>annexin A7</td><td>annexin VII|annexin-7</td><td>10</td><td>73375431</td><td>73414083</td></tr>
<tr><td>BCL2A1</td><td>597</td><td>BCL2A1</td><td>BCL2-related protein A1</td><td>bcl-2-like protein 5|bcl-2-related protein A1|bcl2-L-5|hematopoietic BCL2-related protein A1|hemopoietic-specific early response protein|protein BFL-1</td><td>15</td><td>79960890</td><td>79971301</td></tr>
<tr><td>CEBPB</td><td>1051</td><td>CEBPB</td><td>CCAAT/enhancer binding protein (C/EBP), beta</td><td>CCAAT/enhancer-binding protein beta|interleukin 6-dependent DNA-binding protein|nuclear factor NF-IL6|nuclear factor of interleukin 6|transcription factor 5</td><td>20</td><td>50190583</td><td>50192690</td></tr>
<tr><td>CSF2</td><td>1437</td><td>CSF2</td><td>colony stimulating factor 2 (granulocyte-macrophage)</td><td>CSF|granulocyte-macrophage colony-stimulating factor|molgramostin|sargramostim</td><td>5</td><td>132073792</td><td>132076170</td></tr>
<tr><td>CTLA4</td><td>1493</td><td>CTLA4</td><td>cytotoxic T-lymphocyte-associated protein 4</td><td>CD152 isoform|celiac disease 3|cytotoxic T lymphocyte associated antigen 4 short spliced form|cytotoxic T-lymphocyte antigen 4|cytotoxic T-lymphocyte protein 4|cytotoxic T-lymphocyte-associated antigen 4|cytotoxic T-lymphocyte-associated serine esterase-4|insulin-dependent diabetes mellitus 12|ligand and transmembrane spliced cytotoxic T lymphocyte associated antigen 4</td><td>2</td><td>203867788</td><td>203873960</td></tr>
<tr><td>FABP7</td><td>2173</td><td>FABP7</td><td>fatty acid binding protein 7, brain</td><td>Hypothetical protein DKFZp547J2313|brain lipid binding protein|brain lipid-binding protein|brain-type fatty acid-binding protein|fatty acid-binding protein 7|fatty acid-binding protein, brain|mammary-derived growth inhibitor related|mammary-derived growth inhibitor-related</td><td>6</td><td>122779501</td><td>122784074</td></tr>
<tr><td>GBP1</td><td>2633</td><td>GBP1</td><td>guanylate binding protein 1, interferon-inducible</td><td>GBP-1|GTP-binding protein 1|guanine nucleotide-binding protein 1|guanylate binding protein 1, interferon-inducible, 67kDa|huGBP-1|interferon-induced guanylate-binding protein 1</td><td>1</td><td>89052304</td><td>89065360</td></tr>
<tr><td>GZMH</td><td>2999</td><td>GZMH</td><td>granzyme H (cathepsin G-like 2, protein h-CCPX)</td><td>cytotoxic T-lymphocyte proteinase|cytotoxic T-lymphocyte-associated serine esterase 1|cytotoxin serine protease-C|granzyme H</td><td>14</td><td>24606480</td><td>24609720</td></tr>
<tr><td>GZMB</td><td>3002</td><td>GZMB</td><td>granzyme B (granzyme 2, cytotoxic T-lymphocyte-associated serine esterase 1)</td><td>C11|CTLA-1|T-cell serine protease 1-3E|cathepsin G-like 1|cytotoxic T-lymphocyte proteinase 2|cytotoxic serine protease B|fragmentin 2|fragmentin-2|granzyme B|human lymphocyte protein</td><td>14</td><td>24630954</td><td>24634226</td></tr>
<tr><td>HLA-DQA2</td><td>3118</td><td>HLA-DQA2</td><td>major histocompatibility complex, class II, DQ alpha 2</td><td>DX alpha chain|HLA class II histocompatibility antigen, DQ alpha 2 chain|HLA class II histocompatibility antigen, DQ(6) alpha chain|MHC class II DQA2</td><td>6</td><td>32741386</td><td>32746887</td></tr>
<tr><td>HSD11B1</td><td>3290</td><td>HSD11B1</td><td>hydroxysteroid (11-beta) dehydrogenase 1</td><td>corticosteroid 11-beta-dehydrogenase isozyme 1|short chain dehydrogenase/reductase family 26C, member 1</td><td>1</td><td>209686180</td><td>209734950</td></tr>
</table>

<table>
<tr><td>IFNB1</td><td>3456</td><td>IFNB1</td><td>interferon, beta 1, fibroblast</td><td>IFN-beta|fibroblast interferon|interferon beta</td><td>9</td><td>21077105</td><td>21077944</td></tr>
<tr><td>IFNG</td><td>3458</td><td>IFNG</td><td>interferon, gamma</td><td>IFN-gamma|immune interferon|interferon gamma</td><td>12</td><td>68154770</td><td>68159741</td></tr>
<tr><td>IDO1</td><td>3620</td><td>IDO1</td><td>indoleamine 2,3-dioxygenase 1</td><td>indolamine 2,3 dioxygenase|indole 2,3-dioxygenase|indoleamine-pyrrole 2,3-dioxygenase</td><td>8</td><td>39913809</td><td>39928790</td></tr>
<tr><td>CXCL10</td><td>3627</td><td>CXCL10</td><td>chemokine (C-X-C motif) ligand 10</td><td>10 kDa interferon gamma-induced protein|C-X-C motif chemokine 10|gamma IP10|gamma-IP10|interferon-inducible cytokine IP-10|protein 10 from interferon (gamma)-induced cell line|small inducible cytokine subfamily B (Cys-X-Cys), member 10|small-inducible cytokine B10</td><td>4</td><td>76021116</td><td>76023536</td></tr>
<tr><td>IRF2</td><td>3660</td><td>IRF2</td><td>interferon regulatory factor 2</td><td></td><td>4</td><td>184387713</td><td>184474580</td></tr>
<tr><td>KIR2DL4</td><td>3805</td><td>KIR2DL4</td><td>killer cell immunoglobulin-like receptor, two domains, long cytoplasmic tail, 4</td><td>CD158 antigen-like family member D|MHC class I NK cell receptor KIR103AS|NK cell receptor|killer Ig receptor|killer cell immunoglobulin-like receptor 2DL4|killer cell inhibitory receptor 103AS|natural killer cell inhibitory receptor</td><td>19</td><td>54803612</td><td>54814517</td></tr>
<tr><td>LAG3</td><td>3902</td><td>LAG3</td><td>lymphocyte-activation gene 3</td><td>lymphocyte activation gene 3 protein</td><td>12</td><td>6772504</td><td>6778455</td></tr>
<tr><td>MGMT</td><td>4255</td><td>MGMT</td><td>O-6-methylguanine-DNA methyltransferase</td><td>6-O-methylguanine-DNA methyltransferase|O-6-methylguanine-DNA-alkyltransferase|O6-methylguanine-DNA methyltransferase|methylated-DNA--protein-cysteine methyltransferase|methylguanine-DNA methyltransferase</td><td>10</td><td>129467190</td><td>129767519</td></tr>
<tr><td>CXCL9</td><td>4283</td><td>CXCL9</td><td>chemokine (C-X-C motif) ligand 9</td><td>C-X-C motif chemokine 9|gamma-interferon-induced monokine|monokine induced by gamma interferon|monokine induced by interferon-gamma|small-inducible cytokine B9</td><td>4</td><td>76001470</td><td>76007488</td></tr>
<tr><td>MT1H</td><td>4496</td><td>MT1H</td><td>metallothionein 1H</td><td>metallothionein-0|metallothionein-1H|metallothionein-IH</td><td>16</td><td>56669814</td><td>56671129</td></tr>
<tr><td>MYO7A</td><td>4647</td><td>MYO7A</td><td>myosin VIIA</td><td>myosin VIIA (Usher syndrome 1B (autosomal recessive, severe))|unconventional myosin-VIIa</td><td>11</td><td>77128256</td><td>77215241</td></tr>
<tr><td>NDP</td><td>4693</td><td>NDP</td><td>Norrie disease (pseudoglioma)</td><td>X-linked exudative vitreoretinopathy 2 protein|norrie disease protein|norrin</td><td>X</td><td>43948776</td><td>43973675</td></tr>
<tr><td>NFKBIA</td><td>4792</td><td>NFKBIA</td><td>nuclear factor of kappa light polypeptide gene enhancer in B-cells inhibitor, alpha</td><td>I-kappa-B-alpha|IkappaBalpha|NF-kappa-B inhibitor alpha|ikB-alpha|major histocompatibility complex enhancer-binding protein MAD3|nuclear factor of kappa light chain gene enhancer in B-cells</td><td>14</td><td>35401510</td><td>35404754</td></tr>
<tr><td>PDCD1</td><td>5133</td><td>PDCD1</td><td>programmed cell death 1</td><td>programmed cell death protein 1|protein PD-1|systemic lupus erythematosus susceptibility 2</td><td>2</td><td>241849881</td><td>241858908</td></tr>
<tr><td>PDK1</td><td>5163</td><td>PDK1</td><td>pyruvate dehydrogenase kinase, isozyme 1</td><td>PDH kinase 1|[Pyruvate dehydrogenase (acetyl-transferring)] kinase isozyme 1, mitochondrial|mitochondrial pyruvate dehydrogenase, lipoamide, kinase isoenzyme 1|pyruvate dehydrogenase kinase, isoenzyme 1</td><td>2</td><td>172555373</td><td>172625623</td></tr>
<tr><td>PENK</td><td>5179</td><td>PENK</td><td>proenkephalin</td><td>enkephalin A|preproenkephalin|proenkephalin-A</td><td>8</td><td>56440954</td><td>56446723</td></tr>
<tr><td>PFDN1</td><td>5201</td><td>PFDN1</td><td>prefoldin subunit 1</td><td>prefoldin 1</td><td>5</td><td>140245050</td><td>140303104</td></tr>
<tr><td>MAP2K6</td><td>5608</td><td>MAP2K6</td><td>mitogen-activated protein kinase kinase 6</td><td>MAPK/ERK kinase 6|MAPKK 6|MEK 6|SAPK kinase 3|dual specificity mitogen-activated protein kinase kinase 6|protein kinase, mitogen-activated, kinase 6 (MAP kinase kinase 6)|stress-activated protein kinase kinase 3</td><td>17</td><td>69414697</td><td>69542329</td></tr>
</table>

<table>
<tr><td>PRSS1</td><td>5644</td><td>PRSS1</td><td>protease, serine, 1 (trypsin 1)</td><td>beta-trypsin|cationic trypsinogen|digestive zymogen|nonfunctional trypsin 1|trypsin-1|trypsinogen</td><td>7</td><td>142749468</td><td>142753076</td></tr>
<tr><td>PSMB9</td><td>5698</td><td>PSMB9</td><td>proteasome (prosome, macropain) subunit, beta type, 9</td><td>large multifunctional peptidase 2|low molecular mass protein 2|macropain chain 7|multicatalytic endopeptidase complex chain 7|proteasome (prosome, macropain) subunit, beta type, 9 (large multifunctional peptidase 2)|proteasome catalytic subunit 1i|proteasome chain 7|proteasome subunit beta 6i|proteasome subunit beta type-9|proteasome subunit beta-1i|proteasome-related gene 2|really interesting new gene 12 protein</td><td>6</td><td>32854161</td><td>32859851</td></tr>
<tr><td>PSME1</td><td>5720</td><td>PSME1</td><td>proteasome (prosome, macropain) activator subunit 1 (PA28 alpha)</td><td>11S regulator complex subunit alpha|29-kD MCP activator subunit|IGUP I-5111|activator of multicatalytic protease subunit 1|interferon gamma up-regulated I-5111 protein|interferon-gamma IEF SSP 5111|interferon-gamma-inducible protein 5111|proteasome activator complex subunit 1</td><td>14</td><td>24136158</td><td>24138967</td></tr>
<tr><td>PSME2</td><td>5721</td><td>PSME2</td><td>proteasome (prosome, macropain) activator subunit 2 (PA28 beta)</td><td>11S regulator complex beta subunit|11S regulator complex subunit beta|MCP activator, 31-kD subunit|REG-beta|activator of multicatalytic protease subunit 2|cell migration-inducing protein 22|proteasome activator 28 subunit beta|proteasome activator 28-beta|proteasome activator complex subunit 2|proteasome activator hPA28 subunit beta</td><td>14</td><td>24143365</td><td>24147316</td></tr>
<tr><td>PTPN2</td><td>5771</td><td>PTPN2</td><td>protein tyrosine phosphatase, non-receptor type 2</td><td>T-cell protein tyrosine phosphatase|tyrosine-protein phosphatase non-receptor type 2</td><td>18</td><td>12785478</td><td>12884338</td></tr>
<tr><td>RLN1</td><td>6013</td><td>RLN1</td><td>relaxin 1</td><td>preprorelaxin H1|prorelaxin H1</td><td>9</td><td>5307637</td><td>5339873</td></tr>
<tr><td>CCL4</td><td>6351</td><td>CCL4</td><td>chemokine (C-C motif) ligand 4</td><td>C-C motif chemokine 4|G-26 T-lymphocyte-secreted protein|MIP-1-beta(1-69)|PAT 744|SIS-gamma|T-cell activation protein 2|lymphocyte activation gene 1 protein|macrophage inflammatory protein 1-beta|secreted protein G-26|small inducible cytokine A4 (homologous to mouse Mip-1b)</td><td>17</td><td>36103827</td><td>36105621</td></tr>
<tr><td>CCL5</td><td>6352</td><td>CCL5</td><td>chemokine (C-C motif) ligand 5</td><td>C-C motif chemokine 5|T-cell specific protein p288|beta-chemokine RANTES|eosinophil chemotactic cytokine|regulated upon activation, normally T-expressed, and presumably secreted|small inducible cytokine subfamily A (Cys-Cys), member 5</td><td>17</td><td>35871491</td><td>35880373</td></tr>
<tr><td>CCL13</td><td>6357</td><td>CCL13</td><td>chemokine (C-C motif) ligand 13</td><td>C-C motif chemokine 13|CK-beta-10|monocyte chemoattractant protein 4|monocyte chemotactic protein 4|new CC chemokine 1|small inducible cytokine subfamily A (Cys-Cys), member 13|small-inducible cytokine A13</td><td>17</td><td>34356452</td><td>34358610</td></tr>
<tr><td>CCL18</td><td>6362</td><td>CCL18</td><td>chemokine (C-C motif) ligand 18 (pulmonary and activation-regulated)</td><td>C-C motif chemokine 18|CC chemokine PARC|CC chemokine ligand 18|alternative macrophage activation-associated CC chemokine 1|chemokine (C-C), dendritic|dendritic cell chemokine 1|macrophage inflammatory protein 4|pulmonary and activation-regulated chemokine|small inducible cytokine A18|small inducible cytokine subfamily A (Cys-Cys), member 18, pulmonary and activation-regulated|small-inducible cytokine</td><td>17</td><td>36064283</td><td>36071481</td></tr>
</table>

<table>
<tr><td>CCL25</td><td>6370</td><td>CCL25</td><td>chemokine (C-C motif) ligand 25</td><td>C-C motif chemokine 25|Ck beta-15|TECKvar|chemokine TECK|small inducible cytokine subfamily A (Cys-Cys), member 25|small-inducible cytokine A25|thymus expressed chemokine|thymus-expressed chemokine</td><td>19</td><td>8052762</td><td>8062663</td></tr>
<tr><td>CXCL11</td><td>6373</td><td>CXCL11</td><td>chemokine (C-X-C motif) ligand 11</td><td>C-X-C motif chemokine 11|beta-R1|interferon gamma-inducible protein 9|interferon-inducible T-cell alpha chemoattractant|small inducible cytokine B11|small inducible cytokine subfamily B (Cys-X-Cys), member 11|small inducible cytokine subfamily B (Cys-X-Cys), member 9B|small-inducible cytokine B11</td><td>4</td><td>76033682</td><td>76036197</td></tr>
<tr><td>SFTPB</td><td>6439</td><td>SFTPB</td><td>surfactant protein B</td><td>18 kDa pulmonary-surfactant protein|6 kDa protein|pulmonary surfactant-associated protein B|pulmonary surfactant-associated proteolipid SPL(Phe)</td><td>2</td><td>85657317</td><td>85668741</td></tr>
<tr><td>STAT1</td><td>6772</td><td>STAT1</td><td>signal transducer and activator of transcription 1, 91kDa</td><td>signal transducer and activator of transcription 1-alpha/beta|signal transducer and activator of transcription-1|transcription factor ISGF-3 components p91/p84</td><td>2</td><td>190969036</td><td>191014250</td></tr>
<tr><td>TSHR</td><td>7253</td><td>TSHR</td><td>thyroid stimulating hormone receptor</td><td>seven transmembrane helix receptor|thyrotropin receptor|thyrotropin receptor-I, hTSHR-I</td><td>14</td><td>80954989</td><td>81146302</td></tr>
<tr><td>WARS</td><td>7453</td><td>WARS</td><td>tryptophanyl-tRNA synthetase</td><td>hWRS|interferon-induced protein 53|trpRS|tryptophan tRNA ligase 1, cytoplasmic|tryptophan--tRNA ligase, cytoplasmic</td><td>14</td><td>100333788</td><td>100376343</td></tr>
<tr><td>PLA2G7</td><td>7941</td><td>PLA2G7</td><td>phospholipase A2, group VII (platelet-activating factor acetylhydrolase, plasma)</td><td>1-alkyl-2-acetylglycerophosphocholine esterase|2-acetyl-1-alkylglycerophosphocholine esterase|LDL-PLA(2)|LDL-associated phospholipase A2|PAF 2-acylhydrolase|PAF acetylhydrolase|gVIIA-PLA2|group-VIIA phospholipase A2|lipoprotein-associated phospholipase A2|platelet-activating factor acetylhydrolase</td><td>6</td><td>46704316</td><td>46735693</td></tr>
<tr><td>BFSP2</td><td>8419</td><td>BFSP2</td><td>beaded filament structural protein 2, phakinin</td><td>49 kDa cytoskeletal protein|beaded filament protein CP49|bfps2, Cytoskeletal protein, 49-kD|lens fiber cell beaded filament protein CP 47|lens fiber cell beaded filament protein CP 49|lens intermediate filament-like light|phakinin</td><td>3</td><td>133399995</td><td>133475212</td></tr>
<tr><td>OASL</td><td>8638</td><td>OASL</td><td>2'-5'-oligoadenylate synthetase-like</td><td>2'-5'-OAS-RP|2'-5'-OAS-related protein|2'-5'-oligoadenylate synthase-like protein|59 kDa 2'-5'-oligoadenylate synthase-like protein|59 kDa 2'-5'-oligoadenylate synthetase-like protein|TR-interacting protein 14|thyroid receptor-interacting protein 14</td><td>12</td><td>121020292</td><td>121039242</td></tr>
<tr><td>BUB3</td><td>9184</td><td>BUB3</td><td>BUB3 mitotic checkpoint protein</td><td>BUB3 budding uninhibited by benzimidazoles 3 homolog|budding uninhibited by benomyl|budding uninhibited by benzimidazoles 3 homolog|mitotic checkpoint component|mitotic checkpoint protein BUB3</td><td>10</td><td>123154244</td><td>123165370</td></tr>
<tr><td>UBE2L6</td><td>9246</td><td>UBE2L6</td><td>ubiquitin-conjugating enzyme E2L 6</td><td>retinoic acid induced gene B protein|retinoic acid-induced gene B protein|ubiquitin carrier protein L6|ubiquitin-protein ligase L6|ubiquitin/ISG15-conjugating enzyme E2 L6</td><td>11</td><td>57551655</td><td>57568330</td></tr>
<tr><td>SH3BP5</td><td>9467</td><td>SH3BP5</td><td>SH3-domain binding protein 5 (BTK-associated)</td><td>SH3 domain-binding protein 5|SH3 domain-binding protein that preferentially associates with BTK</td><td>3</td><td>15254353</td><td>15341394</td></tr>
<tr><td>PSMF1</td><td>9491</td><td>PSMF1</td><td>proteasome (prosome, macropain) inhibitor subunit 1 (PI31)</td><td>hPI31|proteasome inhibitor PI31 subunit|proteasome inhibitor hP131 subunit</td><td>20</td><td>1113263</td><td>1168373</td></tr>
<tr><td>FAM53B</td><td>9679</td><td>FAM53B</td><td>family with sequence similarity 53, member B</td><td>protein FAM53B</td><td>10</td><td>124619294</td><td>124744361</td></tr>
</table>

<table>
<tr><td>CXCL13</td><td>10563</td><td>CXCL13</td><td>chemokine (C-X-C motif) ligand 13</td><td>B-cell chemoattractant|B-cell-attracting chemokine 1|B-cell-homing chemokine (ligand for Burkitt's lymphoma receptor-1)|B-lymphocyte chemoattractant|C-X-C motif chemokine 13|CXC chemokine BLC|b cell-attracting chemokine 1|b lymphocyte chemoattractant|chemokine (C-X-C motif) ligand 13 (B-cell chemoattractant)|small inducible cytokine B subfamily (Cys-X-Cys motif), member 13 (B-cell chemoattractant)|small-inducible cytokine B13</td><td>4</td><td>77511753</td><td>77611834</td></tr>
<tr><td>CXCR6</td><td>10663</td><td>CXCR6</td><td>chemokine (C-X-C motif) receptor 6</td><td>C-X-C chemokine receptor type 6|CDw186|CXC-R6|CXCR-6|G protein-coupled receptor|G-protein coupled receptor STRL33|G-protein coupled receptor bonzo</td><td>3</td><td>45943481</td><td>45948353</td></tr>
<tr><td>EGFL6</td><td>25975</td><td>EGFL6</td><td>EGF-like-domain, multiple 6</td><td>EGF repeat-containing protein 6|EGF-like protein 6|MAM and EGF domain containing|MAM and EGF domains-containing gene protein|epidermal growth factor-like protein 6</td><td>X</td><td>13569575</td><td>13633575</td></tr>
<tr><td>CLEC4E</td><td>26253</td><td>CLEC4E</td><td>C-type lectin domain family 4, member E</td><td>C-type (calcium dependent, carbohydrate-recognition domain) lectin, superfamily member 9|C-type lectin domain family 4 member E|C-type lectin superfamily member 9|macrophage-inducible C-type lectin</td><td>12</td><td>8533305</td><td>8540962</td></tr>
<tr><td>FBXO6</td><td>26270</td><td>FBXO6</td><td>F-box protein 6</td><td>F-box only protein 6|F-box protein FBG2|F-box protein Fbx6|F-box protein that recognizes sugar chains 2|F-box/G-domain protein 2</td><td>1</td><td>11664093</td><td>11674354</td></tr>
<tr><td>LAMP3</td><td>27074</td><td>LAMP3</td><td>lysosomal-associated membrane protein 3</td><td>DC-lysosome-associated membrane glycoprotein|lysosome-associated membrane glycoprotein 3</td><td>3</td><td>183122215</td><td>183162879</td></tr>
<tr><td>SNX10</td><td>29887</td><td>SNX10</td><td>sorting nexin 10</td><td>sorting nexin-10</td><td>7</td><td>26291895</td><td>26374383</td></tr>
<tr><td>SERTAD3</td><td>29946</td><td>SERTAD3</td><td>SERTA domain containing 3</td><td>RPA-binding trans-activator|SERTA domain-containing protein 3|replication protein-binding trans-activator</td><td>19</td><td>40440841</td><td>40444375</td></tr>
<tr><td>FAM20A</td><td>54757</td><td>FAM20A</td><td>family with sequence similarity 20, member A</td><td>protein FAM20A</td><td>17</td><td>68535116</td><td>68600954</td></tr>
<tr><td>SLC29A3</td><td>55315</td><td>SLC29A3</td><td>solute carrier family 29 (equilibrative nucleoside transporter), member 3</td><td>equilibrative nucleoside transporter 3|solute carrier family 29 (nucleoside transporters), member 3</td><td>10</td><td>71319253</td><td>71363390</td></tr>
<tr><td>C19orf66</td><td>55337</td><td>C19orf66</td><td>chromosome 19 open reading frame 66</td><td>UPF0515 protein C19orf66</td><td>19</td><td>10086130</td><td>10093252</td></tr>
<tr><td>PPP2R2D</td><td>55844</td><td>PPP2R2D</td><td>protein phosphatase 2, regulatory subunit B, delta</td><td>PP2A subunit B isoform B55-delta|PP2A subunit B isoform PR55-delta|PP2A subunit B isoform R2-delta|PP2A subunit B isoform delta|protein phosphatase 2, regulatory subunit B, delta isoform|serine/threonine-protein phosphatase 2A 55 kDa regulatory subunit B delta isoform</td><td>10</td><td>131900644</td><td>131969946</td></tr>
<tr><td>BCCIP</td><td>56647</td><td>BCCIP</td><td>BRCA2 and CDKN1A interacting protein</td><td>BCCIPalpha|BCCIPbeta|BRCA2 and CDKN1A-interacting protein|BRCA2 and Cip1/p21 interacting protein|TOK-1alpha|TOK-1beta|cdk inhibitor p21 binding protein|p21- and CDK-associated protein 1|protein TOK-1</td><td>10</td><td>125823535</td><td>125853695</td></tr>
<tr><td>C18orf2</td><td>56651</td><td>LINC00470</td><td>long intergenic non-protein coding RNA 470</td><td></td><td>18</td><td>1268311</td><td>1359629</td></tr>
<tr><td>PARP12</td><td>64761</td><td>PARP12</td><td>poly (ADP-ribose) polymerase family, member 12</td><td>ADP-ribosyltransferase diphtheria toxin-like 12|poly [ADP-ribose] polymerase 12|zinc finger CCCH domain-containing protein 1|zinc finger CCCH type domain containing 1</td><td>7</td><td>140023744</td><td>140064286</td></tr>
<tr><td>C11orf48</td><td>79081</td><td>C11orf48</td><td>chromosome 11 open reading frame 48</td><td>uncharacterized protein C11orf48</td><td>11</td><td>62662817</td><td>62671769</td></tr>
</table>

<table>
<tr><td>ZBED2</td><td>79413</td><td>ZBED2</td><td>zinc finger, BED-type containing 2</td><td>zinc finger BED domain-containing protein 2|zinc finger, BED domain containing 2</td><td>3</td><td>111592900</td><td>111595335</td></tr>
<tr><td>AGBL2</td><td>79841</td><td>AGBL2</td><td>ATP/GTP binding protein-like 2</td><td>cytosolic carboxypeptidase 2</td><td>11</td><td>47659486</td><td>47715376</td></tr>
<tr><td>TMEM163</td><td>81615</td><td>TMEM163</td><td>transmembrane protein 163</td><td></td><td>2</td><td>134455759</td><td>134719001</td></tr>
<tr><td>ELOVL3</td><td>83401</td><td>ELOVL3</td><td>ELOVL fatty acid elongase 3</td><td>3-keto acyl-CoA synthase ELOVL3|ELOVL FA elongase 3|cold-inducible glycoprotein of 30 kDa|elongation of very long chain fatty acids (FEN1/Elo2, SUR4/Elo3, yeast)-like 3|elongation of very long chain fatty acids protein 3|very-long-chain 3-oxoacyl-CoA synthase 3</td><td>10</td><td>102226278</td><td>102229589</td></tr>
<tr><td>RTBDN</td><td>83546</td><td>RTBDN</td><td>retbindin</td><td></td><td>19</td><td>12825477</td><td>12835428</td></tr>
<tr><td>RTP3</td><td>83597</td><td>RTP3</td><td>receptor (chemosensory) transporter protein 3</td><td>receptor transporter protein 3|receptor transporting protein 3|receptor-transporting protein 3|transmembrane protein 7|zinc finger, 3CxxC-type 3</td><td>3</td><td>46497995</td><td>46500949</td></tr>
<tr><td>PCGF6</td><td>84108</td><td>PCGF6</td><td>polycomb group ring finger 6</td><td>Mel18 and Bmi1-like RING finger protein|polycomb group RING finger protein 6|ring finger protein 134</td><td>10</td><td>103302796</td><td>103351134</td></tr>
<tr><td>MAP1LC3A</td><td>84557</td><td>MAP1LC3A</td><td>microtubule-associated protein 1 light chain 3 alpha</td><td>MAP1 light chain 3-like protein 1|MAP1A/1B light chain 3 A|MAP1A/MAP1B LC3 A|MAP1A/MAP1B light chain 3 A|autophagy-related ubiquitin-like modifier LC3 A|microtubule-associated proteins 1A/1B light chain 3|microtubule-associated proteins 1A/1B light chain 3A</td><td>20</td><td>34546884</td><td>34560345</td></tr>
<tr><td>AFAP1L2</td><td>84632</td><td>AFAP1L2</td><td>actin filament associated protein 1-like 2</td><td>AFAP1-like protein 2|actin filament-associated protein 1-like 2</td><td>10</td><td>114281494</td><td>114404778</td></tr>
<tr><td>TUBA1C</td><td>84790</td><td>TUBA1C</td><td>tubulin, alpha 1c</td><td>alpha-tubulin 6|tubulin alpha-1C chain|tubulin alpha-6 chain|tubulin, alpha 6</td><td>12</td><td>49265082</td><td>49273330</td></tr>
<tr><td>TMEM60</td><td>85025</td><td>TMEM60</td><td>transmembrane protein 60</td><td></td><td>7</td><td>77793728</td><td>77798430</td></tr>
<tr><td>KLHDC7B</td><td>113730</td><td>KLHDC7B</td><td>kelch domain containing 7B</td><td>kelch domain-containing protein 7B</td><td>22</td><td>50548033</td><td>50551023</td></tr>
<tr><td>ODF3</td><td>113746</td><td>ODF3</td><td>outer dense fiber of sperm tails 3</td><td>cancer/testis antigen 135|outer dense fiber protein 3|sperm tail protein SHIPPO1|transcript induced in spermiogenesis protein 50</td><td>11</td><td>196761</td><td>200258</td></tr>
<tr><td>C21orf88</td><td>114041</td><td>C21orf88</td><td>chromosome 21 open reading frame 88</td><td></td><td>21</td><td>39597147</td><td>39612822</td></tr>
<tr><td>BATF2</td><td>116071</td><td>BATF2</td><td>basic leucine zipper transcription factor, ATF-like 2</td><td>B-ATF-2|basic leucine zipper transcriptional factor ATF-like 2|suppressor of AP-1 regulated by IFN</td><td>11</td><td>64987945</td><td>64997045</td></tr>
<tr><td>ZNF511</td><td>118472</td><td>ZNF511</td><td>zinc finger protein 511</td><td></td><td>10</td><td>133308919</td><td>133313162</td></tr>
<tr><td>MMP21</td><td>118856</td><td>MMP21</td><td>matrix metallopeptidase 21</td><td>matrix metalloproteinase 21|matrix metalloproteinase-21</td><td>10</td><td>125766453</td><td>125775821</td></tr>
<tr><td>ANKRD22</td><td>118932</td><td>ANKRD22</td><td>ankyrin repeat domain 22</td><td>ankyrin repeat domain-containing protein 22</td><td>10</td><td>88819899</td><td>88851975</td></tr>
<tr><td>ISCA2</td><td>122961</td><td>ISCA2</td><td>iron-sulfur cluster assembly 2</td><td>HESB-like domain-containing protein 1|iron-sulfur cluster assembly 2 homolog, mitochondrial</td><td>14</td><td>74493720</td><td>74495568</td></tr>
<tr><td>ZPBP2</td><td>124626</td><td>ZPBP2</td><td>zona pellucida binding protein 2</td><td>ZPBP-like protein|zona pellucida-binding protein 2</td><td>17</td><td>39868164</td><td>39877896</td></tr>
<tr><td>C1orf88</td><td>128344</td><td>PIFO</td><td>primary cilia formation</td><td>protein pitchfork</td><td>1</td><td>111324663</td><td>111353017</td></tr>
<tr><td>MITD1</td><td>129531</td><td>MITD1</td><td>MIT, microtubule interacting and transport, domain containing 1</td><td>MIT domain-containing protein 1</td><td>2</td><td>99169263</td><td>99181035</td></tr>
<tr><td>C7orf13</td><td>129790</td><td>C7orf13</td><td>chromosome 7 open reading frame 13</td><td></td><td>7</td><td>156638366</td><td>156640654</td></tr>
</table>

<table>
<tr><td>GALM</td><td>130589</td><td>GALM</td><td>galactose mutarotase (aldose 1-epimerase)</td><td>aldose 1-epimerase|epididymis secretory sperm binding protein Li 63p|galactomutarotase</td><td>2</td><td>38665910</td><td>38734767</td></tr>
<tr><td>LRGUK</td><td>136332</td><td>LRGUK</td><td>leucine-rich repeats and guanylate kinase domain containing</td><td>leucine-rich repeat and guanylate kinase domain-containing protein</td><td>7</td><td>134127352</td><td>134264181</td></tr>
<tr><td>ASB9</td><td>140462</td><td>ASB9</td><td>ankyrin repeat and SOCS box containing 9</td><td>ASB-9|ankyrin repeat and SOCS box protein 9|ankyrin repeat and SOCS box-containing 9|ankyrin repeat and suppressor of cytokine signaling box protein 9</td><td>X</td><td>15243987</td><td>15270467</td></tr>
<tr><td>PAR1</td><td>145624</td><td>PWAR1</td><td>Prader Willi/Angelman region RNA 1</td><td></td><td>15</td><td>25135642</td><td>25138053</td></tr>
<tr><td>GTSF1L</td><td>149699</td><td>GTSF1L</td><td>gametocyte specific factor 1-like</td><td>family with sequence similarity 112, member A|gametocyte-specific factor 1-like</td><td>20</td><td>43726161</td><td>43727007</td></tr>
<tr><td>CSAG1</td><td>158511</td><td>CSAG1</td><td>chondrosarcoma associated gene 1</td><td>cancer/testis antigen 24.1|cancer/testis antigen CSAGE|cancer/testis antigen family 24, member 1|putative chondrosarcoma-associated gene 1 protein</td><td>X</td><td>152727484</td><td>152733735</td></tr>
<tr><td>GBP6</td><td>163351</td><td>GBP6</td><td>guanylate binding protein family, member 6</td><td>GBP-6|GTP-binding protein 6|guanine nucleotide-binding protein 6|guanylate-binding protein 6</td><td>1</td><td>89363758</td><td>89388160</td></tr>
<tr><td>FAM24B</td><td>196792</td><td>FAM24B</td><td>family with sequence similarity 24, member B</td><td>protein FAM24B</td><td>10</td><td>122849094</td><td>122879641</td></tr>
<tr><td>THAP8</td><td>199745</td><td>THAP8</td><td>THAP domain containing 8</td><td>THAP domain-containing protein 8</td><td>19</td><td>36034984</td><td>36054762</td></tr>
<tr><td>FBXL13</td><td>222235</td><td>FBXL13</td><td>F-box and leucine-rich repeat protein 13</td><td>F-box/LRR-repeat protein 13</td><td>7</td><td>102812861</td><td>103074841</td></tr>
<tr><td>AKNAD1</td><td>254268</td><td>AKNAD1</td><td>AKNA domain containing 1</td><td>protein AKNAD1</td><td>1</td><td>108815898</td><td>108858242</td></tr>
<tr><td>COL29A1</td><td>256076</td><td>COL6A5</td><td>collagen, type VI, alpha 5</td><td>collagen alpha-5(VI) chain|collagen, type XXIX, alpha 1|von Willebrand factor A domain-containing protein 4</td><td>3</td><td>130345516</td><td>130484846</td></tr>
<tr><td>ZNF683</td><td>257101</td><td>ZNF683</td><td>zinc finger protein 683</td><td>hypothetical protein MGC33414</td><td>1</td><td>26361634</td><td>26374532</td></tr>
<tr><td>CCDC79</td><td>283847</td><td>CCDC79</td><td>coiled-coil domain containing 79</td><td>coiled-coil domain-containing protein 79|telomere repeats-binding bouquet formation protein 1</td><td>16</td><td>66754596</td><td>66801620</td></tr>
<tr><td>RUFY4</td><td>285180</td><td>RUFY4</td><td>RUN and FYVE domain containing 4</td><td>RUN and FYVE domain-containing protein 4</td><td>2</td><td>218034934</td><td>218090581</td></tr>
<tr><td>C9orf47</td><td>286223</td><td>C9orf47</td><td>chromosome 9 open reading frame 47</td><td>uncharacterized protein C9orf47</td><td>9</td><td>88990863</td><td>88996142</td></tr>
<tr><td>TMEM179B</td><td>374395</td><td>TMEM179B</td><td>transmembrane protein 179B</td><td></td><td>11</td><td>62786809</td><td>62790400</td></tr>
<tr><td>MGC72080</td><td>389538</td><td>MGC72080</td><td>MGC72080 pseudogene</td><td></td><td>7</td><td>97966596</td><td>97972326</td></tr>
<tr><td>USP27X</td><td>389856</td><td>USP27X</td><td>ubiquitin specific peptidase 27, X-linked</td><td>X-linked ubiquitin carboxyl-terminal hydrolase 27|deubiquitinating enzyme 27|ubiquitin carboxyl-terminal hydrolase 27|ubiquitin specific protease 27, X chromosome|ubiquitin thioesterase 27|ubiquitin-specific-processing protease 27</td><td>X</td><td>49879867</td><td>49882565</td></tr>
</table>

| | | | | | | | |
|---|---|---|---|---|---|---|---|
| CSAG3 | 389903 | CSAG3 | CSAG family, member 3 | CSAG family, member 3A\|cancer/testis antigen 24.2\|chondrosarcoma-associated gene 2/3 protein\|taxol resistance associated gene 3\|taxol-resistant-associated gene 3 | X | 152759218 | 152760222 |
| METTL10 | 399818 | METTL10 | methyltransferase like 10 | methyltransferase-like protein 10 | 10 | 124758837 | 124791870 |
| C15orf53 | 400359 | C15orf53 | chromosome 15 open reading frame 53 | uncharacterized protein C15orf53 | 15 | 38696598 | 38700038 |
| LOC400759 | 400759 | GBP1P1 | guanylate binding protein 1, interferon-inducible pseudogene 1 | | 1 | 89407679 | 89424934 |
| C5orf40 | 408263 | FNDC9 | fibronectin type III domain containing 9 | fibronectin type III domain-containing protein 9\|fibronectin type-III domain-containing protein C5orf40 | 5 | 157341599 | 157345721 |
| CARD17 | 440068 | CARD17 | caspase recruitment domain family, member 17 | Inhibitory CARD\|caspase recruitment domain-containing protein 17\|caspase-1 inhibitor INCA\|inhibitory caspase recruitment domain (CARD) protein\|inhibitory caspase recruitment domain protein | 11 | 105092469 | 105101431 |
| C4orf47 | 441054 | C4orf47 | chromosome 4 open reading frame 47 | UPF0602 protein C4orf47 | 4 | 185426028 | 185449828 |
| POTEE | 445582 | POTEE | POTE ankyrin domain family, member E | ANKRD26-like family C member 1A\|ANKRD26-like family C, member 1A\|POTE ankyrin domain family member E\|cancer/testis antigen family 104, member 2\|prostate, ovary, testis-expressed protein on chromosome 2\|protein expressed in prostate, ovary, testis, and placenta 2 | 2 | 131218351 | 131265448 |
| CSAG2 | 728461 | CSAG2 | CSAG family, member 2 | CSAG family, member 3B\|cancer/testis antigen 24.2\|cancer/testis antigen family 24, member 2\|chondrosarcoma-associated gene 2/3 protein\|taxol resistance associated gene 3\|taxol-resistant-associated gene 3 protein | X | NaN | NaN |
| LOC1001889 | 1E+08 | LINC00426 | long intergenic non-protein coding RNA 426 | | 13 | 30340270 | 30373899 |

**Figure 10**

| Id | GeneID | Symbol | description | other_designations | chromosome | start_position | end_position |
|---|---|---|---|---|---|---|---|
| CACNA1S | 779 | CACNA1S | calcium channel, voltage-dependent, L type, alpha 1S subunit | calcium channel, L type, alpha 1 polypeptide, isoform 3 (skeletal muscle, hypokalemic periodic paralysis)\|dihydropyridine receptor\|dihydropyridine-sensitive L-type calcium channel alpha-1 subunit\|voltage-dependent L-type calcium channel subunit alpha-1S\|voltage-gated calcium channel subunit alpha Cav1.1 | 1 | 201039507 | 201112566 |
| DDR1 | 780 | DDR1 | discoidin domain receptor tyrosine kinase 1 | CD167 antigen-like family member A\|PTK3A protein tyrosine kinase 3A\|cell adhesion kinase\|epithelial discoidin domain-containing receptor 1\|mammary carcinoma kinase 10\|neuroepithelial tyrosine kinase\|neurotrophic tyrosine kinase, receptor, type 4\|protein-tyrosine kinase RTK-6\|tyrosine kinase DDR\|tyrosine-protein kinase CAK | 6 | 30880909 | 30900156 |
| CLDN7 | 1366 | CLDN7 | claudin 7 | claudin-7\|clostridium perfringens enterotoxin receptor-like 2 | 17 | 7259903 | 7263193 |
| DUT | 1854 | DUT | deoxyuridine triphosphatase | dUTP nucleotidohydrolase\|dUTP pyrophosphatase\|deoxyuridine 5'-triphosphate nucleotidohydrolase, mitochondrial | 15 | 48331167 | 48343373 |
| EPHB4 | 2050 | EPHB4 | EPH receptor B4 | ephrin receptor EphB4\|ephrin type-B receptor 4\|hepatoma transmembrane kinase\|soluble EPHB4 variant 1\|soluble EPHB4 variant 2\|soluble EPHB4 variant 3\|tyrosine-protein kinase TYRO11\|tyrosine-protein kinase receptor HTK | 7 | 100802565 | 100827521 |
| FGF7 | 2252 | FGF7 | fibroblast growth factor 7 | FGF-7\|heparin-binding growth factor 7\|keratinocyte growth factor | 15 | 49423178 | 49487326 |
| FHL1 | 2273 | FHL1 | four and a half LIM domains 1 | LIM protein SLIMMER\|four and a half LIM domains protein 1\|four-and-a-half Lin11, Isl-1 and Mec-3 domains 1\|skeletal muscle LIM-protein 1 | X | 136146702 | 136211359 |
| GNA11 | 2767 | GNA11 | guanine nucleotide binding protein (G protein), alpha 11 (Gq class) | G-protein subunit alpha-11\|g alpha-11\|guanine nucleotide-binding protein G(y) subunit alpha\|guanine nucleotide-binding protein subunit alpha-11 | 19 | 3094410 | 3124002 |
| HAS1 | 3036 | HAS1 | hyaluronan synthase 1 | HA synthase 1\|huHAS1\|hyaluronate synthase 1\|hyaluronic acid synthase 1 | 19 | 51713112 | 51723986 |
| ID4 | 3400 | ID4 | inhibitor of DNA binding 4, dominant negative helix-loop-helix protein | DNA-binding protein inhibitor ID-4\|class B basic helix-loop-helix protein 27 | 6 | 19837348 | 19842200 |
| IGF2R | 3482 | IGF2R | insulin-like growth factor 2 receptor | 300 kDa mannose 6-phosphate receptor\|CI Man-6-P receptor\|CI-MPR\|IGF-II receptor\|M6P/IGF2 receptor\|M6P/IGF2R\|M6PR\|MPR 300\|cation-independent mannose-6 phosphate receptor\|cation-independent mannose-6-phosphate receptor\|insulin-like growth factor II receptor | 6 | 159969099 | 160106551 |
| ITGB3 | 3690 | ITGB3 | integrin, beta 3 (platelet glycoprotein IIIa, antigen CD61) | integrin beta-3\|platelet membrane glycoprotein IIIa | 17 | 47253842 | 47312711 |
| LAMA4 | 3910 | LAMA4 | laminin, alpha 4 | laminin alpha 4 chain\|laminin subunit alpha-4 | 6 | 112107931 | 112254722 |
| MAP3K4 | 4216 | MAP3K4 | mitogen-activated protein kinase kinase kinase 4 | MAP three kinase 1\|MAP/ERK kinase kinase 4\|MAPK/ERK kinase kinase 4\|MEK kinase 4\|MEKK 4\|SSK2/SSK22 MAP kinase kinase kinase, yeast, homolog of\|dJ473J16.1 (mitogen-activated protein kinase kinase kinase 4)\|predicted protein of HQ0412 | 6 | 160991784 | 161117385 |
| MLLT4 | 4301 | MLLT4 | myeloid/lymphoid or mixed-lineage leukemia (trithorax homolog, Drosophila); translocated to, 4 | ALL1-fused gene from chromosome 6 protein\|afadin\|protein AF-6 | 6 | 167826922 | 167972023 |
| MYO1D | 4642 | MYO1D | myosin ID | myosin-I gamma\|myosin-Id\|unconventional myosin-Id | 17 | 32492610 | 32876884 |

<table>
<tr><th></th><th></th><th></th><th></th><th></th><th></th><th></th><th></th></tr>
<tr><td>NOV</td><td>4856</td><td>NOV</td><td>nephroblastoma overexpressed</td><td>CCN family member 3|IGF-binding protein 9|insulin-like growth factor-binding protein 9|nephroblastoma-overexpressed gene protein homolog|protein NOV homolog</td><td>8</td><td>119416312</td><td>119424438</td></tr>
<tr><td>NTSR1</td><td>4923</td><td>NTSR1</td><td>neurotensin receptor 1 (high affinity)</td><td>NT-R-1|NTR1|NTRH|high-affinity levocabastine-insensitive neurotensin receptor|neurotensin receptor type 1</td><td>20</td><td>62708837</td><td>62762771</td></tr>
<tr><td>PARK2</td><td>5071</td><td>PARK2</td><td>parkin RBR E3 ubiquitin protein ligase</td><td>E3 ubiquitin-protein ligase parkin|Parkinson disease (autosomal recessive, juvenile) 2, parkin|parkinson juvenile disease protein 2|parkinson protein 2, E3 ubiquitin protein ligase (parkin)</td><td>6</td><td>161347558</td><td>162727802</td></tr>
<tr><td>PDE3A</td><td>5139</td><td>PDE3A</td><td>phosphodiesterase 3A, cGMP-inhibited</td><td>cAMP phosphodiesterase, myocardial cGMP-inhibited|cGMP-inhibited 3',5'-cyclic phosphodiesterase A|cyclic GMP-inhibited phosphodiesterase A</td><td>12</td><td>20368087</td><td>20687641</td></tr>
<tr><td>PGM5</td><td>5239</td><td>PGM5</td><td>phosphoglucomutase 5</td><td>PGM-RP|aciculin|phosphoglucomutase-like protein 5|phosphoglucomutase-related protein</td><td>9</td><td>68356899</td><td>68531061</td></tr>
<tr><td>RELN</td><td>5649</td><td>RELN</td><td>reelin</td><td></td><td>7</td><td>103471784</td><td>103989516</td></tr>
<tr><td>SGCA</td><td>6442</td><td>SGCA</td><td>sarcoglycan, alpha (50kDa dystrophin-associated glycoprotein)</td><td>50 kDa dystrophin-associated glycoprotein|50DAG|50kD DAG|alpha-SG|alpha-sarcoglycan|dystroglycan-2</td><td>17</td><td>50165879</td><td>50175932</td></tr>
<tr><td>SLIT3</td><td>6586</td><td>SLIT3</td><td>slit homolog 3 (Drosophila)</td><td>multiple EGF-like domains protein 5|multiple epidermal growth factor-like domains protein 5|slit homolog 3 protein</td><td>5</td><td>168661733</td><td>169301129</td></tr>
<tr><td>STXBP1</td><td>6812</td><td>STXBP1</td><td>syntaxin binding protein 1</td><td>N-Sec1|neuronal SEC1|protein unc-18 homolog 1|protein unc-18 homolog A|syntaxin-binding protein 1|unc-18A|unc18-1</td><td>9</td><td>127612207</td><td>127692716</td></tr>
<tr><td>THBS1</td><td>7057</td><td>THBS1</td><td>thrombospondin 1</td><td>thrombospondin-1|thrombospondin-1p180</td><td>15</td><td>39581079</td><td>39597467</td></tr>
<tr><td>EPM2A</td><td>7957</td><td>EPM2A</td><td>epilepsy, progressive myoclonus type 2A, Lafora disease (laforin)</td><td>LAFPTPase|epilepsy, progressive myoclonus type 2, Lafora disease (laforin)|glucan phosphatase|lafora PTPase|laforin</td><td>6</td><td>145625304</td><td>145737216</td></tr>
<tr><td>DHX16</td><td>8449</td><td>DHX16</td><td>DEAH (Asp-Glu-Ala-His) box polypeptide 16</td><td>ATP-dependent RNA helicase #3|DEAD/H (Asp-Glu-Ala-Asp/His) box polypeptide 16|DEAD/H box 16|DEAH-box protein 16|RNA helicase|putative pre-mRNA-splicing factor ATP-dependent RNA helicase DHX16</td><td>6</td><td>30653119</td><td>30673053</td></tr>
<tr><td>DLGAP2</td><td>9228</td><td>DLGAP2</td><td>discs, large (Drosophila) homolog-associated protein 2</td><td>PSD-95/SAP90-binding protein 2|SAP90/PSD-95-associated protein 2|discs large-associated protein 2|disks large-associated protein 2</td><td>8</td><td>927021</td><td>1708476</td></tr>
<tr><td>PPT2</td><td>9374</td><td>PPT2</td><td>palmitoyl-protein thioesterase 2</td><td>S-thioesterase G14|VE-statin2|lysosomal thioesterase PPT2|palmitoyl-protein hydrolase 2</td><td>6</td><td>32153452</td><td>32163681</td></tr>
<tr><td>TJP2</td><td>9414</td><td>TJP2</td><td>tight junction protein 2</td><td>Friedreich ataxia region gene X104 (tight junction protein ZO-2)|tight junction protein ZO-2|zona occludens 2|zonula occludens protein 2</td><td>9</td><td>69099959</td><td>69255208</td></tr>
<tr><td>HOMER2</td><td>9455</td><td>HOMER2</td><td>homer homolog 2 (Drosophila)</td><td>cupidin|homer homolog 3|homer protein homolog 2|homer, neuronal immediate early gene, 2</td><td>15</td><td>82841086</td><td>82986161</td></tr>
<tr><td>RPH3AL</td><td>9501</td><td>RPH3AL</td><td>rabphilin 3A-like (without C2 domains)</td><td>no C2 domains protein|rab effector Noc2</td><td>17</td><td>212389</td><td>386254</td></tr>
<tr><td>AKAP12</td><td>9590</td><td>AKAP12</td><td>A kinase (PRKA) anchor protein 12</td><td>A-kinase anchor protein 12|A-kinase anchor protein, 250kDa|AKAP 250|Src-Suppressed C Kinase Substrate|kinase scaffold protein gravin|myasthenia gravis autoantigen gravin</td><td>6</td><td>151239999</td><td>151358559</td></tr>
<tr><td>FEZ1</td><td>9638</td><td>FEZ1</td><td>fasciculation and elongation protein zeta 1 (zygin I)</td><td>fasciculation and elongation protein zeta-1|zygin I|zygin-1</td><td>11</td><td>125445745</td><td>125496310</td></tr>
<tr><td>WSCD2</td><td>9671</td><td>WSCD2</td><td>WSC domain containing 2</td><td>WSC domain-containing protein 2</td><td>12</td><td>108129567</td><td>108250537</td></tr>
<tr><td>PRND</td><td>23627</td><td>PRND</td><td>prion protein 2 (dublet)</td><td>prion gene complex, downstream|prion-like protein doppel</td><td>20</td><td>4721854</td><td>4728462</td></tr>
</table>

<table>
<tr><td>PLXNB2</td><td>23654</td><td>PLXNB2</td><td>plexin B2</td><td>plexin-B2</td><td>22</td><td>50274979</td><td>50307642</td></tr>
<tr><td>MTHFD1L</td><td>25902</td><td>MTHFD1L</td><td>methylenetetrahydrofolate dehydrogenase (NADP+ dependent) 1-like</td><td>10-formyl-THF synthetase|formyltetrahydrofolate synthetase domain containing 1|monofunctional C1-tetrahydrofolate synthase, mitochondrial</td><td>6</td><td>150865679</td><td>151101887</td></tr>
<tr><td>SRPK3</td><td>26576</td><td>SRPK3</td><td>SRSF protein kinase 3</td><td>SFRS protein kinase 3|SR-protein-specific kinase 3|muscle-specific serine kinase 1|serine arginine rich protein-specific kinase 3|serine/arginine-rich protein-specific kinase 3|serine/threonine kinase 23|serine/threonine-protein kinase 23|serine/threonine-protein kinase SRPK3</td><td>X</td><td>153781001</td><td>153785732</td></tr>
<tr><td>HSPB7</td><td>27129</td><td>HSPB7</td><td>heat shock 27kDa protein family, member 7 (cardiovascular)</td><td>cardiovascular heat shock protein|heat shock 27kD protein family, member 7 (cardiovascular)|heat shock protein beta-7</td><td>1</td><td>16014028</td><td>16018790</td></tr>
<tr><td>PCDH17</td><td>27253</td><td>PCDH17</td><td>protocadherin 17</td><td>protocadherin 68|protocadherin-17|protocadherin-68</td><td>13</td><td>57630104</td><td>57729311</td></tr>
<tr><td>BSPRY</td><td>54836</td><td>BSPRY</td><td>B-box and SPRY domain containing</td><td>B box and SPRY domain-containing protein|B-box and SPRY-domain containing protein|zetin 1</td><td>9</td><td>113349532</td><td>113371233</td></tr>
<tr><td>VPS53</td><td>55275</td><td>VPS53</td><td>vacuolar protein sorting 53 homolog (S. cerevisiae)</td><td>vacuolar protein sorting-associated protein 53 homolog</td><td>17</td><td>508668</td><td>714856</td></tr>
<tr><td>CDCA7L</td><td>55536</td><td>CDCA7L</td><td>cell division cycle associated 7-like</td><td>cell division cycle-associated 7-like protein|transcription factor RAM2</td><td>7</td><td>21900899</td><td>21945924</td></tr>
<tr><td>MYO5C</td><td>55930</td><td>MYO5C</td><td>myosin VC</td><td>myosin 5C|myosin-Vc|unconventional myosin-Vc</td><td>15</td><td>52192318</td><td>52295798</td></tr>
<tr><td>PCDHB12</td><td>56124</td><td>PCDHB12</td><td>protocadherin beta 12</td><td>PCDH-beta-12|protocadherin beta-12</td><td>5</td><td>141208719</td><td>141212126</td></tr>
<tr><td>LRRC8A</td><td>56262</td><td>LRRC8A</td><td>leucine rich repeat containing 8 family, member A</td><td>leucine-rich repeat-containing protein 8A</td><td>9</td><td>128882112</td><td>128918039</td></tr>
<tr><td>BARHL1</td><td>56751</td><td>BARHL1</td><td>BarH-like homeobox 1</td><td>barH-like 1 homeobox protein</td><td>9</td><td>132582606</td><td>132590253</td></tr>
<tr><td>TBC1D14</td><td>57533</td><td>TBC1D14</td><td>TBC1 domain family, member 14</td><td>TBC1 domain family member 14</td><td>4</td><td>6909444</td><td>7033118</td></tr>
<tr><td>DOCK6</td><td>57572</td><td>DOCK6</td><td>dedicator of cytokinesis 6</td><td>dedicator of cytokinesis protein 6</td><td>19</td><td>11199293</td><td>11262492</td></tr>
<tr><td>TMEM181</td><td>57583</td><td>TMEM181</td><td>transmembrane protein 181</td><td>G protein-coupled receptor 178</td><td>6</td><td>158536436</td><td>158635435</td></tr>
<tr><td>PERP</td><td>64065</td><td>PERP</td><td>PERP, TP53 apoptosis effector</td><td>1110017A08Rik|KCP-1|keratinocyte associated protein 1|keratinocyte-associated protein 1|keratinocytes associated protein 1|p53 apoptosis effector related to PMP-22|p53 apoptosis effector related to PMP22|p53-induced protein PIGPC1|transmembrane protein THW</td><td>6</td><td>138088505</td><td>138107523</td></tr>
<tr><td>LONRF3</td><td>79836</td><td>LONRF3</td><td>LON peptidase N-terminal domain and ring finger 3</td><td>LON peptidase N-terminal domain and RING finger protein 3|ring finger protein 127</td><td>X</td><td>118974614</td><td>119018355</td></tr>
<tr><td>ULBP2</td><td>80328</td><td>ULBP2</td><td>UL16 binding protein 2</td><td>ALCAN-alpha|N2DL-2|NKG2D ligand 2|NKG2DL2|UL16-binding protein 2|retinoic acid early transcript 1 H|retinoic acid early transcript 1H</td><td>6</td><td>149934765</td><td>149949235</td></tr>
<tr><td>MRO</td><td>83876</td><td>MRO</td><td>maestro</td><td>beside the Ma29 deletion|male-specific transcription in the developing reproductive organs|protein maestro</td><td>18</td><td>50795120</td><td>50825438</td></tr>
<tr><td>KCTD10</td><td>83892</td><td>KCTD10</td><td>potassium channel tetramerization domain containing 10</td><td>BTB/POZ domain-containing adapter for CUL3-mediated RhoA degradation protein 3|BTB/POZ domain-containing protein KCTD10|potassium channel tetramerisation domain containing 10|potassium channel tetramerization domain-containing protein 10</td><td>12</td><td>109448655</td><td>109477360</td></tr>
<tr><td>FAM120B</td><td>84498</td><td>FAM120B</td><td>family with sequence similarity 120B</td><td>PPARG constitutive coactivator 1|PPARgamma constitutive coactivator 1|constitutive coactivator of PPAR-gamma|constitutive coactivator of peroxisome proliferator-activated receptor gamma</td><td>6</td><td>170290703</td><td>170407071</td></tr>
</table>

| | | | | | | | |
|---|---|---|---|---|---|---|---|
| LRP11 | 84918 | LRP11 | low density lipoprotein receptor-related protein 11 | LRP-11\|low-density lipoprotein receptor-related protein 11 | 6 | 149795188 | 149864348 |
| SHANK3 | 85358 | SHANK3 | SH3 and multiple ankyrin repeat domains 3 | SH3 and multiple ankyrin repeat domains protein 3\|proline rich synapse associated protein 2\|shank postsynaptic density protein | 22 | 50674642 | 50733212 |
| SLC25A25 | 114789 | SLC25A25 | solute carrier family 25 (mitochondrial carrier; phosphate carrier), member 25 | calcium-binding mitochondrial carrier protein SCaMC-2\|mitochondrial ATP-Mg/Pi carrier protein 3\|mitochondrial Ca(2+)-dependent solute carrier protein 3\|short calcium-binding mitochondrial carrier 2\|small calcium-binding mitochondrial carrier 2\|solute carrier family 25, member 25 | 9 | 128068200 | 128109258 |
| RAET1E | 135250 | RAET1E | retinoic acid early transcript 1E | NKG2D ligand 4\|RAE-1-like transcript 4\|lymphocyte effector toxicity activation ligand | 6 | 149883375 | 149896059 |
| FAM81A | 145773 | FAM81A | family with sequence similarity 81, member A | protein FAM81A | 15 | 59402276 | 59523552 |
| SLC38A8 | 146167 | SLC38A8 | solute carrier family 38, member 8 | putative sodium-coupled neutral amino acid transporter 8\|solute carrier family 38 member 8 | 16 | 84009667 | 84043037 |
| BTBD19 | 149478 | BTBD19 | BTB (POZ) domain containing 19 | BTB/POZ domain-containing protein 19\|novel BTB domain containing protein | 1 | 44807704 | 44814151 |
| C9orf44 | 158314 | LINC00475 | long intergenic non-protein coding RNA 475 | | 9 | 92141467 | 92159608 |
| LOC221122 | 221122 | LOC221122 | uncharacterized LOC221122 | | 11 | 44973902 | 44978028 |
| IL29 | 282618 | IFNL1 | interferon, lambda 1 | IFN-lambda-1\|cytokine Zcyto21\|interferon lambda-1\|interferon-lambda-1\|interleukin 29 (interferon, lambda 1)\|interleukin-29 | 19 | 39296325 | 39298672 |
| RAET1G | 353091 | RAET1G | retinoic acid early transcript 1G | retinoic acid early transcript 1G protein\|retinoic acid early transcript 1G pseudogene | 6 | 149916878 | 149923133 |
| IGFL1 | 374918 | IGFL1 | IGF-like family member 1 | insulin growth factor-like family member 1 | 19 | 46229752 | 46231243 |
| KLHL30 | 377007 | KLHL30 | kelch-like family member 30 | kelch-like 30\|kelch-like protein 30 | 2 | 238138722 | 238152906 |
| C15orf52 | 388115 | C15orf52 | chromosome 15 open reading frame 52 | uncharacterized protein C15orf52 | 15 | 40331452 | 40340967 |
| MAFA | 389692 | MAFA | v-maf avian musculoaponeurotic fibrosarcoma oncogene homolog A | pancreatic beta-cell-specific transcriptional activator\|transcription factor MafA\|transcription factor RIPE3b1 | 8 | 143428060 | 143430432 |
| SPRED3 | 399473 | SPRED3 | sprouty-related, EVH1 domain containing 3 | sprouty-related, EVH1 domain-containing protein 3 | 19 | 38389979 | 38399884 |
| PATE4 | 399968 | PATE4 | prostate and testis expressed 4 | PATE-like protein B\|prostate and testis expressed protein 4\|prostate and testis expression B | 11 | 125833316 | 125840072 |
| LOC441177 | 441177 | LINC00602 | long intergenic non-protein coding RNA 602 | | 6 | 165987551 | 165989615 |
| SNORA20 | 677806 | SNORA20 | small nucleolar RNA, H/ACA box 20 | | 6 | 159780250 | 159780381 |

**Figure 11**

RNA-seq reads

Trimming of low quality (<Q30) bases at extremities

Alignment of reads on reference sequence

Example of aligned sequences

RefSeq

Aligned RNA-seq reads

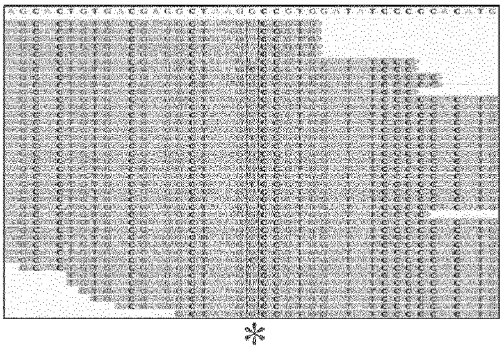

*

Bioinformatical filters

Only alignments with >90% identity

Exclusion of reads with alignments that involved <70% of their length

The first and last five bases of each of the remaining alignments are excluded

Positions where the number of reads are greater than 100 for at least half of the patients of each group of the training set

Positions where there is at least 1 RDD for 1000 reads for 1 patient

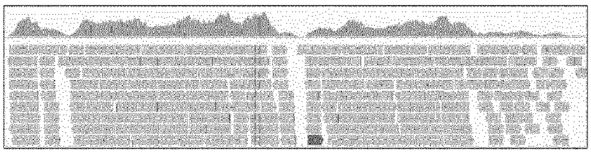

RDD rate calculation for transcript 1

RDD rate = sum of RDD / sum of reads

RDD rate of 5 transcripts for 1 patient

RDD rate of 5 transcripts for each patients

Discriminant model using RDD rate of 5 transcripts for all patients

Prediction of clinical outcome

Figure 12A

| Patient | RDD rate NM_001494.3 **(GDI2)** | RDD rate NM_001089591.1 **(UQCRHL)** | RDD rate NM_003815.4 **(ADAM15)** | RDD rate NM_001099415.2 **(POM121C)** | RDD rate NM_005775.4 **(SORBS3)** | PLS Score | Pre diction | Vital status | Survival time (days) |
|---|---|---|---|---|---|---|---|---|---|
| TCGA-AO-A128 | 0,001249472 | 0,000415578 | 0,000612245 | 0,002905034 | 0,000926932 | 0,434164017 | Good | Alive | 2877 |
| TCGA-AO-A124 | 0,000418698 | 0,003255144 | 0,003414959 | 0,005248112 | 0,002327011 | 2,705535995 | Good | Alive | 3119 |
| TCGA-AO-A129 | 0,000580766 | 0,00198908 | 0,00379911 | 0,004847255 | 0,001403054 | 1,828627287 | Good | Alive | 2923 |
| TCGA-B6-A0IQ | 0,000962944 | 0,001017358 | 0,001093832 | 0,005472724 | 0,002105608 | 1,664509931 | Good | Alive | 3871 |
| TCGA-B6-A0RN | 0,000818893 | 0,001870093 | 0,000283226 | 0,0041909 | 0,002067519 | 1,259802507 | Good | Alive | 5261 |
| TCGA-B6-A0RT | 0,000997027 | 0,000436591 | 0,000468808 | 0,004866437 | 0,002263246 | 1,202840379 | Good | Alive | 2721 |
| TCGA-B6-A0RU | 0,000854142 | 0,002421475 | 0,001131094 | 0,004808463 | 0,002203562 | 2,103061133 | Good | Alive | 3991 |
| TCGA-E2-A1LH | 0,001080745 | 0,001150081 | 0,001954619 | 0 | 0,002648539 | 1,509555996 | Good | Alive | 2875 |
| TCGA-E2-A1LI | 0,001053469 | 0,000719796 | 0,00109384 | 0,003291216 | 0,002689216 | 1,627177825 | Good | Alive | 2750 |
| TCGA-B6-A0I6 | 0,000382625 | 0,000446442 | 0,00053135 | 0,000610612 | 0,001145114 | -1,442669674 | Poor | Dead | 991 |
| TCGA-A2-A0CM | 0,000429959 | 0,000528444 | 0,000612343 | 0 | 0,000806861 | -1,630998602 | Poor | Dead | 754 |
| TCGA-A1-A0SK | 0,000316113 | 0,000475935 | 0,000336814 | 0,000320513 | 0,000271174 | -2,242567513 | Poor | Dead | 967 |
| TCGA-A2-A3XU | 0,000356417 | 0,000415604 | 0,000563387 | 0,000639971 | 0,001041251 | -1,55517059 | Poor | Dead | 912 |
| TCGA-A2-A04P | 0,000299374 | 0,000420716 | 0,00044194 | 0 | 0,001381559 | -1,602921775 | Poor | Dead | 547 |
| TCGA-A8-A09X | 0,000508496 | 0,000597715 | 0,000346406 | 0,000683138 | 0,00062043 | -1,536029089 | Poor | Dead | 426 |
| TCGA-AR-A1AR | 0,000493096 | 0,001158078 | 0,000764489 | 0,000704155 | 0,001419179 | -0,617188932 | Poor | Dead | 523 |
| TCGA-AR-A2LH | 0,000354099 | 0,000372492 | 0,000420821 | 0 | 0,001548475 | -1,427438785 | Poor | Dead | 615 |
| TCGA-B6-A0IK | 0,000402283 | 0,000420221 | 0,000724329 | 0,001783265 | 0,000151853 | -1,76636802 | Poor | Dead | 571 |
| TCGA-B6-A0WX | 0,00057846 | 0,000667945 | 0,000790757 | 0,000875274 | 0,000737295 | -1,105694211 | Poor | Dead | 653 |
| TCGA-EW-A1P8 | 0,000603513 | 0,000715698 | 0,00087007 | 0,006099674 | 0,001601905 | 0,591772119 | Good | Dead | 239 |
| TCGA-A2-A3XS | 0,000532427 | 0,000625939 | 0,000520833 | 0,002244475 | 0,001363196 | -0,623889445 | Poor | Dead | 1032 |
| TCGA-BH-A18V | 0,000350731 | 0,001436528 | 0,000543429 | 0,003419751 | 0,001056583 | -0,509679927 | Poor | Dead | 1555 |
| TCGA-BH-A18Q | 0,00083769 | 0,000634786 | 0,000603318 | 0,000656254 | 0,001264619 | -0,424585689 | Poor | Dead | 1692 |
| TCGA-BH-A1EW | 0,000728896 | 0,000397269 | 0,00040036 | 0,003726637 | 0,001328244 | -0,155414714 | Poor | Dead | 1694 |
| TCGA-AR-A256 | 0,000373152 | 0,000450504 | 0,005217977 | 0,003557863 | 0,002118531 | 1,413145798 | Good | Dead | 2854 |
| TCGA-AO-A03U | 0,000824229 | 0,000600275 | 0,000816231 | 0,000450417 | 0,001945782 | 0,01223908 | Poor | Dead | 1796 |
| TCGA-A2-A3XX | 0,000350583 | 0,00041809 | 0,000937261 | 0,000369822 | 0,001521003 | -1,173848662 | Poor | Dead | 1439 |
| TCGA-AO-A0JL | 0,00076632 | 0,000370479 | 0,004748142 | 0,0023146 | 0,001202781 | 1,067720186 | Good | Alive | 1683 |
| TCGA-E2-A1AZ | 0,000837301 | 0,00053513 | 0,001360809 | 0,004084967 | 0,002721009 | 1,422723345 | Good | Alive | 2329 |
| TCGA-A2-A3XT | 0,000515064 | 0,000593752 | 0,00057199 | 0,000598245 | 0,000999363 | -1,216798045 | Poor | Alive | 2525 |
| TCGA-E2-A14N | 0,001059812 | 0,001104169 | 0,002384359 | 0,00167304 | 0,001715555 | 1,325749173 | Good | Alive | 1434 |
| TCGA-BH-A0B3 | 0,000926801 | 0,000409415 | 0,001564456 | 0,005051825 | 0,002429191 | 1,59489476 | Good | Alive | 1203 |
| TCGA-AR-A0TS | 0,000349494 | 0,000407105 | 0,00055788 | 0,006270705 | 0,000992204 | -0,48671204 | Poor | Alive | 1138 |
| TCGA-B6-A0RG | 0,000801231 | 0,000477872 | 0,000275672 | 0,002333178 | 0,003259591 | 0,950624548 | Good | Alive | 2082 |
| TCGA-AO-A1KR | 0,00116287 | 0,001786412 | 0,000400212 | 0,002447869 | 0,001006479 | 0,837428792 | Good | Alive | 2140 |
| TCGA-A2-A04T | 0,000979941 | 0,000463488 | 0,009665276 | 0,006185135 | 0,00133534 | 4,082901867 | Good | Alive | 2246 |
| TCGA-AR-A1AI | 0,000871674 | 0,002668538 | 0,001154264 | 0,007835634 | 0,003238173 | 3,539102734 | Good | Alive | 1881 |
| TCGA-GM-A3XL | 0,00270137 | 0,002765732 | 0,002235603 | 0,002442599 | 0,002436189 | 5,644555537 | Good | Alive | 2108 |
| TCGA-A2-A0ST | 0,000499133 | 0,000444295 | 0,00424881 | 0,003924704 | 0,002182496 | 1,408453648 | Good | Alive | 3017 |
| TCGA-AR-A0U4 | 0,000860302 | 0,00055506 | 0,002198147 | 0,000538235 | 0,002229049 | 0,743366592 | Good | Alive | 1627 |
| TCGA-GM-A2DB | 0,000898533 | 0,001608745 | 0,00244263 | 0,000633814 | 0,002433448 | 1,566355626 | Good | Alive | 1615 |
| TCGA-AO-A12F | 0,000432805 | 0,000510634 | 0,000988247 | 0,004844766 | 0,001244585 | -0,25438061 | Poor | Alive | 1471 |
| TCGA-AR-A1AQ | 0,001001813 | 0,000551449 | 0,00044575 | 0,003419253 | 0,001157403 | 0,251663972 | Good | Alive | 1309 |
| TCGA-GM-A2DF | 0,000904021 | 0,001269512 | 0,001094965 | 0,003314809 | 0,003094581 | 1,898545259 | Good | Alive | 1299 |
| TCGA-BH-A0B9 | 0,000454155 | 0,002548238 | 0,000300368 | 0,003613788 | 0,002823245 | 1,323731665 | Good | Alive | 1572 |
| TCGA-A2-A0SX | 0,001149911 | 0,0004178 | 0,00028585 | 0,007104796 | 0,002498169 | 2,000295683 | Good | Alive | 1534 |
| TCGA-A2-A04Q | 0,000714211 | 0,0004301 | 0,000424268 | 0,003628344 | 0,002957935 | 0,884865845 | Good | Alive | 1275 |
| TCGA-GM-A2DI | 0,00116944 | 0,000558175 | 0,000658328 | 0,002920871 | 0,002064526 | 1,12136308 | Good | Alive | 1883 |
| TCGA-BH-A0BL | 0,000982279 | 0,000438379 | 0,001229911 | 0,005393342 | 0,001748062 | 1,214598404 | Good | Alive | 1339 |
| TCGA-GM-A2DD | 0,000853713 | 0,001143613 | 0,001087623 | 0,003954172 | 0,003170644 | 1,920917247 | Good | Alive | 1309 |
| TCGA-GM-A2DH | 0,001339837 | 0,002976833 | 0,001527184 | 0,003824092 | 0,003014302 | 3,715533768 | Good | Alive | 1286 |
| TCGA-BH-A0AV | 0,000375752 | 0,000481085 | 0,000201126 | 0,000233563 | 0,001492279 | -1,403100115 | Poor | Alive | 1180 |

**Figure 12B**

Europäisches Patentamt
European Patent Office
Office européen des brevets

# EUROPEAN SEARCH REPORT

Application Number
EP 14 18 4663

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MILAN RADOVICH ET AL: "Characterizing the heterogeneity of triple-negative breast cancers using microdissected normal ductal epithelium and RNA-sequencing", BREAST CANCER RESEARCH AND TREATMENT, vol. 143, no. 1, 1 January 2014 (2014-01-01), pages 57-68, XP055172672, ISSN: 0167-6806, DOI: 10.1007/s10549-013-2780-y ----- | 1-14 | INV. C12Q1/68 |
| A | OGIER, V. ET AL: "Abstract 2225: First double validation of transcription infidelity biomarkers for breast cancer diagnosis", CANCER RESEARCH. PROCEEDINGS: AACR 102ND ANNUAL MEETING 2011, APR 2-6, 2011; ORLANDO, FL, vol. 71, no. 8, 15 April 2011 (2011-04-15), XP002736586, Retrieved from the Internet: URL:http://cancerres.aacrjournals.org/content/71/8_Supplement/2225> * abstract * ----- | 1-14 | |
| A | LIU YANG ET AL: "Triple negative breast cancer therapy with CDK1 siRNA delivered by cationic lipid assisted PEG-PLA nanoparticles", JOURNAL OF CONTROLLED RELEASE, vol. 192, 10 July 2014 (2014-07-10), pages 114-121, XP029060799, ISSN: 0168-3659, DOI: 10.1016/J.JCONREL.2014.07.001 ----- | 1-14 | |

| TECHNICAL FIELDS SEARCHED (IPC) |
|---|
| C12Q |

2

~~The present search report has been drawn up for all claims~~

| Place of search | Date of completion of the search | Examiner |
|---|---|---|
| The Hague | 2 March 2015 | Aslund, Fredrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 14 18 4663

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-14

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 14 18 4663

The Search Division considers that the present European patent application does not comply with the requirements of unity of invention and relates to several inventions or groups of inventions, namely:

1. claims: 1-14

   A method for predicting the clinical outcome of triple negative breast cancer (TNBC) in a subject having TNBC, the method comprising measuring the rate of divergences between RNA and DNA sequences (RDD) in at least one target gene in a sample from said subject, wherein the rate of divergences is indicative of the clinical outcome of said cancer.

---

2. claims: 15, 16

   A compound that modulates the amount or activity of a protein selected from GDI2, SORBS3, POM121C, ADAM15, UQCRHL, or a combination thereof; for use in a method for treating TNBC in a subject having TNBC.

---

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2046986 B1


### Non-patent literature cited in the description


- Worldwide cancer mortality statistics. *Cancer Research UK,* 11 June 2014, http://www.cancerresearchuk.org/cancer-info/cancerstats/world/mortality
- **COLEMAN MP et al.** Cancer survival in five continents: a worldwide population-based study (CONCORD). *Lancet Oncol,* 2008, vol. 9, 730-756
- The Cancer Genome Atlas Network (2012) Comprehensive molecular portraits of human breast tumors. *Nature,* 2012, vol. 490, 61-70
- **PEROU CM et al.** Molecular portraits of human breast tumours. *Nature,* 2000, vol. 406, 747-752
- **AZIM HA JR et al.** Utility of prognostic genomic tests in breast cancer practice: The IMPAKT 2012 Working Group Consensus Statement. *Ann Oncol Off J Eur Soc Med Oncol ESMO,* 2013, vol. 24, 647-654
- **SYMMANS WF.** A perspective on genomic tests for breast cancer: the need for progress. *Oncol Williston Park N,* 2012, vol. 26, 364-365
- **HORNBERGER J et al.** Clinical validity/utility, change in practice patterns, and economic implications of risk stratifiers to predict outcomes for early-stage breast cancer: a systematic review. *J Natl Cancer Inst,* 2012, vol. 104, 1068-1079
- **RUIJTER TC ; VEECK J ; DE HOON JPJ ; VAN ENGELAND M ; TJAN-HEIJNEN VC.** Characteristics of triple-negative breast cancer. *J Cancer Res Clin Oncol,* 2011, vol. 137, 183-192
- **DENT R et al.** Triple-negative breast cancer: clinical features and patterns of recurrence. *Clin Cancer Res Off J Am Assoc Cancer Res,* 2007, vol. 13, 4429-4434
- **BRULLIARD M et al.** Nonrandom variations in human cancer ESTs indicate that mRNA heterogeneity increases during carcinogenesis. *Proc Natl Acad Sci U S A,* 2007, vol. 104, 7522-7527
- **WANG IX et al.** RNA-DNA Differences Are Generated in Human Cells within Seconds after RNA Exits Polymerase II. *Cell Rep,* 2014, vol. 6, 906-915
- **LI M ; WANG IX ; CHEUNG VG.** Response to Comments on "Widespread RNA and DNA Sequence Differences in the Human Transcriptome. *Science,* 2012, 335
- **LI M et al.** Widespread RNA and DNA sequence differences in the human transcriptome. *Science,* 2011, vol. 333, 53-58
- **LIN W ; PISKOL R ; TAN MH ; LI JB.** Comment on "Widespread RNA and DNA sequence differences in the human transcriptome. *Science,* 2012, vol. 335, 1302
- **KLEINMAN CL ; MAJEWSKI J.** Comment on "Widespread RNA and DNA sequence differences in the human transcriptome. *Science,* 2012, vol. 335, 1302
- **PICKRELL JK ; GILAD Y ; PRITCHARD JK.** Comment on "Widespread RNA and DNA sequence differences in the human transcriptome. *Science,* 2012, vol. 335, 1302
- **BAHN JH et al.** Accurate identification of A-to-I RNA editing in human by transcriptome sequencing. *Genome Res,* 2012, vol. 22, 142-50
- **BIANCHETTI L ; KIEFFER D ; FÉDERKEIL R ; POCH O.** Increased frequency of single base substitutions in a population of transcripts expressed in cancer cells. *BMC Cancer,* 2012, vol. 12, 509
- **PENG Z et al.** Comprehensive analysis of RNA-Seq data reveals extensive RNA editing in a human transcriptome. *Nat Biotechnol,* 2012, vol. 30, 253-60
- **ANDRÉ F et al.** Comparative genomic hybridisation array and DNA sequencing to direct treatment of metastatic breast cancer: a multicentre, prospective trial (SAFIR01/UNICANCER). *Lancet Oncol,* 2014, vol. 15, 267-274
- **SWANTON C.** Intratumor heterogeneity: evolution through space and time. *Cancer Res,* 2012, vol. 72, 4875-4882
- **PAIK S et al.** A multigene assay to predict recurrence of tamoxifen-treated, node-negative breast cancer. *N Engl J Med,* 2004, vol. 351, 2817-2826
- **VAN 'T VEER LJ et al.** Gene expression profiling predicts clinical outcome of breast cancer. *Nature,* 2002, vol. 415, 530-536
- **CHEN X et al.** XBP1 promotes triple-negative breast cancer by controlling the HIF1$\alpha$ pathway. *Nature,* 2014, vol. 508, 103-107

- **PATHMANATHAN N ; BALLEINE RL.** Ki67 and proliferation in breast cancer. *J Clin Pathol,* 2013, vol. 66, 512-516
- **SCHREIBER RD ; OLD LJ ; SMYTH MJ.** Cancer immunoediting: integrating immunity's roles in cancer suppression and promotion. *Science,* 2011, vol. 331, 1565-1570
- **LI H ; DURBIN R.** Fast and accurate short read alignment with Burrows-Wheeler transform. *Bioinforma Oxf Engl,* 2009, vol. 25, 1754-1760
- **LI B ; DEWEY CN.** RSEM: accurate transcript quantification from RNA-Seq data with or without a reference genome. *BMC Bioinformatics,* 2011, vol. 12, 323
- **LÊ CAO K-A ; BOITARD S ; BESSE P.** Sparse PLS discriminant analysis: biologically relevant feature selection and graphical displays for multiclass problems. *BMC Bioinformatics,* 2011, vol. 12, 253
- Development Core Team R: A language and environment for statistical computing. *R Foundation for Statistical Computing, http://www.R-project.org.*
- **DEJEAN, I. GONZALEZ ; K.-A. LÊ CAO.** mixOmics: Omics Data Integration. *Project. R Package Version 50-1 HttpCRANR-Proj,* 2013
- **MA X-J et al.** A two-gene expression ratio predicts clinical outcome in breast cancer patients treated with tamoxifen. *Cancer Cell,* 2004, vol. 5, 607-616
- **KOHL M.** SLqPCR: Functions for analysis of real-time quantitative PCR data at SIRS-Lab GmbH. *R Package SIRS-Lab GmbH Jena,* 2007